# EUROPEAN PATENT APPLICATION

(11) **EP 2 677 034 A1**
(43) Date of publication of application: **25.12.2013**
(21) Application number: 12172389.4
(22) Date of filing: 18.06.2012
(51) Int. Cl.: C12N 15/70

(54) **Genome sequence based targeted cloning of DNA fragments**

(71) Applicant: LEK Pharmaceuticals d.d., 1526 Ljubljana (SI)
(72) Inventor: The designation of the inventor has not yet been filed
(74) Representative: Kunic Tesovic, Barbara

(57) **Abstract**

The present invention belongs to the field of molecular biology, microbiology and microbial genetics and specifies the plasmid rescue method for targeted cloning of DNA fragments from a donor organism into a host cell, comprising *inter alia* the steps of transferring a Bacterial Artificial Chromosome (BAC)-vector that comprises a site-specific recombination system containming an integrase and an attP site into a donor organism, wherein said donor organism does not contain a functional attB site. Further, the present invention refers to a plasmid rescue method for targeted cloning of DNA fragments from a donor organism into a host cell, comprising inter alia the steps of transferring a BAC-vector that comprises a site-specific recombination system containing an attP site however no functional integrase into a donor organism, wherein in a further step a functional integrase has to be introduced into a final host cell. Further, the present invention refers to a vector comprising *inter alia* a site-specific recombination system containing an attP site however no functional integrase, and to a vector comprising inter alia a site-specific recombination system containing an integrase and an attP site and a DNA sequence suitable for homologous recombination. The present invention further refers to the use of said vectors for cloning and/or transferring of a DNA fragment. Additionally, the present invention refers to suitable donor organisms comprising the BAC vectors according to the present invention, to an expression system comprising a host cell and a BAC vector, as well as to the use of said expression system for cloning and/or transfer of the DNA fragment, and /or for expression of said DNA fragment.

SEQUENCE LISTING

<110> Lek Pharmaceuticals d.d.

<120> Genome sequence based targeted cloning of DNA fragments

<130> L 255-P28993EP00

<160> 53

<170> BiSSAP 1.0

<210> 1
<211> 55
<212> DNA
<213> Artificial Sequence

<220>
<221> source
<222> 1..55
<223> /mol_type="DNA"
      /note="oligonucleotide primer"
      /organism="Artificial Sequence"

<400> 1
cggcctcgag atgcatccta ggtgagatta tcaaaaagga tcttcaccta gatcc          55


<210> 2
<211> 47
<212> DNA
<213> Artificial Sequence

<220>
<221> source
<222> 1..47
<223> /mol_type="DNA"
      /note="oligonucleotide primer"
      /organism="Artificial Sequence"

<400> 2
ggccctcgag ggatccgtgc acaaattccc caatgtcaag cacttcc                   47


<210> 3
<211> 10789
<212> DNA
<213> Artificial Sequence

<220>
<221> source
<222> 1..10789
<223> /mol_type="DNA"
      /note="pBeloBAC11_pSET152"
      /organism="Artificial Sequence"

<400> 3
ctcgacatgc atcctaggtg agattatcaa aaaggatctt cacctagatc cttttggttc      60

atgtgcagct ccatcagcaa aaggggatga taagtttatc accaccgact atttgcaaca     120

gcgccggtga tcgtgctatg atcgactgat gtcatcagcg gtggagtgca atgtcgtgca     180

atacgaatgg cgaaaagccg agctcatcgg tcagcttctc aaccttgggg ttacccccgg     240

cggtgtgctg ctggtccaca gctccttccg tagcgtccgg cccctcgaag atgggccact 300

tggactgatc gaggccctgc gtgctgcgct gggtccggga gggacgctcg tcatgccctc 360

gtggtcaggt ctggacgacg agccgttcga tcctgccacg tcgcccgtta caccggacct 420

tggagttgtc tctgacacat tctggcgcct gccaaatgta aagcgcagcg cccatccatt 480

tgcctttgcg gcagcggggc cacaggcaga gcagatcatc tctgatccat tgcccctgcc 540

acctcactcg cctgcaagcc cggtcgcccg tgtccatgaa ctcgatgggc aggtacttct 600

cctcggcgtg ggacacgatg ccaacacgac gctgcatctt gccgagttga tggcaaaggt 660

tccctatggg gtgccgagac actgcaccat tcttcaggat ggcaagttgg tacgcgtcga 720

ttatctcgag aatgaccact gctgtgagcg ctttgccttg gcggacaggt ggctcaagga 780

gaagagcctt cagaaggaag gtccagtcgg tcatgccttt gctcggttga tccgctcccg 840

cgacattgtg gcgacagccc tgggtcaact gggccgagat ccgttgatct tcctgcatcc 900

gccagaggcg ggatgcgaag aatgcgatgc cgctcgccag tcgattggct gagctcatga 960

gcggagaacg agatgacgtt ggaggggcaa ggtcgcgctg attgctgggg caacacgtgg 1020

agcggatcgg ggattgtctt tcttcagctc gctgatgata tgctgacgct caatgccgtt 1080

tggcctccga ctaacgaaaa tcccgcattt ggacggctga tccgattggc acggcggacg 1140

gcgaatggcg gagcagacgc tcgtccgggg gcaatgagat atgaaaaagc ctgaactcac 1200

cgcgacgtat cgggccctgg ccagctagct agagtcgacc tgcaggtccc cggggatcgg 1260

tcttgccttg ctcgtcggtg atgtacttca ccagcttcgc gaagtcgctc ttcttgatgg 1320

agcgcatggg gacgtgcttg gcaatcacgc gcaccccccg gccgttttag cggctaaaaa 1380

agtcatggct ctgccctcgg gcggaccacg cccatcatga ccttgccaag ctcgtcctgc 1440

ttctcttcga tcttcgccag cagggcgagg atcgtggcat caccgaaccg cgccgtgcgc 1500

gggtcgtcgg tgagccagag tttcagcagg ccgcccaggc ggcccaggtc gccattgatg 1560

cgggccagct cgcggacgtg ctcatagtcc acgacgcccg tgattttgta gccctggccg 1620

acggccagca ggtaggccga caggctcatg ccggccgccg ccgccttttc ctcaatcgct 1680

cttcgttcgt ctggaaggca gtacaccttg ataggtgggc tgcccttcct ggttggcttg 1740

gtttcatcag ccatccgctt gccctcatct gttacgccgg cggtagccgg ccagcctcgc 1800

agagcaggat tcccgttgag caccgccagg tgcgaataag ggacagtgaa gaaggaacac 1860

ccgctcgcgg gtgggcctac ttcacctatc ctgcccggct gacgccgttg gatacaccaa 1920

ggaaagtcta cacgaaccct ttggcaaaat cctgtatatc gtgcgaaaaa ggatggatat 1980

accgaaaaaa tcgctataat gaccccgaag cagggttatg cagcggaaaa gatccgtcga 2040

cctgcaggca tgcaagctct agcgattgcc atgacgtccc gaaggcgtgg cgcggcttcc 2100

ccgtgccgga gcaatcgccc tgggtgggtt acacgacgcc cctctatggc ccgtactgac 2160

ggacacaccg aagccccggc ggcaaccctc agcggatgcc ccggggcttc acgttttccc 2220

aggtcagaag cggttttcgg gagtagtgcc ccaactgggg taacctttga gttctctcag 2280

ttgggggcgt agggtcgccg acatgacaca aggggttgtg accggggtgg acacgtacgc 2340

gggtgcttac gaccgtcagt cgcgcgagcg cgaaattcga gcgcagcaag cccagcgaca 2400

cagcgtagcg ccaacgaaga caaggcggcc gaccttcagc gcgaagtcga gcgcgacggg 2460

ggccggttca ggttcgtcgg gcatttcagc gaagcgccgg gcacgtcggc gttcgggacg 2520

gcggagcgcc cggagttcga acgcatcctg aacgaatgcc gcgccgggcg gctcaacatg 2580

atcattgtct atgacgtgtc gcgcttctcg cgcctgaagg tcatggacgc gattccgatt 2640

gtctcggaat tgctcgccct gggcgtgacg attgtttcca ctcaggaagg cgtcttccgg 2700

cagggaaacg tcatggacct gattcacctg attatgcggc tcgacgcgtc gcacaaagaa 2760

tcttcgctga agtcggcgaa gattctcgac acgaagaacc ttcagcgcga attgggcggg 2820

tacgtcggcg ggaaggcgcc ttacggcttc gagcttgttt cggagacgaa ggagatcacg 2880

cgcaacggcc gaatggtcaa tgtcgtcatc aacaagcttg cgcactcgac cactcccctt 2940

accggaccct tcgagttcga gcccgacgta atccggtggt ggtggcgtga gatcaagacg 3000

cacaaacacc ttcccttcaa gccgggcagt caagccgcca ttcacccggg cagcatcacg 3060

gggctttgta agcgcatgga cgctgacgcc gtgccgaccc ggggcgagac gattgggaag 3120

aagaccgctt caagcgcctg ggacccggca accgttatgc gaatccttcg ggacccgcgt 3180

attgcgggct tcgccgctga ggtgatctac aagaagaagc cggacggcac gccgaccacg 3240

aagattgagg gttaccgcat tcagcgcgac ccgatcacgc tccggccggt cgagcttgat 3300

tgcggaccga tcatcgagcc cgctgagtgg tatgagcttc aggcgtggtt ggacggcagg 3360

gggcgcggca aggggctttc ccgggggcaa gccattctgt ccgccatgga caagctgtac 3420

tgcgagtgtg gcgccgtcat gacttcgaag cgcggggaag aatcgatcaa ggactcttac 3480

cgctgccgtc gccggaaggt ggtcgacccg tccgcacctg ggcagcacga aggcacgtgc 3540

aacgtcagca tggcggcact cgacaagttc gttgcggaac gcatcttcaa caagatcagg 3600

cacgccgaag gcgacgaaga gacgttggcg cttctgtggg aagccgcccg acgcttcggc 3660

aagctcactg aggcgcctga gaagagcggc gaacggcgaa accttgttgc ggagcgcgcc 3720

gacgccctga acgcccttga agagctgtac gaagaccgcg cggcaggcgc gtacgacgga 3780

cccgttggca ggaagcactt ccggaagcaa caggcagcgc tgacgctccg gcagcaaggg 3840

gcggaagagc ggcttgccga acttgaagcc gccgaagccc cgaagcttcc ccttgaccaa 3900

tggttccccg aagacgccga cgctgacccg accggcccta agtcgtggtg ggggcgcgcg 3960

tcagtagacg acaagcgcgt gttcgtcggg ctcttcgtag acaagatcgt tgtcacgaag 4020

tcgactacgg gcagggggca gggaacgccc atcgagaagc gcgcttcgat cacgtgggcg 4080

aagccgccga ccgacgacga cgaagacgac gcccaggacg gcacggaaga cgtagcggcg 4140

tagcgagaca cccgggaagc ctgatctacg tctgtcgaga agtttctgat cgaaaagttc 4200

gacagcgtct ccgacctgat gcagctctcg gagggcgaag aatctcgtgc tttcagcttc 4260

gatgtaggag ggcgtggata tgtcctgcgg gtaaatagct gcgccgatgg tttctacaaa 4320

gatcgttatg tttatcggca ctttgcatcg gccgcgctcc cgattccgga agtgcttgac 4380

attggggaat ttgtgcacgg atccgtcgag caattctcat gtttgacagc ttatcatcga 4440

atttctgcca ttcatccgct tattatcact tattcaggcg tagcaaccag gcgtttaagg 4500

gcaccaataa ctgccttaaa aaaattacgc cccgccctgc cactcatcgc agtactgttg 4560

taattcatta agcattctgc cgacatggaa gccatcacaa acggcatgat gaacctgaat 4620

cgccagcggc atcagcacct tgtcgccttg cgtataatat ttgcccatgg tgaaaacggg 4680

ggcgaagaag ttgtccatat tggccacgtt taaatcaaaa ctggtgaaac tcacccaggg 4740

attggctgag acgaaaaaca tattctcaat aaacccttta gggaaatagg ccaggttttc 4800

accgtaacac gccacatctt gcgaatatat gtgtagaaac tgccggaaat cgtcgtggta 4860

ttcactccag agcgatgaaa acgtttcagt ttgctcatgg aaaacggtgt aacaagggtg 4920

aacactatcc catatcacca gctcaccgtc tttcattgcc atacggaatt ccggatgagc 4980

attcatcagg cgggcaagaa tgtgaataaa ggccggataa aacttgtgct tatttttctt 5040

tacggtcttt aaaaaggccg taatatccag ctgaacggtc tggttatagg tacattgagc 5100

aactgactga aatgcctcaa aatgttcttt acgatgccat tgggatatat caacggtggt 5160

atatccagtg atttttttct ccattttagc ttccttagct cctgaaaatc tcgataactc 5220

aaaaaatacg cccggtagtg atcttatttc attatggtga aagttggaac ctcttacgtg 5280

ccgatcaacg tctcattttc gccaaaagtt ggcccagggc ttcccggtat caacagggac 5340

accaggattt atttattctg cgaagtgatc ttccgtcaca ggtatttatt cgcgataagc 5400

tcatggagcg gcgtaaccgt cgcacaggaa ggacagagaa agcgcggatc tgggaagtga 5460

cggacagaac ggtcaggacc tggattgggg aggcggttgc cgccgctgct gctgacggtg 5520

tgacgttctc tgttccggtc acaccacata cgttccgcca ttcctatgcg atgcacatgc 5580

tgtatgccgg tataccgctg aaagttctgc aaagcctgat gggacataag tccatcagtt 5640

caacggaagt ctacacgaag gtttttgcgc tggatgtggc tgcccggcac cgggtgcagt 5700

ttgcgatgcc ggagtctgat gcggttgcga tgctgaaaca attatcctga gaataaatgc 5760

cttggccttt atatggaaat gtggaactga gtggatatgc tgtttttgtc tgttaaacag 5820

agaagctggc tgttatccac tgagaagcga acgaaacagt cgggaaaatc tcccattatc 5880

gtagagatcc gcattattaa tctcaggagc ctgtgtagcg tttataggaa gtagtgttct 5940

gtcatgatgc ctgcaagcgg taacgaaaac gatttgaata tgccttcagg aacaatagaa 6000

atcttcgtgc ggtgttacgt tgaagtggag cggattatgt cagcaatgga cagaacaacc 6060

taatgaacac agaaccatga tgtggtctgt ccttttacag ccagtagtgc tcgccgcagt 6120

cgagcgacag ggcgaagccc tcgagtgagc gaggaagcac cagggaacag cacttatata 6180

ttctgcttac acacgatgcc tgaaaaaact tcccttgggg ttatccactt atccacgggg 6240

atatttttat aattattttt tttatagttt ttagatcttc ttttttagag cgccttgtag 6300

gcctttatcc atgctggttc tagagaaggt gttgtgacaa attgcccttt cagtgtgaca 6360

aatcaccctc aaatgacagt cctgtctgtg acaaattgcc cttaaccctg tgacaaattg 6420

ccctcagaag aagctgtttt ttcacaaagt tatccctgct tattgactct tttttattta 6480

gtgtgacaat ctaaaaactt gtcacacttc acatggatct gtcatggcgg aaacagcggt 6540

tatcaatcac aagaaacgta aaaatagccc gcgaatcgtc cagtcaaacg acctcactga 6600

ggcggcatat agtctctccc gggatcaaaa acgtatgctg tatctgttcg ttgaccagat 6660

cagaaaatct gatggcaccc tacaggaaca tgacggtatc tgcgagatcc atgttgctaa 6720

atatgctgaa atattcggat tgacctctgc ggaagccagt aaggatatac ggcaggcatt 6780

gaagagtttc gcggggaagg aagtggtttt ttatcgccct gaagaggatg ccggcgatga 6840

aaaaggctat gaatcttttc cttggtttat caaacgtgcg cacagtccat ccagagggct 6900

ttacagtgta catatcaacc catatctcat tcccttcttt atcgggttac agaaccggtt 6960

tacgcagttt cggcttagtg aaacaaaaga aatcaccaat ccgtatgcca tgcgtttata 7020

cgaatccctg tgtcagtatc gtaagccgga tggctcaggc atcgtctctc tgaaaatcga 7080

ctggatcata gagcgttacc agctgcctca aagttaccag cgtatgcctg acttccgccg 7140

ccgcttcctg caggtctgtg ttaatgagat caacagcaga actccaatgc gcctctcata 7200

cattgagaaa aagaaaggcc gccagacgac tcatatcgta ttttccttcc gcgatatcac 7260

ttccatgacg acaggatagt ctgagggtta tctgtcacag atttgagggt ggttcgtcac 7320

atttgttctg acctactgag ggtaatttgt cacagttttg ctgtttcctt cagcctgcat 7380

ggattttctc atactttttg aactgtaatt tttaaggaag ccaaatttga gggcagtttg 7440

tcacagttga tttccttctc tttcccttcg tcatgtgacc tgatatcggg ggttagttcg 7500

tcatcattga tgagggttga ttatcacagt ttattactct gaattggcta tccgcgtgtg 7560

tacctctacc tggagttttt cccacggtgg atatttcttc ttgcgctgag cgtaagagct 7620

atctgacaga acagttcttc tttgcttcct cgccagttcg ctcgctatgc tcggttacac 7680

ggctgcggcg agcgctagtg ataataagtg actgaggtat gtgctcttct tatctccttt 7740

tgtagtgttg ctcttatttt aaacaacttt gcggtttttt gatgactttg cgattttgtt 7800

gttgctttgc agtaaattgc aagatttaat aaaaaaacgc aaagcaatga ttaaaggatg 7860

ttcagaatga aactcatgga aacacttaac cagtgcataa acgctggtca tgaaatgacg 7920

aaggctatcg ccattgcaca gtttaatgat gacagcccgg aagcgaggaa aataacccgg 7980

cgctggagaa taggtgaagc agcggattta gttggggttt cttctcaggc tatcagagat 8040

gccgagaaag cagggcgact accgcacccg gatatggaaa ttcgaggacg ggttgagcaa 8100

cgtgttggtt atacaattga acaaattaat catatgcgtg atgtgtttgg tacgcgattg 8160

cgacgtgctg aagacgtatt tccaccggtg atcggggttg ctgcccataa aggtggcgtt 8220

tacaaaacct cagtttctgt tcatcttgct caggatctgg ctctgaaggg gctacgtgtt 8280

ttgctcgtgg aaggtaacga cccccaggga acagcctcaa tgtatcacgg atgggtacca 8340

gatcttcata ttcatgcaga agacactctc ctgcctttct atcttgggga aaaggacgat 8400

gtcacttatg caataaagcc cacttgctgg ccggggcttg acattattcc ttcctgtctg 8460

gctctgcacc gtattgaaac tgagttaatg ggcaaatttg atgaaggtaa actgcccacc 8520

gatccacacc tgatgctccg actggccatt gaaactgttg ctcatgacta tgatgtcata 8580

gttattgaca gcgcgcctaa cctgggtatc ggcacgatta atgtcgtatg tgctgctgat 8640

gtgctgattg ttcccacgcc tgctgagttg tttgactaca cctccgcact gcagtttttc 8700

gatatgcttc gtgatctgct caagaacgtt gatcttaaag ggttcgagcc tgatgtacgt 8760

attttgctta ccaaatacag caatagtaat ggctctcagt ccccgtggat ggaggagcaa 8820

attcgggatg cctggggaag catggttcta aaaaatgttg tacgtgaaac ggatgaagtt 8880

ggtaaaggtc agatccggat gagaactgtt tttgaacagg ccattgatca acgctcttca 8940

actggtgcct ggagaaatgc tctttctatt tgggaacctg tctgcaatga aattttcgat 9000

cgtctgatta aaccacgctg ggagattaga taatgaagcg tgcgcctgtt attccaaaac 9060

atacgctcaa tactcaaccg gttgaagata cttcgttatc gacaccagct gccccgatgg 9120

tggattcgtt aattgcgcgc gtaggagtaa tggctcgcgg taatgccatt actttgcctg 9180

tatgtggtcg ggatgtgaag tttactcttg aagtgctccg gggtgatagt gttgagaaga 9240

cctctcgggt atggtcaggt aatgaacgtg accaggagct gcttactgag gacgcactgg 9300

atgatctcat cccttctttt ctactgactg gtcaacagac accggcgttc ggtcgaagag 9360

tatctggtgt catagaaatt gccgatggga gtcgccgtcg taaagctgct gcacttaccg 9420

aaagtgatta tcgtgttctg gttggcgagc tggatgatga gcagatggct gcattatcca 9480

gattgggtaa cgattatcgc ccaacaagtg cttatgaacg tggtcagcgt tatgcaagcc 9540

gattgcagaa tgaatttgct ggaaatattt ctgcgctggc tgatgcggaa aatatttcac 9600

gtaagattat tacccgctgt atcaacaccg ccaaattgcc taaatcagtt gttgctcttt 9660

tttctcaccc cggtgaacta tctgcccggt caggtgatgc acttcaaaaa gcctttacag 9720

ataaagagga attacttaag cagcaggcat ctaaccttca tgagcagaaa aaagctgggg 9780

tgatatttga agctgaagaa gttatcactc ttttaacttc tgtgcttaaa acgtcatctg 9840

catcaagaac tagtttaagc tcacgacatc agtttgctcc tggagcgaca gtattgtata 9900

agggcgataa aatggtgctt aacctggaca ggtctcgtgt tccaactgag tgtatagaga 9960

aaattgaggc cattcttaag gaacttgaaa agccagcacc ctgatgcgac cacgttttag 10020

tctacgttta tctgtcttta cttaatgtcc tttgttacag gccagaaagc ataactggcc 10080

tgaatattct ctctgggccc actgttccac ttgtatcgtc ggtctgataa tcagactggg 10140

accacggtcc cactcgtatc gtcggtctga ttattagtct gggaccacgg tcccactcgt 10200

atcgtcggtc tgattattag tctgggacca cggtcccact cgtatcgtcg gtctgataat 10260

cagactggga ccacggtccc actcgtatcg tcggtctgat tattagtctg ggaccatggt 10320

cccactcgta tcgtcggtct gattattagt ctgggaccac ggtcccactc gtatcgtcgg 10380

tctgattatt agtctggaac cacggtccca ctcgtatcgt cggtctgatt attagtctgg 10440

gaccacggtc ccactcgtat cgtcggtctg attattagtc tgggaccacg atcccactcg 10500

tgttgtcggt ctgattatcg gtctgggacc acggtcccac ttgtattgtc gatcagacta 10560

tcagcgtgag actacgattc catcaatgcc tgtcaagggc aagtattgac atgtcgtcgt 10620

aacctgtaga acggagtaac ctcggtgtgc ggttgtatgc ctgctgtgga ttgctgctgt 10680

gtcctgctta tccacaacat tttgcgcacg gttatgtgga caaaatacct ggttacccag 10740

gccgtgccgg cacgttaacc gggctgcatc cgatgcaagt gtgtcgctg 10789

<210> 4
<211> 35
<212> DNA
<213> Artificial Sequence

<220>
<221> source
<222> 1..35
<223> /mol_type="DNA"
/note="oligonucleotide primer"
/organism="Artificial Sequence"

<400> 4
cggcgtgcac agtgtgtgac ggacggctat ctcaa 35

<210> 5
<211> 44
<212> DNA
<213> Artificial Sequence

<220>
<221> source
<222> 1..44
<223> /mol_type="DNA"
/note="oligonucleotide primer"
/organism="Artificial Sequence"

<400> 5
ggccggatcc cctcaggagt ggtagttgat cggatcgagg atgt 44

<210> 6
<211> 9450
<212> DNA
<213> Artificial Sequence

<220>
<221> source
<222> 1..9450
<223> /mol_type="DNA"
      /note="pBeloBAC11_pSET152-C31"
      /organism="Artificial Sequence"

<400> 6
cggcgtcgag atgcatccta ggtgagatta tcaaaaagga tcttcaccta gatccttttg      60

gttcatgtgc agctccatca gcaaaagggg atgataagtt tatcaccacc gactatttgc     120

aacagcgccg gtgatcgtgc tatgatcgac tgatgtcatc agcggtggag tgcaatgtcg     180

tgcaatacga atggcgaaaa gccgagctca tcggtcagct tctcaacctt ggggttaccc     240

ccggcggtgt gctgctggtc cacagctcct tccgtagcgt ccggcccctc gaagatgggc     300

cacttggact gatcgaggcc ctgcgtgctg cgctgggtcc gggagggacg ctcgtcatgc     360

cctcgtggtc aggtctggac gacgagccgt tcgatcctgc cacgtcgccc gttacaccgg     420

accttggagt tgtctctgac acattctggc gcctgccaaa tgtaaagcgc agcgcccatc     480

catttgcctt tgcggcagcg gggccacagg cagagcagat catctctgat ccattgcccc     540

tgccacctca ctcgcctgca agcccggtcg cccgtgtcca tgaactcgat gggcaggtac     600

ttctcctcgg cgtgggacac gatgccaaca cgacgctgca tcttgccgag ttgatggcaa     660

aggttcccta tggggtgccg agacactgca ccattcttca ggatggcaag ttggtacgcg     720

tcgattatct cgagaatgac cactgctgtg agcgctttgc cttggcggac aggtggctca     780

aggagaagag ccttcagaag gaaggtccag tcggtcatgc ctttgctcgg ttgatccgct     840

cccgcgacat tgtggcgaca gccctgggtc aactgggccg agatccgttg atcttcctgc     900

atccgccaga ggcgggatgc gaagaatgcg atgccgctcg ccagtcgatt ggctgagctc     960

atgagcggag aacgagatga cgttggaggg gcaaggtcgc gctgattgct ggggcaacac    1020

gtggagcgga tcggggattg tctttcttca gctcgctgat gatatgctga cgctcaatgc    1080

cgtttggcct ccgactaacg aaaatcccgc atttggacgg ctgatccgat tggcacggcg    1140

gacggcgaat ggcggagcag acgctcgtcc gggggcaatg agatatgaaa aagcctgaac    1200

tcaccgcgac gtatcgggcc ctggccagct agctagagtc gacctgcagg tccccgggga    1260

tcggtcttgc cttgctcgtc ggtgatgtac ttcaccagct tcgcgaagtc gctcttcttg    1320

atggagcgca tggggacgtg cttggcaatc acgcgcaccc cccggccgtt ttagcggcta    1380

aaaaagtcat ggctctgccc tcgggcggac cacgcccatc atgaccttgc caagctcgtc    1440

ctgcttctct tcgatcttcg ccagcagggc gaggatcgtg gcatcaccga accgcgccgt    1500

gcgcgggtcg tcggtgagcc agagtttcag caggccgccc aggcggccca ggtcgccatt    1560

gatgcgggcc agctcgcgga cgtgctcata gtccacgacg cccgtgattt tgtagccctg    1620

gccgacggcc agcaggtagg ccgacaggct catgccggcc gccgccgcct tttcctcaat    1680

cgctcttcgt tcgtctggaa ggcagtacac cttgataggt gggctgccct tcctggttgg    1740

cttggtttca tcagccatcc gcttgccctc atctgttacg ccggcggtag ccggccagcc 1800

tcgcagagca ggattcccgt tgagcaccgc caggtgcgaa taagggacag tgaagaagga 1860

acacccgctc gcgggtgggc ctacttcacc tatcctgccc ggctgacgcc gttggataca 1920

ccaaggaaag tctacacgaa ccctttggca aaatcctgta tatcgtgcga aaaaggatgg 1980

atataccgaa aaaatcgcta taatgacccc gaagcagggt tatgcagcgg aaaagatccg 2040

tcgacctgca ggcatgcaag ctctagcgat tgccatgacg tcccgaaggc gtggcgcggc 2100

ttccccgtgc cggagcaatc gccctgggtg ggttacacga cgcccctcta tggcccgtac 2160

tgacggacac accgaagccc cggcggcaac cctcagcgga tgccccgggg cttcacgttt 2220

tcccaggtca gaagcggttt tcgggagtag tgccccaact ggggtaacct ttgagttctc 2280

tcagttgggg gcgtagggtc gccgacatga cacaaggggt tgtgaccggg gtggacacgt 2340

acgcgggtgc ttacgaccgt cagtcgcgcg agcgcgaaat tcgagcgcag caagcccagc 2400

gacacagcgt agcgccaacg aagacaaggc ggccgacctt cagcgcgaag tcgagcgcga 2460

cgggggccgg ttcaggttcg tcgggcattt cagcgaagcg ccgggcacgt cggcgttcgg 2520

gacggcggag cgcccggagt tccttcccct tgaccaatgg ttccccgaag acgccgacgc 2580

tgacccgacc ggccctaagt cgtggtgggg gcgcgcgtca gtagacgaca agcgcgtgtt 2640

cgtcgggctc ttcgtagaca agatcgttgt cacgaagtcg actacgggca gggggcaggg 2700

aacgcccatc gagaagcgcg cttcgatcac gtgggcgaag ccgccgaccg acgacgacga 2760

agacgacgcc caggacggca cggaagacgt agcggcgtag cgagacaccc gggaagcctg 2820

atctacgtct gtcgagaagt ttctgatcga aaagttcgac agcgtctccg acctgatgca 2880

gctctcggag ggcgaagaat ctcgtgcttt cagcttcgat gtaggagggc gtggatatgt 2940

cctgcgggta aatagctgcg ccgatggttt ctacaaagat cgttatgttt atcggcactt 3000

tgcatcggcc gcgctcccga ttccggaagt gcttgacatt ggggaatttg gatccgtgca 3060

cctcgacccg gccaattctc atgtttgaca gcttatcatc gaatttctgc cattcatccg 3120

cttattatca cttattcagg cgtagcaacc aggcgtttaa gggcaccaat aactgcctta 3180

aaaaaattac gccccgccct gccactcatc gcagtactgt tgtaattcat taagcattct 3240

gccgacatgg aagccatcac aaacggcatg atgaacctga atcgccagcg gcatcagcac 3300

cttgtcgcct tgcgtataat atttgcccat ggtgaaaacg ggggcgaaga agttgtccat 3360

attggccacg tttaaatcaa aactggtgaa actcacccag ggattggctg agacgaaaaa 3420

catattctca ataaaccctt tagggaaata ggccaggttt tcaccgtaac acgccacatc 3480

ttgcgaatat atgtgtagaa actgccggaa atcgtcgtgg tattcactcc agagcgatga 3540

aaacgtttca gtttgctcat ggaaaacggt gtaacaaggg tgaacactat cccatatcac 3600

cagctcaccg tctttcattg ccatacggaa ttccggatga gcattcatca ggcgggcaag 3660

aatgtgaata aaggccggat aaaacttgtg cttatttttc tttacggtct ttaaaaaggc 3720

cgtaatatcc agctgaacgg tctggttata ggtacattga gcaactgact gaaatgcctc 3780

aaaatgttct ttacgatgcc attgggatat atcaacggtg gtatatccag tgattttttt 3840

ctccatttta gcttccttag ctcctgaaaa tctcgataac tcaaaaaata cgcccggtag 3900

tgatcttatt tcattatggt gaaagttgga acctcttacg tgccgatcaa cgtctcattt 3960

tcgccaaaag ttggcccagg gcttcccggt atcaacaggg acaccaggat ttatttattc 4020

tgcgaagtga tcttccgtca caggtattta ttcgcgataa gctcatggag cggcgtaacc 4080

gtcgcacagg aaggacagag aaagcgcgga tctgggaagt gacggacaga acggtcagga 4140

cctggattgg ggaggcggtt gccgccgctg ctgctgacgg tgtgacgttc tctgttccgg 4200

tcacaccaca tacgttccgc cattcctatg cgatgcacat gctgtatgcc ggtataccgc 4260

tgaaagttct gcaaagcctg atgggacata agtccatcag ttcaacggaa gtctacacga 4320

aggtttttgc gctggatgtg gctgcccggc accgggtgca gtttgcgatg ccggagtctg 4380

atgcggttgc gatgctgaaa caattatcct gagaataaat gccttggcct ttatatggaa 4440

atgtggaact gagtggatat gctgtttttg tctgttaaac agagaagctg gctgttatcc 4500

actgagaagc gaacgaaaca gtcgggaaaa tctcccatta tcgtagagat ccgcattatt 4560

aatctcagga gcctgtgtag cgtttatagg aagtagtgtt ctgtcatgat gcctgcaagc 4620

ggtaacgaaa acgatttgaa tatgccttca ggaacaatag aaatcttcgt gcggtgttac 4680

gttgaagtgg agcggattat gtcagcaatg gacagaacaa cctaatgaac acagaaccat 4740

gatgtggtct gtccttttac agccagtagt gctcgccgca gtcgagcgac agggcgaagc 4800

cctcgagtga gcgaggaagc accagggaac agcacttata tattctgctt acacacgatg 4860

cctgaaaaaa cttcccttgg ggttatccac ttatccacgg ggatattttt ataattattt 4920

tttttatagt ttttagatct tcttttttag agcgccttgt aggcctttat ccatgctggt 4980

tctagagaag gtgttgtgac aaattgccct ttcagtgtga caaatcaccc tcaaatgaca 5040

gtcctgtctg tgacaaattg cccttaaccc tgtgacaaat tgccctcaga agaagctgtt 5100

ttttcacaaa gttatccctg cttattgact cttttttatt tagtgtgaca atctaaaaac 5160

ttgtcacact tcacatggat ctgtcatggc ggaaacagcg gttatcaatc acaagaaacg 5220

taaaaatagc ccgcgaatcg tccagtcaaa cgacctcact gaggcggcat atagtctctc 5280

ccgggatcaa aaacgtatgc tgtatctgtt cgttgaccag atcagaaaat ctgatggcac 5340

cctacaggaa catgacggta tctgcgagat ccatgttgct aaatatgctg aaatattcgg 5400

attgacctct gcggaagcca gtaaggatat acggcaggca ttgaagagtt tcgcggggaa 5460

ggaagtggtt ttttatcgcc ctgaagagga tgccggcgat gaaaaaggct atgaatcttt 5520

tccttggttt atcaaacgtg cgcacagtcc atccagaggg ctttacagtg tacatatcaa 5580

cccatatctc attcccttct ttatcgggtt acagaaccgg tttacgcagt ttcggcttag 5640

tgaaacaaaa gaaatcacca atccgtatgc catgcgttta tacgaatccc tgtgtcagta 5700

tcgtaagccg gatggctcag gcatcgtctc tctgaaaatc gactggatca tagagcgtta 5760

ccagctgcct caaagttacc agcgtatgcc tgacttccgc cgccgcttcc tgcaggtctg 5820

tgttaatgag atcaacagca gaactccaat gcgcctctca tacattgaga aaaagaaagg 5880

ccgccagacg actcatatcg tattttcctt ccgcgatatc acttccatga cgacaggata 5940

gtctgagggt tatctgtcac agatttgagg gtggttcgtc acatttgttc tgacctactg 6000

agggtaattt gtcacagttt tgctgtttcc ttcagcctgc atggattttc tcatactttt 6060

tgaactgtaa tttttaagga agccaaattt gagggcagtt tgtcacagtt gatttccttc 6120

tctttccctt cgtcatgtga cctgatatcg ggggttagtt cgtcatcatt gatgagggtt 6180

gattatcaca gtttattact ctgaattggc tatccgcgtg tgtacctcta cctggagttt 6240

ttcccacggt ggatatttct tcttgcgctg agcgtaagag ctatctgaca gaacagttct 6300

tctttgcttc ctcgccagtt cgctcgctat gctcggttac acggctgcgg cgagcgctag 6360

tgataataag tgactgaggt atgtgctctt cttatctcct tttgtagtgt tgctcttatt 6420

ttaaacaact ttgcggtttt ttgatgactt tgcgattttg ttgttgcttt gcagtaaatt 6480

gcaagattta ataaaaaaac gcaaagcaat gattaaagga tgttcagaat gaaactcatg 6540

gaaacactta accagtgcat aaacgctggt catgaaatga cgaaggctat cgccattgca 6600

cagtttaatg atgacagccc ggaagcgagg aaaataaccc ggcgctggag aataggtgaa 6660

gcagcggatt tagttggggt ttcttctcag gctatcagag atgccgagaa agcagggcga 6720

ctaccgcacc cggatatgga aattcgagga cgggttgagc aacgtgttgg ttatacaatt 6780

gaacaaatta atcatatgcg tgatgtgttt ggtacgcgat tgcgacgtgc tgaagacgta 6840

tttccaccgg tgatcggggt tgctgcccat aaaggtggcg tttacaaaac ctcagtttct 6900

gttcatcttg ctcaggatct ggctctgaag gggctacgtg ttttgctcgt ggaaggtaac 6960

gacccccagg gaacagcctc aatgtatcac ggatgggtac cagatcttca tattcatgca 7020

gaagacactc tcctgccttt ctatcttggg gaaaaggacg atgtcactta tgcaataaag 7080

cccacttgct ggccggggct tgacattatt ccttcctgtc tggctctgca ccgtattgaa 7140

actgagttaa tgggcaaatt tgatgaaggt aaactgccca ccgatccaca cctgatgctc 7200

cgactggcca ttgaaactgt tgctcatgac tatgatgtca tagttattga cagcgcgcct 7260

aacctgggta tcggcacgat taatgtcgta tgtgctgctg atgtgctgat tgttcccacg 7320

cctgctgagt tgtttgacta cacctccgca ctgcagtttt tcgatatgct tcgtgatctg 7380

ctcaagaacg ttgatcttaa agggttcgag cctgatgtac gtattttgct taccaaatac 7440

agcaatagta atggctctca gtccccgtgg atggaggagc aaattcggga tgcctgggga 7500

agcatggttc taaaaaatgt tgtacgtgaa acggatgaag ttggtaaagg tcagatccgg 7560

atgagaactg tttttgaaca ggccattgat caacgctctt caactggtgc ctggagaaat 7620

gctctttcta tttgggaacc tgtctgcaat gaaattttcg atcgtctgat taaaccacgc 7680

tgggagatta gataatgaag cgtgcgcctg ttattccaaa acatacgctc aatactcaac 7740

cggttgaaga tacttcgtta tcgacaccag ctgccccgat ggtggattcg ttaattgcgc 7800

gcgtaggagt aatggctcgc ggtaatgcca ttactttgcc tgtatgtggt cgggatgtga 7860

agtttactct tgaagtgctc cggggtgata gtgttgagaa gacctctcgg gtatggtcag 7920

gtaatgaacg tgaccaggag ctgcttactg aggacgcact ggatgatctc atcccttctt 7980

ttctactgac tggtcaacag acaccggcgt tcggtcgaag agtatctggt gtcatagaaa 8040

ttgccgatgg gagtcgccgt cgtaaagctg ctgcacttac cgaaagtgat tatcgtgttc 8100

tggttggcga gctggatgat gagcagatgg ctgcattatc cagattgggt aacgattatc 8160

gcccaacaag tgcttatgaa cgtggtcagc gttatgcaag ccgattgcag aatgaatttg 8220

ctggaaatat ttctgcgctg gctgatgcgg aaaatatttc acgtaagatt attacccgct 8280

gtatcaacac cgccaaattg cctaaatcag ttgttgctct tttttctcac cccggtgaac 8340

tatctgcccg gtcaggtgat gcacttcaaa aagcctttac agataaagag gaattactta 8400

agcagcaggc atctaacctt catgagcaga aaaaagctgg ggtgatattt gaagctgaag 8460

aagttatcac tcttttaact tctgtgctta aaacgtcatc tgcatcaaga actagtttaa 8520

gctcacgaca tcagtttgct cctggagcga cagtattgta taagggcgat aaaatggtgc 8580

ttaacctgga caggtctcgt gttccaactg agtgtataga gaaaattgag gccattctta 8640

aggaacttga aaagccagca ccctgatgcg accacgtttt agtctacgtt tatctgtctt 8700

tacttaatgt cctttgttac aggccagaaa gcataactgg cctgaatatt ctctctgggc 8760

ccactgttcc acttgtatcg tcggtctgat aatcagactg ggaccacggt cccactcgta 8820

tcgtcggtct gattattagt ctgggaccac ggtcccactc gtatcgtcgg tctgattatt 8880

agtctgggac cacggtccca ctcgtatcgt cggtctgata atcagactgg gaccacggtc 8940

ccactcgtat cgtcggtctg attattagtc tgggaccatg gtcccactcg tatcgtcggt 9000

ctgattatta gtctgggacc acggtcccac tcgtatcgtc ggtctgatta ttagtctgga 9060

accacggtcc cactcgtatc gtcggtctga ttattagtct gggaccacgg tcccactcgt 9120

atcgtcggtc tgattattag tctgggacca cgatcccact cgtgttgtcg gtctgattat 9180

cggtctggga ccacggtccc acttgtattg tcgatcagac tatcagcgtg agactacgat 9240

tccatcaatg cctgtcaagg gcaagtattg acatgtcgtc gtaacctgta gaacggagta 9300

acctcggtgt gcggttgtat gcctgctgtg gattgctgct gtgtcctgct tatccacaac 9360

attttgcgca cggttatgtg gacaaaatac ctggttaccc aggccgtgcc ggcacgttaa 9420

ccgggctgca tccgatgcaa gtgtgtcgct 9450

<210> 7
<211> 30
<212> DNA
<213> Artificial Sequence

<220>
<221> source
<222> 1..30
<223> /mol_type="DNA"
      /note="oligonucleotide primer"
      /organism="Artificial Sequence"

<400> 7
gcgctctaga gcgcttgtag tcgatggcct                                        30


<210> 8
<211> 34
<212> DNA
<213> Artificial Sequence

<220>
<221> source
<222> 1..34
<223> /mol_type="DNA"
      /note="oligonucleotide primer"
      /organism="Artificial Sequence"

<400> 8
gcggcatatg gttgacatct tttgccgatt ctgg                                   34


<210> 9
<211> 37
<212> DNA
<213> Artificial Sequence

<220>
<221> source
<222> 1..37
<223> /mol_type="DNA"
      /note="oligonucleotide primer"
      /organism="Artificial Sequence"

<400> 9
cgcgtctaga ggatcttcac ctagatcctt ttggttc                                37


<210> 10
<211> 28
<212> DNA
<213> Artificial Sequence

<220>
<221> source
<222> 1..28
<223> /mol_type="DNA"
      /note="oligonucleotide primer"
      /organism="Artificial Sequence"

<400> 10
ccgggaattc cccgatccgc tccacgtg                                          28


<210> 11

<211> 31
<212> DNA
<213> Artificial Sequence

<220>
<221> source
<222> 1..31
<223> /mol_type="DNA"
      /note="oligonucleotide primer"
      /organism="Artificial Sequence"

<400> 11
gcgcgaattc ccaggatgga cttcgcggtg t                                    31


<210> 12
<211> 31
<212> DNA
<213> Artificial Sequence

<220>
<221> source
<222> 1..31
<223> /mol_type="DNA"
      /note="oligonucleotide primer"
      /organism="Artificial Sequence"

<400> 12
ggccggatcc cgggcatggg aagacctcct t                                    31


<210> 13
<211> 31
<212> DNA
<213> Artificial Sequence

<220>
<221> source
<222> 1..31
<223> /mol_type="DNA"
      /note="oligonucleotide primer"
      /organism="Artificial Sequence"

<400> 13
cggcggatcc acggccttca gctctgggtc a                                    31


<210> 14
<211> 31
<212> DNA
<213> Artificial Sequence

<220>
<221> source
<222> 1..31
<223> /mol_type="DNA"
      /note="oligonucleotide primer"
      /organism="Artificial Sequence"

<400> 14
gccgaagctt accgttgtcg atgagcgcct t                                    31


<210> 15
<211> 30

<212> DNA
<213> Artificial Sequence

<220>
<221> source
<222> 1..30
<223> /mol_type="DNA"
      /note="oligonucleotide primer"
      /organism="Artificial Sequence"

<400> 15
acgatgatgc tacccaccag taataagttg                                        30


<210> 16
<211> 26
<212> DNA
<213> Artificial Sequence

<220>
<221> source
<222> 1..26
<223> /mol_type="DNA"
      /note="oligonucleotide primer"
      /organism="Artificial Sequence"

<400> 16
gacataggcg agtccgttga agtcct                                            26


<210> 17
<211> 39
<212> DNA
<213> Artificial Sequence

<220>
<221> source
<222> 1..39
<223> /mol_type="DNA"
      /note="oligonucleotide primer"
      /organism="Artificial Sequence"

<400> 17
ccaactgggg taacctcgag ttctctcagt tgggggcgt                               39


<210> 18
<211> 39
<212> DNA
<213> Artificial Sequence

<220>
<221> source
<222> 1..39
<223> /mol_type="DNA"
      /note="oligonucleotide primer"
      /organism="Artificial Sequence"

<400> 18
acgcccccaa ctgagagaac tcgaggttac cccagttgg                               39


<210> 19
<211> 33
<212> DNA

<213> Artificial Sequence

<220>
<221> source
<222> 1..33
<223> /mol_type="DNA"
      /note="oligonucleotide primer"
      /organism="Artificial Sequence"

<400> 19
aatgattaaa ggatgttcag aatgaaactc atg                                    33


<210> 20
<211> 31
<212> DNA
<213> Artificial Sequence

<220>
<221> source
<222> 1..31
<223> /mol_type="DNA"
      /note="oligonucleotide primer"
      /organism="Artificial Sequence"

<400> 20
gggactgaga gccattacta ttgctgtatt t                                      31


<210> 21
<211> 27
<212> DNA
<213> Artificial Sequence

<220>
<221> source
<222> 1..27
<223> /mol_type="DNA"
      /note="oligonucleotide primer"
      /organism="Artificial Sequence"

<400> 21
agttgtgaag gagacatcgt gtcatgg                                           27


<210> 22
<211> 27
<212> DNA
<213> Artificial Sequence

<220>
<221> source
<222> 1..27
<223> /mol_type="DNA"
      /note="oligonucleotide primer"
      /organism="Artificial Sequence"

<400> 22
tcgcactctg cattatttcc catagag                                           27


<210> 23
<211> 30
<212> DNA
<213> Artificial Sequence

<220>
<221> source
<222> 1..30
<223> /mol_type="DNA"
      /note="oligonucleotide primer"
      /organism="Artificial Sequence"

<400> 23
gccgcatatg gtggagcgca tcgactcgca                                        30


<210> 24
<211> 37
<212> DNA
<213> Artificial Sequence

<220>
<221> source
<222> 1..37
<223> /mol_type="DNA"
      /note="oligonucleotide primer"
      /organism="Artificial Sequence"

<400> 24
cgcgtctaga cgatgtctcc ttcacaactg ggttctg                                37


<210> 25
<211> 32
<212> DNA
<213> Artificial Sequence

<220>
<221> source
<222> 1..32
<223> /mol_type="DNA"
      /note="oligonucleotide primer"
      /organism="Artificial Sequence"

<400> 25
gccgtctaga accgaccagg tctgacaccc gt                                     32


<210> 26
<211> 35
<212> DNA
<213> Artificial Sequence

<220>
<221> source
<222> 1..35
<223> /mol_type="DNA"
      /note="oligonucleotide primer"
      /organism="Artificial Sequence"

<400> 26
gccggaattc ggtcatcggt tccgtatcct gtacc                                  35


<210> 27
<211> 29
<212> DNA
<213> Artificial Sequence

<220>
<221> source
<222> 1..29
<223> /mol_type="DNA"
      /note="oligonucleotide primer"
      /organism="Artificial Sequence"

<400> 27
cggccatatg atgccatccg cactccagg                                          29


<210> 28
<211> 32
<212> DNA
<213> Artificial Sequence

<220>
<221> source
<222> 1..32
<223> /mol_type="DNA"
      /note="oligonucleotide primer"
      /organism="Artificial Sequence"

<400> 28
gccgtctaga ggtgtcagac ctggtcggtg gg                                      32


<210> 29
<211> 31
<212> DNA
<213> Artificial Sequence

<220>
<221> source
<222> 1..31
<223> /mol_type="DNA"
      /note="oligonucleotide primer"
      /organism="Artificial Sequence"

<400> 29
gcgccatatg atgatgaacg aggcagcgcc t                                       31


<210> 30
<211> 32
<212> DNA
<213> Artificial Sequence

<220>
<221> source
<222> 1..32
<223> /mol_type="DNA"
      /note="oligonucleotide primer"
      /organism="Artificial Sequence"

<400> 30
gcgctctaga ctagccctcg gtgaccgtga tg                                      32


<210> 31
<211> 33
<212> DNA
<213> Artificial Sequence

<220>

<221> source
<222> 1..33
<223> /mol_type="DNA"
      /note="oligonucleotide primer"
      /organism="Artificial Sequence"

<400> 31
ccggcatatg gtgattgccc tgcgcgagag aac                                    33


<210> 32
<211> 30
<212> DNA
<213> Artificial Sequence

<220>
<221> source
<222> 1..30
<223> /mol_type="DNA"
      /note="oligonucleotide primer"
      /organism="Artificial Sequence"

<400> 32
gcgctctaga acggggacgg cctaggcggt                                        30


<210> 33
<211> 36
<212> DNA
<213> Artificial Sequence

<220>
<221> source
<222> 1..36
<223> /mol_type="DNA"
      /note="oligonucleotide primer"
      /organism="Artificial Sequence"

<400> 33
cgcgcatatg aggagacagg acacgatggt ttgatg                                 36


<210> 34
<211> 35
<212> DNA
<213> Artificial Sequence

<220>
<221> source
<222> 1..35
<223> /mol_type="DNA"
      /note="oligonucleotide primer"
      /organism="Artificial Sequence"

<400> 34
cgcgagatct gatcaccgag gtgtacctct accgg                                  35


<210> 35
<211> 27
<212> DNA
<213> Artificial Sequence

<220>
<221> source

<222> 1..27
<223> /mol_type="DNA"
      /note="oligonucleotide primer"
      /organism="Artificial Sequence"

<400> 35
gccgcatatg gcggcactgt ggtcccg                                          27


<210> 36
<211> 32
<212> DNA
<213> Artificial Sequence

<220>
<221> source
<222> 1..32
<223> /mol_type="DNA"
      /note="oligonucleotide primer"
      /organism="Artificial Sequence"

<400> 36
ggcctctaga tcatgccacc gctcccttca cc                                     32


<210> 37
<211> 17
<212> DNA
<213> Artificial Sequence

<220>
<221> source
<222> 1..17
<223> /mol_type="DNA"
      /note="oligonucleotide primer Scla_claR_F"
      /organism="Artificial Sequence"

<400> 37
acccggctcc acatcac                                                     17


<210> 38
<211> 14
<212> DNA
<213> Artificial Sequence

<220>
<221> source
<222> 1..14
<223> /mol_type="DNA"
      /note="oligonucleotide primer Scla_claR_R"
      /organism="Artificial Sequence"

<400> 38
gagcggcgca ccag                                                        14


<210> 39
<211> 15
<212> DNA
<213> Artificial Sequence

<220>
<221> source
<222> 1..15

<223> /mol_type="DNA"
      /note="oligonucleotide primer Scla_claR_S"
      /organism="Artificial Sequence"

<400> 39
ctcgcccacc caccc                                                        15


<210> 40
<211> 18
<212> DNA
<213> Artificial Sequence

<220>
<221> source
<222> 1..18
<223> /mol_type="DNA"
      /note="oligonucleotide primer Scla_pah2_F"
      /organism="Artificial Sequence"

<400> 40
gcggcgatgg tccagatc                                                     18


<210> 41
<211> 15
<212> DNA
<213> Artificial Sequence

<220>
<221> source
<222> 1..15
<223> /mol_type="DNA"
      /note="oligonucleotide primer Scla_pah2_R"
      /organism="Artificial Sequence"

<400> 41
tcgtccgccg tgacc                                                        15


<210> 42
<211> 16
<212> DNA
<213> Artificial Sequence

<220>
<221> source
<222> 1..16
<223> /mol_type="DNA"
      /note="oligonucleotide primer Scla_pah2_S"
      /organism="Artificial Sequence"

<400> 42
ctcgctcgac tacgcc                                                       16


<210> 43
<211> 15
<212> DNA
<213> Artificial Sequence

<220>
<221> source
<222> 1..15
<223> /mol_type="DNA"

/note="oligonucleotide primer Scla_orf13_F"
/organism="Artificial Sequence"

<400> 43
gccgctggtg ctgac                                                        15


<210> 44
<211> 17
<212> DNA
<213> Artificial Sequence

<220>
<221> source
<222> 1..17
<223> /mol_type="DNA"
/note="oligonucleotide primer Scla_orf13_R"
/organism="Artificial Sequence"

<400> 44
ccggctcgcg atattgc                                                      17


<210> 45
<211> 16
<212> DNA
<213> Artificial Sequence

<220>
<221> source
<222> 1..16
<223> /mol_type="DNA"
/note="oligonucleotide primer Scla_orf13_S"
/organism="Artificial Sequence"

<400> 45
ccgaacgccg ccacca                                                       16


<210> 46
<211> 19
<212> DNA
<213> Artificial Sequence

<220>
<221> source
<222> 1..19
<223> /mol_type="DNA"
/note="oligonucleotide primer Scla_ceaS1_F"
/organism="Artificial Sequence"

<400> 46
cggctacatg gacggcatt                                                    19


<210> 47
<211> 18
<212> DNA
<213> Artificial Sequence

<220>
<221> source
<222> 1..18
<223> /mol_type="DNA"
/note="oligonucleotide primer Scla_ceaS1_R"

      /organism="Artificial Sequence"

<400> 47
gatcgccagg aggaggtc                                                    18


<210> 48
<211> 15
<212> DNA
<213> Artificial Sequence

<220>
<221> source
<222> 1..15
<223> /mol_type="DNA"
      /note="oligonucleotide primer Scla_ceaS1_S"
      /organism="Artificial Sequence"

<400> 48
cacccggccc tcgac                                                       15


<210> 49
<211> 41
<212> DNA
<213> Artificial Sequence

<220>
<221> source
<222> 1..41
<223> /mol_type="DNA"
      /note="oligonucleotide primer"
      /organism="Artificial Sequence"

<400> 49
gcgcgagctc tctgacagtt accaatgctt aatcagtgag g                          41


<210> 50
<211> 34
<212> DNA
<213> Artificial Sequence

<220>
<221> source
<222> 1..34
<223> /mol_type="DNA"
      /note="oligonucleotide primer"
      /organism="Artificial Sequence"

<400> 50
ccgggagctc tcactgacga atcgaggtcg agga                                  34


<210> 51
<211> 41
<212> DNA
<213> Artificial Sequence

<220>
<221> source
<222> 1..41
<223> /mol_type="DNA"
      /note="oligonucleotide primer "
      /organism="Artificial Sequence"

<400> 51
gcgcgagctc tctgacagtt accaatgctt aatcagtgag g                          41


<210> 52
<211> 34
<212> DNA
<213> Artificial Sequence

<220>
<221> source
<222> 1..34
<223> /mol_type="DNA"
      /note="oligonucleotide primer"
      /organism="Artificial Sequence"

<400> 52
ccgggagctc tcactgacga atcgaggtcg agga                                  34


<210> 53
<211> 9742
<212> DNA
<213> Artificial Sequence

<220>
<221> source
<222> 1..9742
<223> /mol_type="DNA"
      /note="pBAC_LEK"
      /organism="Artificial Sequence"

<400> 53
ctcgactgag attatcaaaa aggatcttca cctagatcct tttggttcat gtgcagctcc      60

atcagcaaaa ggggatgata agtttatcac caccgactat ttgcaacagc gccggtgatc     120

gtgctatgat cgactgatgt catcagcggt ggagtgcaat gtcgtgcaat acgaatggcg     180

aaaagccgag ctcatcggtc agcttctcaa ccttggggtt acccccggcg gtgtgctgct     240

ggtccacagc tccttccgta gcgtccggcc cctcgaagat gggccacttg gactgatcga     300

ggccctgcgt gctgcgctgg gtccgggagg gacgctcgtc atgccctcgt ggtcaggtct     360

ggacgacgag ccgttcgatc ctgccacgtc gcccgttaca ccggaccttg gagttgtctc     420

tgacacattc tggcgcctgc caaatgtaaa gcgcagcgcc catccatttg cctttgcggc     480

agcggggcca caggcagagc agatcatctc tgatccattg cccctgccac ctcactcgcc     540

tgcaagcccg gtcgcccgtg tccatgaact cgatgggcag gtacttctcc tcggcgtggg     600

acacgatgcc aacacgacgc tgcatcttgc cgagttgatg gcaaaggttc cctatggggt     660

gccgagacac tgcaccattc ttcaggatgg caagttggta cgcgtcgatt atctcgagaa     720

tgaccactgc tgtgagcgct ttgccttggc ggacaggtgg ctcaaggaga agagccttca     780

gaaggaaggt ccagtcggtc atgcctttgc tcggttgatc cgctcccgcg acattgtggc     840

gacagccctg ggtcaactgg gccgagatcc gttgatcttc ctgcatccgc cagaggcggg     900

atgcgaagaa tgcgatgccg ctcgccagtc gattggctga gctcatgagc ggagaacgag     960

atgacgttgg aggggcaagg tcgcgctgat tgctggggca acacgtggag cggatcgggg 1020

attgtctttc ttcagctcgc tgatgatatg ctgacgctca atgccgtttg gcctccgact 1080

aacgaaaatc ccgcatttgg acggctgatc cgattggcac ggcggacggc gaatggcgga 1140

gcagacgctc gtccgggggc aatgagatat gaaaaagcct gaactcaccg cgacgtatcg 1200

gtccccgggg atcggtcttg ccttgctcgt cggtgatgta cttcaccagc ttgcgaagtc 1260

gctcttcttg atggagcgca tggggacgtg cttggcaatc acgcgcaccc cccggccgtt 1320

ttagcggcta aaaaagtcat ggctctgccc tcgggcggac cacgcccatc atgaccttgc 1380

caagctcgtc ctgcttctct tcgatcttcg ccagcagggc gaggatcgtg gcatcaccga 1440

accgcgccgt gcgcgggtcg tcggtgagcc agagtttcag caggccgccc aggcggccca 1500

ggtcgccatt gatgcgggcc agctcgcgga cgtgctcata gtccacgacg cccgtgattt 1560

tgtagccctg gccgacggcc agcaggtagg ccgacaggct catgccggcc gccgccgcct 1620

tttcctcaat cgctcttcgt tcgtctggaa ggcagtacac cttgataggt gggctgccct 1680

tcctggttgg cttggtttca tcagccatcc gcttgccctc atctgttacg ccggcggtag 1740

ccggccagcc tcgcagagca ggattcccgt tgagcaccgc caggtgcgaa taagggacag 1800

tgaagaagga acacccgctc gcgggtgggc ctacttcacc tatcctgccc ggctgacgcc 1860

gttggataca ccaaggaaag tctacacgaa ccctttggca aaatcctgta tatcgtgcga 1920

aaaaggatgg atataccgaa aaaatcgcta taatgacccc gaagcagggt tatgcagcgg 1980

aaaagatcca gctctagcga ttgccatgac gtcccgaagg cgtggcgcgg cttccccgtg 2040

ccggagcaat cgccctgggt gggttacacg acgcccctct atggcccgta ctgacggaca 2100

caccgaagcc ccggcggcaa ccctcagcgg atgccccggg gcttcacgtt ttcccaggtc 2160

agaagcggtt ttcgggagta gtgccccaac tggggtaacc tttgagttct ctcagttggg 2220

ggcgtagggt cgccgacatg acacaagggg ttgtgaccgg ggtggacacg tacgcgggtg 2280

cttacgaccg tcagtcgcgc gagcgcgaaa ttcgagcgca gcaagcccag cgacacagcg 2340

tagcgccaac gaagacaagg cggccgacct tcagcgcgaa gtcgagcgcg acgggggccg 2400

gttcaggttc gtcgggcatt tcagcgaagc gccgggcacg tcggcgttcg ggacggcgga 2460

gcgcccggag ttggaacgca tcctgaacga atgccgcgcc gggcggctca acatgatcat 2520

tgtctatgac gtgtcgcgct tctcgcgcct gaaggtcatg gacgcgattc cgattgtctc 2580

ggaattgctc gccctgggcg tgacgattgt ttccactcag gaaggcgtct tccggcaggg 2640

aaacgtcatg gacctgattc acctgattat gcggctcgac gcgtcgcaca aagaatcttc 2700

gctgaagtcg gcgaagattc tcgacacgaa gaaccttcag cgcgaattgg gcgggtacgt 2760

cggcgggaag gcgccttacg gcttcgagct tgtttcggag acgaaggaga tcacgcgcaa 2820

cggccgaatg gtcaatgtcg tcatcaacaa gctcgcgcac tcgaccactc cccttaccgg 2880

acccttcgag ttcgagcccg acgtaatccg gtggtggtgg cgtgagatca agacgcacaa 2940

acaccttccc ttcaagccgg gcagtcaagc cgccattcac ccgggcagca tcacggggct 3000

ttgtaagcgc atggacgctg acgccgtgcc gacccggggc gagacgattg ggaagaagac 3060

cgcttcaagc gcctgggacc cggcaaccgt tatgcgaatc cttcgggacc cgcgtattgc 3120

gggcttcgcc gctgaggtga tctacaagaa gaagccggac ggcacgccga ccacgaagat 3180

tgagggttac cgcattcagc gcgacccgat cacgctccgg ccggtcgagc ttgattgcgg 3240

accgatcatc gagcccgctg agtggtatga gcttcaggcg tggttggacg gcagggggcg 3300

cggcaagggg ctttcccggg ggcaagccat tctgtccgcg atggacaagc tgtactgcga 3360

gtgtggcgcc gtcatgacta gcaagcgcgg ggaagagtcg atcaaggact cttaccgctg 3420

ccgtcgccgg aaggtggtgg acccgtccgc acctgggcag cacgaaggca cgtgcaacgt 3480

cagcatggcg gcactcgaca agttcgttgc ggaacgcatc ttcaacaaga tcaggcacgc 3540

cgaaggcgac gaagagacgt tggcgcttct gtgggaagcc gcccgacgct tcggcaagct 3600

cactgaggcg cctgagaaga gcggcgaacg ggcgaacctt gttgcggagc gggccgacgc 3660

cctgaacgcc cttgaagagc tgtacgaaga ccgcgcggca ggcgcgtacg acggacccgt 3720

tggcaggaag cacttccgca agcaacaggc agcgctgacg ctccggcagc aaggggcgga 3780

agagcggctt gccgaacttg aagccgccga agccccgaag ctcccccttg accaatggtt 3840

ccccgaagac gccgacgctg acccgaccgg ccctaagtcg tggtggggcc gcgcgtcagt 3900

agacgacaag cgcgtgttcg tcgggctctt cgtagacaag atcgttgtca cgaagtccac 3960

tacgggcagg gggcagggaa cgcccatcga gaagcgggct tcgatcacgt gggcgaagcc 4020

gccgaccgac gacgacgaag acgacgccca ggacggcacg gaagacgtag cggcgtagcg 4080

agacacccgg gaagcctgat ctacgtctgt cgagaagttt ctgatcgaaa agttcgacag 4140

cgtctccgac ctgatgcagc tctcggaggg cgaagaatct cgtgctttca gcttcgatgt 4200

aggagggcgt ggatatgtcc tgcgggtaaa tagctgcgcc gatggtttct acaaagatcg 4260

ttatgtttat cggcactttg catcggccgc gctcccgatt agatctctta agatcgatga 4320

tatcaagctt ggtacccata tggtcgaccc tgaggttcga aactagtgct ctagacaatt 4380

gaacgttcag ctgtccggac cggttaactc gcgaacctgg ttggccatac gtactgaaag 4440

ttcgtgcacg gcgcgccgcg atcgccctag gggatccatg catgctagcg tatacggccg 4500

gccgaattcg cggccgctta attaatgttt aaacccgcgg cctgcaggag tactgcccgg 4560

gcgcaaagcc tgatgggaca taagtccatc agttcaacgg aagtctacac gaaggttttt 4620

gcgctggatg tggctgcccg gcaccgggtg cagtttgcga tgccggagtc tgatgcggtt 4680

gcgatgctga aacaattatc ctgagaataa atgccttggc ctttatatgg aaatgtggaa 4740

ctgagtggat atgctgtttt tgtctgttaa acagagaagc tggctgttat ccactgagaa 4800

gcgaacgaaa cagtcgggaa aatctcccat tatcgtagag atccgcatta ttaaactcag 4860

gagcctgtgt agcgtttata ggaagtagtg ttctgtcatg atgcctgcaa gcggtaacga 4920

aaacgatttg aatatgcctt caggaacaat agaaatcttc gtgcggtgtt acgttgaagt 4980

ggagcggatt atgtcagcaa tggacagaac aacctaatga acacagaacc atgatgtggt 5040

ctgtcctttt acagccagta gtgctcgccg cagtcgagcg acagggcgaa gccctcgagt 5100

gagcgaggaa gcaccaggga acagcactta tatattctgc ttacacacga tgcctgaaaa 5160

aacttccctt ggggttatcc acttatccac ggggatattt ttataattat tttttttata 5220

gtttttagat attctttttt agagcgcctt gtaggccttt atccatgctg gtttagagaa 5280

ggtgttgtga caaattgccc tttcagtgtg acaaatcacc ctcaaatgac agtcctgtct 5340

gtgacaaatt gcccttaacc ctgtgacaaa ttgccctcag aagaagctgt tttttcacaa 5400

agttatccct gcttattgac tcttttttat ttagtgtgac aatctaaaaa cttgtcacac 5460

ttcacatgga tctgtcatgg cggaaacagc ggttatcaat cacaagaaac gtaaaaatag 5520

cccgcgaatc gtccagtcaa acgacctcac tgaggcggca tatagtctct cccgggatca 5580

aaaacgtatg ctgtatctgt tcgttgacca gatcagaaaa tctgatggca ccctacagga 5640

acatgacggt atctgcgaga tccatgttgc taaatatgct gaaatattcg gattgacctc 5700

tgcggaagcc agtaaggata tacggcaggc attgaagagt ttcgcgggga aggaagtggt 5760

tttttatcgc cctgaagagg atgccggcga tgaaaaaggc tatgaatctt ttccttggtt 5820

tatcaaacgt gcgcacagtc catccagagg gctttacagt gtacatatca acccatatct 5880

cattcccttc tttatcgggt tacagaaccg gtttacgcag tttcggctta gtgaaacaaa 5940

agaaatcacc aatccgtatg ccatgcgttt atacgaatcc ctgtgtcagt atcgtaagcc 6000

ggatggctca ggcatcgtct ctctgaaaat cgactggatc atagagcgtt accagctccc 6060

tcaaagttac cagcgtatgc ctgacttccg ccgccgcttc ctccaggtct gtgttaatga 6120

gatcaacagc agaactccaa tgcgcctctc atacattgag aaaaagaaag gccgccagac 6180

gactcatatc gtattttcct tccgcgatat aacttccatg acgacaggat agtctgaggg 6240

ttatctgtca cagatttgag ggtggttcgt cacatttgtt ctgacctact gagggtaatt 6300

tgtcacagtt ttgctgtttc cttcagcctg catggatttt ctcatacttt ttgaactgta 6360

atttttaagg aagccaaatt tgagggcagt ttgtcacagt tgatttcctt ctctttccct 6420

tcgtcatgtg acctgatatg gggggttagt tcgtcatcat tgatgagggt tgattatcac 6480

agtttattac tctgaattgg ctatccgcgt gtgtacctct acctggagtt tttcccacgg 6540

tggatatttc ttcttgcgct gagcgtaaga gctatctgac agaacagttc ttctttgctt 6600

cctcgccagt tcgctcgcta tgctcggtta cacggctgcg gcgagcgcta gtgataataa 6660

gtgactgagg tatgtgctct tcttatctcc ttttgtagtg ttgctcttat attaaacaac 6720

tttgcggttt tttgatgact ttgcgatttt gttgttgctt tgcagtaaat tgcaagattt 6780

aataaaaaaa cgcaaagcaa tgattaaagg atgttcagaa tgaaactcat ggaaacactt 6840
aaccagtgca taaacgctgg tcatgaaatg acgaaggcta tcgccattgc acagtttaat 6900
gatgacagcc cggaagcgag gaaaataacc cggcgctgga gaataggtga agcagcggat 6960
ttagttgggg tttcttctca ggctatcaga gatgccgaga aagcagggcg actaccgcac 7020
ccggatatgg aaattcgagg acgggttgag caacgtgttg gttatacaat agaacaaata 7080
aatcacatgc gtgatgtgtt tggtacgcga ttgcgacgtg ctgaagacgt atttccaccg 7140
gtgatcgggg ttgctgccca taaaggtggc gtttacaaaa cctcagtttc tgttcatctt 7200
gctcaggatc tggctctgaa ggggctacgt gttttgctcg tggaaggtaa cgacccccag 7260
ggaacagcct caatgtatca cggatgggtt cccgatcttc atattcatgc agaagacact 7320
ctcctgcctt tctatcttgg ggaaaaggac gatgtcactt atgcaataaa gcccacttgc 7380
tggccggggc ttgacattat tccttcctgt ctggctctgc accgtattga aactgagtta 7440
atgggcaaat ttgatgaagg taaactgccc accgatccac acctgatgct ccgactggcg 7500
attgaaactg ttgctcatga ctatgatgtc atagttattg acagcgcccc taacctgggt 7560
atcggcacga taaatgtcgt atgtgctgct gatgtgctga ttgttcccac gcctgctgag 7620
ttgtttgact acacctccgc acttcagttt ttcgatatgc ttcgtgatct gctcaagaac 7680
gtcgatctta aagggttcga gcctgatgtt cgtattttgc ttaccaaata cagcaatagt 7740
aatggctctc agtccccgtg gatggaggag caaattcggg atgcctgggg aagcatggtt 7800
ctaaaaaatg ttgtacgtga aacggatgaa gttggtaaag gtcagatacg gatgagaact 7860
gtttttgaac aggccattga tcaacgctct tcaactggtg cctggagaaa tgctctttct 7920
atttgggaac ctgtctgcaa tgaaattttc gaccgtctga ttaaaccacg ctgggagatt 7980
agataatgaa gcgtgcgcct gttattccaa aacatacgct caatactcaa ccggttgaag 8040
atacttcgtt atcgacacca gcagccccga tggtggattc gttaattgcc cgcgtaggag 8100
taatggctcg cggtaatgcc attactttgc ctgtatgtgg tcgggatgtg aagtttactc 8160
ttgaagtgct ccggggtgat agtgttgaga agacctctcg ggtatggtca ggtaatgaac 8220
gtgaccagga gctgcttact gaggacgcac tggatgatct catcccttct tttctactga 8280
ctggtcaaca gacaccggcg ttcggtcgaa gagtatctgg tgtcatagaa attgccgatg 8340
ggagtcgccg tcgtaaagct gctgcactta ccgaaagtga ttatcgtgtt ctggttggcg 8400
agctggatga tgagcagatg gctgcattat ccagattggg taacgattat cgcccaacaa 8460
gtgcttatga acgtggtcag cgttatgcaa gccgattgca gaatgaattt gctggaaata 8520
tttctgcgct ggctgatgcg gaaaatattt cacgtaagat tattacccgc tgtatcaaca 8580
ccgccaaatt gcctaaatca gttgttgctc ttttttctca ccccggtgaa ctatctgccc 8640
ggtcaggtga tgcacttcaa aaagccttta cagataaaga ggaattactc aagcagcagg 8700
catctaacct tcatgagcag aaaaaagctg gggtgatatt tgaagctgaa gaagttatca 8760

ctcttttaac ttctgtgctt aaaacgtcat ctgcatcaag aacaagttta agctcacgac 8820

atcagtttgc tcctggagcg acagtattgt ataagggcga taaaatggtg cttaacctgg 8880

acaggtctcg tgttccaact gagtgtatag agaaaattga ggccattctc aaggaacttg 8940

aaaagccagc accctgatgc gaccacgttt tagtctacgt ttatctgtct ttacttaatg 9000

tcctttgtta caggccagaa agcataactg gcctgaatat tctctctggg cccactgttc 9060

cacttgtatc gtcggtctga taatcagact gggaccacgg tcccactcgt atcgtcggtc 9120

tgattattag tctgggacca cggtcccact cgtatcgtcg gtctgattat tagtctggga 9180

ccacggtccc actcgtatcg tcggtctgat aatcagactg ggaccacggt cccactcgta 9240

tcgtcggtct gattattagt ctgggaccat ggtcccactc gtatcgtcgg tctgattatt 9300

agtctgggac cacggtccca ctcgtatcgt cggtctgatt attagtctgg aaccacggtc 9360

ccactcgtat cgtcggtctg attattagtc tgggaccacg gtcccactcg tatcgtcggt 9420

ctgattatta gtctgggacc acgatcccac tcgtgttgtc ggtctgatta tcggtctggg 9480

accacggtcc cacttgtatt gtcgatcaga ctatcagcgt gagactacga ttccatcaat 9540

gcctgtcaag ggcaagtatt gacatgtcgt cgtaacctgt agaacggagt aacctcggtg 9600

tgcggttgta tgcctgctgt ggattgctgc tgtgtcctgc ttatccacaa cattttgcgc 9660

acggttatgt ggacaaaata cctgttaccc aggccgtgcc ggcacgttaa accgggctgc 9720

atccgatgca agtgtgtcgc tg 9742

## Description

### Field of the invention

The present invention belongs to the field of molecular biology, microbiology and microbial genetics. Specifically, the present invention relates to methods for targeted cloning of DNA fragments from the genome of a donor organism. Further, the present invention refers to a vector, as well as the use of said vector for cloning and/or transferring of a DNA fragment.

### Decsription of the background art

Bacteria and Actinomycetes (or Actinobacteria) in particular are abundant source of biologically active secondary metabolites. A large number of clinically important secondary metabolites are routinely used for treatment as antibiotics, antifungals and inhibitors of a wide range of metabolic or regulatory functions.

Secondary metabolite gene clusters of Actinobacteria such as Actinobacteria of the genus Actinomyces are large sequences in the genome of these microorganisms which usually contain clustered genetic information for catalytic enzymes, regulatory proteins and auxiliary proteins such as resistance genes, transporters etc. The size of these clusters, especially where very large polyketyde synthase genes or non ribosomal peptide synthase genes are encoded, often exceeds the size of DNA fragments which can be efficiently cloned using current molecular biology tools.

Until now, there are several approaches known for cloning gene fragments of different organisms.

For instance, an existing solution to the size limitation of common cloning vectors for E. coli are Cosmids and BAC (Bacterial artificial chromosome) vectors.

The former (Cosmids) allow construction of random genome libraries consisting of Cosmid clones harbouring approximately 40kb (kilo base) DNA fragments. When a whole cluster cloning is the target of such methods, additional steps of library screening for Cosmid clones containing parts of the target cluster as well as assembly of the identified clones into a functional whole cluster DNA fragment are necessary. The latter, i.e. the BAC vectors, allow cloning of significantly larger fragments of DNA (more than 300kb DNA fragments have been reported), however the step of screening of a BAC library is still a necessary step when targeting a specific gene cluster.

Further, there is a well-known approach of targeted cloning of DNA fragments based on DNA sequence of the desired DNA fragment (the so-called "plasmid rescue approach"), wherein a plasmid with E. coli replication function is integrated into the vicinity of the targeted cluster in the donor microorganism using homologous recombination with a cloned homologous fragment integrated into the said plasmid. Upon stable integration into the donor genome or donor chromosome, respectively, the complete genomic DNA is digested using restriction endonucleases selected on the basis of the available DNA sequence of the region of the targeted genetic cluster. In the next step, a ligation reaction is set up with the digested genomic DNA under conditions promoting intramolecular ligation (cyclisation) and used to transform an E. coli host. The transformants arising from cyclic DNA fragments containing replication functions and resistance markers delivered with the plasmid can be efficiently selected.

With this regard, WO 2009/017692 discloses the cloning and expression of the actinorhodin gene cluster, which is a streptomycetes secondary metabolite biosynthetic gene cluster.

Up to date, the above approaches exhibit significant limitations, e.g. in cloned DNA fragments size (<40kb), which can be stably maintained in E. coli. Further, the known approaches are laborious. So far, there have been no reports of a tool based on a plasmid rescue approach which would facilitate the plasmid rescue method, e.g. in terms of direct transformation of a homologous or heterologous host, such as actinomyces, without additional modification of the plasmid with the cloned DNA fragment.

Despite the above described known approaches and attempts for improving cloning of desired DNA fragments that e.g. carry gene clusters encoding secondary metabolites, there is still a need for an improved cloning method and improved downstream processes such as heterologous expression of the cloned DNA fragment in a broad range of host cells such as bacteria.

### Summary of the invention

The present invention provides the following aspects, subject-matters and preferred embodiments, which respectively taken alone or in combination, contribute to solving the object of the present invention:
(1) A method for targeted cloning and/or transferring of DNA fragments from a donor organism genome into a host cell, the method comprising the following steps:
   (a) Transferring a Bacterial Artificial Chromosome (BAC)-vector into the donor organism, wherein said vector comprises
      (a1) an origin of replication;
      (a2) an origin of transfer;
      (a3) a site-specific recombination system containing a functional integrase and an attP site; (a4) a DNA sequence that is substantially homologous to a desired target integration site in the donor organism and that allows for homologous recombination with a corresponding homologous DNA sequence being present in the genomic DNA of the donor organism; and
      (a5) a selection marker,
      wherein said donor organism does not contain a functional attB site
   (b) Isolating the genomic DNA from the donor organism;
   (c) Transferring the genomic DNA from step (b), which is present in a BAC-vector derived from the BAC-vector of step (a), into a prokaryotic organism;
   (d) Selecting a prokaryotic organism that contains the genomic DNA which is present in the BAC-vector of step (c);
   (e) Optionally, isolating said BAC-vector from the prokaryotic organism of step (d);
   (f) Introducing the BAC-vector from step (d), or, optionally, the isolated BAC-vector from step (e), via conjugation into the host cell, whereupon said BAC-vector integrates into the host cell genome.
(2) The method according to item (1), wherein the integrase of step (a3) is a ΦC31 integrase.
(3) A method for targeted cloning and/or transferring of DNA fragments from a donor organism genome into a host cell, the method comprising the following steps:
   (a) Transferring a BAC-vector into the donor organism,
      wherein said vector comprises
      (a1) an origin of replication;
      (a2) an origin of transfer;
      (a3) a site-specific recombination system containing an attP site however no functional integrase;
      (a4) a DNA sequence that is substantially homologous to a desired target integration site in the donor organism and that allows for homologous recombination with a corresponding homologous DNA sequence being present in the genomic DNA of the donor organism; and
      (a5) a selection marker,
   (b) Isolating the genomic DNA from the donor organism;
   (c) Transferring the genomic DNA from step (b), which is present in a BAC-vector derived from the BAC-vector of step (a), into a prokaryotic organism;
   (d) Selecting a prokaryotic organism that contains the genomic DNA which is present in the BAC-vector of step (c);
   (e) Optionally, isolating said BAC-vector from the prokaryotic organism of step (d);
   (f) Introducing the BAC-vector from step (d), or, optionally, the isolated BAC-vector from step (e), via conjugation into the host cell, whereupon said BAC-vector integrates into the host cell genome by action of an integrase the host cell is provided with.
(4) The method according to any of the previous items, wherein the BAC-vector comprises one origin of replication.
(5) The method according to item (3) or (4), wherein the functional integrase of step (f) is a ΦC31 integrase.
(6) The method according to any of the previous items, wherein the prokaryotic organism is E. coli.
(7) The method according to any of the previous items, wherein the BAC-vector is a shuttle BAC-vector, preferably an E. coli - Actinomycetes conjugative vector.
(8) The method according to any of the previous items, wherein between any of steps (c) to (f), e.g. between steps (c) and (d), or between (d) and (e), or between (e) and (f), or between (c) and (e), or between (c) and (f), or between (d) and (f), preferably between steps (c) and (f) the BAC-vector from step (c), i.e. the BAC-vector derived from the BAC-vector from step (a), is not modified, preferably no attP-site, attP-site integrase cassette, and/or integrase cassette is introduced into said BAC-vector.
(9) The method according to any of the previous items, further comprising the following steps:
   - after step (a) and prior to step (b): Selecting for the donor organism that contains the BAC-vector incorporated into the donor organism genome; and/or
   - after step (b) and prior to step (c): Cleaving and self-ligation of said isolated genomic DNA.
(10) The method according to any of the previous items, wherein the transfer of the BAC-vector into the donor organism comprises a homologous recombination step.
(11) The method according to any of the previous items, wherein the conjugation of step (f) is mediated by electrocompetent E. coli cells that contain a conjugation helper plasmid.
(12) The method according to any of the previous items, wherein the genomic DNA that is present in the BAC-vector from steps (c) to (f) comprises a DNA fragment from the donor organism.
(13) The method according to item (12), wherein the DNA fragment has a size of from about 5 kb to about 500 kb.
(14) The method according to items (12) or (13), wherein the DNA fragment comprises a whole gene cluster or a portion of a gene cluster, preferably a whole gene cluster, of the donor organism.
(15) The method according to item (14), wherein the whole gene cluster or portion of a gene cluster, preferably the whole gene cluster, encodes one or more gene product(s) that is (are) part of a biosynthetic pathway for secondary metabolites.
(16) The method according to items (14) or (15), wherein the whole gene cluster or portion of a gene cluster, preferably the whole gene cluster, comprises the whole clavulanic acid cluster or parts thereof.
(17) The method according to any of the previous items, wherein the donor organism is a bacterium, and preferably is selected from the group consisting of acidobacteria, actinobacteria, bacetroidets, firmicutes, proteobacteria or thermodesulfobacteria;
   more preferably, the donor organism is selected from the group consisting of the family *Acidobacteriaceae, Actinomycetaceae, Actinopolysporaceae, Corynebacteriaceae, Gordoniaceae, Mycobacteriaceae, Nocardiaceae, Brevibacteriaceae, Cellulomonadaceae, Demequinaceae, Microbacteriaceae, Micrococcaceae, Promicromonosporaceae, Aarobacteraceae, Micromonosporaceae, Nocardioidaceae, Propionibacteriaceae, Actinosynnemataceae, Pseudonocardiaceae, Streptomycetaceae, Nocardiopsaceae, Streptosporangiaceae, Thermomonosporaceae, Bifidobacteriaceae, Bacteroidaceae, Marinilabiliaceae, Morphyromonadaceae, Cyclobacteriaceae, Cytophagaceae, Flammeovirgaceae, Rhodothermaceae, Blattabacteriaceae, Cryomorphaceae, Flavobacteriaceae, Schleiferiaceae, Chitinophagaceae, Saprospiraceae, Sphingobacteriaceae, Chlamydiaceae, Parachlamydiaceae, Simkaniaceae, Waddliaceae, Bacillaceae, Listeriaceae, Paenibacillaceae, Planococcaceae, Staphylococcaceae, Aerococcaceae, Carnobacteriaceae, Enterococcaceae, Lactobacillaceae, Leuconostocaceae, Streptococcaceae, Clostridiaceae, Eubacteriaceae, Heliobacteriaceae, Peptococcaceae, Ruminococcaceae, Caulobacteraceae, Hyphomonadaceae, Bradyrhizobiaceae, Brucellaceae, Hyphomicrobiaceae, Methylobacteriaceae, Rhizobiaceae, Xanthobacteraceae, Rhodobacteraceae, Acetobacteraceae, Rhodospirillaceae, Alcaligenaceae, Burkholderiaceae, Comamonadaceae, Oxalobacteraceae, Neisseriaceae, Spirillaceae, Rhodocyclaceae, Desulfobacteraceae, Cystobacteraceae, Myxococcaceae, Nannocystaceae, Phaselicystidaceae, Polyangiaceae, Sandaracinaceae, Syntrophaceae, Syntrophobacteraceae, Syntrophorhabdaceae, Campylobacteraceae, Helicobacteraceae, Acidithiobacillaceae, Aeromonadaceae, Succinivibrionaceae, Alteromonadaceae, Celerinatantimonadaceae, Colwelliaceae, Ferrimonadaceae, Idiomarinaceae, Moritellaceae, Pseudoalteromonadaceae, Psychromonadaceae, Shewanellaceae, Chromatiaceae, Ectothiorhodospiraceae, Granulosicoccaceae, Enterobacteriaceae, Coxiellaceae, Legionellaceae, Alcanivoracaceae, Hahellaceae, Halomonadaceae, Litoricolaceae, Pasteurellaceae, Moraxellaceae, Pseudomonadaceae, Vibrionaceae, Nevskiaceae, Sinobacteraceae, thomonadaceae Brachyspiraceae, Brevinemataceae, Leptospiraceae, Spirochaetaceae, Haloplasmataceae and Mycoplasmataceae;*
   even more preferably, the donor organism is a bacterium selected from the group consisting of the families *Acidobacteriaceae, Actinomycetaceae, Actinopolysporaceae, Corynebacteriaceae, Mycobacteriaceae, Nocardiaceae, Brevibacteriaceae, Microbacteriaceae, Micrococcaceae, Promicromonosporaceae, Aarobacteraceae, Micromonosporaceae, Nocardioidaceae, Propionibacteriaceae, Actinosynnemataceae, Pseudonocardiaceae, Streptomycetaceae, Nocardiopsaceae, Streptosporangiaceae, Bifidobacteriaceae, Bacteroidaceae, Flavobacteriaceae, Chlamydiaceae, Bacillaceae, Listeriaceae, Paenibacillaceae, Planococcaceae, Staphylococcaceae, Aerococcaceae, Enterococcaceae, Lactobacillaceae, Leuconostocaceae, Streptococcaceae, Clostridiaceae, Eubacteriaceae, Heliobacteriaceae, Caulobacteraceae, Methylobacteriaceae, Rhizobiaceae, Xanthobacteraceae, Rhodobacteraceae, Acetobacteraceae, Rhodospirillaceae, Alcaligenaceae, Burkholderiaceae, Comamonadaceae, Neisseriaceae, Myxococcaceae, Nannocystaceae, Phaselicystidaceae, Polyangiaceae, Sandaracinaceae, Syntrophaceae, Syntrophobacteraceae, Syntrophorhabdaceae, Campylobacteraceae, Aeromonadaceae, Alteromonadaceae, Pseudoalteromonadaceae, Psychromonadaceae, Shewanellaceae, Enterobacteriaceae, Alcanivoracaceae, Hahellaceae, , Pseudomonadaceae, Vibrionaceae and Mycoplasmataceae;* most preferably, the donor organism is selected from the group consisting of *Acidobacteriaceae, Actinomycetaceae, Actinopolysporaceae, Corynebacteriaceae, Nocardiaceae, Brevibacteriaceae, Micromonosporaceae, Nocardioidaceae, Actinosynnemataceae, Pseudonocardiaceae, Streptomycetaceae, Nocardiopsaceae, Streptosporangiaceae, Bacillaceae, Listeriaceae, Lactobacillaceae, Leuconostocaceae, Eubacteriaceae, Xanthobacteraceae, Rhodobacteraceae, Acetobacteraceae, Rhodospirillaceae, Alcaligenaceae, Burkholderiaceae, Comamonadaceae, Myxococcaceae, Nannocystaceae, Phaselicystidaceae, Polyangiaceae, Syntrophobacteraceae, Syntrophorhabdaceae, Campylobacteraceae, Aeromonadaceae, Enterobacteriaceae, Alcanivoracaceae and Pseudomonadaceae;* and
   even most preferred, the donor organism is selected from the group consisting of *Actinomycetaceae, Actinopolysporaceae, Brevibacteriaceae, Nocardioidaceae, Micromonosporaceae, Actinosynnemataceae, Streptomycetaceae, cardiopsaceae, Pseudonocardiaceae, Bacillaceae, Listeriaceae, Lactobacillaceae, Leuconostocaceae, Xanthobacteraceae,* and *Rhodobacteraceae.*
(18) The method according to any of the previous items, wherein the donor organism is preferably selected from the group consisting of *Streptomyces ambofaciens, Streptomyces avermitilis, Streptomyces capreolus, Streptomyces carcinostaticus, Streptomyces cervinus, Streptomyces clavuligerus, Streptomyces davawensis, Streptomyces fradiae, Streptomyces griseus, Streptomyces hygroscopicus, Streptomyces lavendulae, Streptomyces lividans, Streptomyces natalensis, Streptomyces noursei, Streptomyces kanamyceticus, Streptomyces nodosum, Streptomyces tsukubaensis, Streptomyces sp., Streptomyces coelicolor, Streptomyces cinnamonensis, Streptomyces platensis, Streptomyces rimosus, Streptomyces spectabilis, Streptomyces verticillus, Streptomyces venezuelae, Streptomyces violaceoniger, Streptomyces violaceoruber, Streptomyces mobaraensis, Streptomyces peuceticus, Streptomyces coeruleorubidus, Actinomyces bovis, Actinomyces bowdenii, Actinomyces canis, Actinomyces catuli, Actinomyces coleocanis, Actinomyces europaeus, Actinomyces funkei, Actinomyces georgiae, Actinomyces hongkongensis, Actinomyces israelii, Actinomyces marimammalium, Actinomyces meyeri, Actinomyces oricola, Actinomyces slackii, Actinomyces streptomycini, Actinomyces suis, Actinoplanes teichomyceticus, Actinosynnema pretiosum, Pseudonocardia autotrophica, Amycolatopsis mediteranei, Amycolatopsis orientalis, Saccharopolyspora eritharaea, Saccharopoylspora hirsuta, Pseudomonas aeruginosa, Pseudomonas putida, Pseudomonas syringae, Xantomonas orysae, Burkholderia cenocepacia, Bacillus subtilis, Bacilllus licheniformis, Bacillus megaterium, Mixococcus xantus, Lactobacillus acidophilus, Lactobacillus casei, Lactobacillus brevis, Lactococcus lactis, Brevibacterium casei, Brevibacterium oxydans, Bifidobacterium bifidum, Bifidobacterum longum, Commamonas sp., Corynebacterium glutamicum, Rhodococcus sp., Serratia marescens, Rhizobium trifolii, Rhizobium radiobacter, Sporangium cellulosum, Chondromyces crocatus, Gluconobacter oxydans, Micromonospora grisea, Micrococcus sp., Micromonospora rhodorangea, Micromonospora zionensis, Micromonospora purpurea, Micromonospora inyoensis, Micromonospora sagamiensis, Palnomonospora parontospora, Dactylosporangium matsuzakiense. Nocardia lactamdurans, Bacillus colistinus, Planobispora rosea, Streptomyces arenae, Streptomyces antibioticus, Streptomyces kasugaensis, Streptomyces mitakaensis, Streptomyces bikiniensis, Streptomyces alboniger, Streptomyces tenebrarius, Bacillus circulans, Streptomyces ribosidificus, Bacillus colistinus, Streptomyces tenjimariensis, and Streptomyces pactum; preferably, the donor organism is Streptomyces clavuligerus.*
(19) The method according to any of the previous items, wherein the host cell is selected from the group consisting of Streptomyces, Saccharopolyspora, Nocardia, Pseudonocardia, Amycolatopsis, Actinobacterium, Pseudomonas, Gluconobacter, Xantomonas, Burkholderia, E. coli, Bacillus, Mixoccoccus, Lactobacillus, Lactococcus, Bifidobacterium. Comamonas, Corynebacterium, Brevibacterium, Rhodococcus, Azotobacter, Citrobacter, Enterobacter, Clostridium, Klebsiella, Salmonella, Saccharomyces, Zygosaccharomyces, Pichia, Kluyveromyces, Candida, Hansenula, Dunahella, Debaryomyces, Mucor, Torulopsis, Serratia, Rhizobium, Zymomonas, Propionibacterium, Acetobacter, Arthrobacter, Ralstonia, Sporangium, Chondromyces, Gluconobacter, Propionibacterium, Aspergillus, Neurospora, Chrysosporium, Thielavia, Neurospora, Aureobasidium, Fihbasidium, Piromyces, Cryplococcus, Acremonium, Tolypocladium, Scytalidium, Schizophyllum, Sporotrichum, Penicillium, Mucor, Fusarium and Trichoderma; preferably the host cell is selected from the group consisting of Streptomyces, Saccharopolyspora, Nocardia, Pseudonocardia, Amycolatopsis, Pseudomonas, Xantomonas, Burkholderia, Bacillus, Mixoccoccus, Lactobacillus, Lactococcus, Brevibacterium, Bifidobacterium, Comamonas, Corynebacterium, Rhodococcus, Saccharomyces, Pichia, Kluyveromyces, Candida, Serratia, Rhizobium, Sporangium, Chondromyces, Gluconobacter, Aspergillus, Neurospora, Acremonium, Penicillium, Mucor and Escherichia coli;
   preferably, the host cell belongs to the genus Streptomyces and, more preferably, is selected from the group consisting of Streptomyces clavuligerus, Streptomyces avermitilis, Streptomyces ambofaciens, Streptomyces fradiae, Streptomyces griseus, Streptomyces hygroscopicus, Streptomyces lavendulae, Streptomyces lividans, Streptomyces natalensis, Streptomyces platensis, Streptomyces rimosus, Streptomyces venezuelae, Pseudonocardia autotrophica, Amycolatopsis mediteranei, Amycolatopsis orientalis, Saccharopolyspora eritharaea, Saccharopoylspora hirsute, Pseudomonas aeruginosa, Pseudomonas putida, Pseudomonas syringae, Penicillium citrinum, Penicillium chrisogenium, Xantomonas orysae, Burkholderia cenocepacia, Bacillus subtilis, Bacilllus licheniformis, Bacillus megaterium, Mixococcus xantus, Lactobacillus acidophilus, Lactobacillus casei, Lactobacillus brevis, Lactococcus lactis, Brevibacterium casei, Brevibacterium oxydans, Bifidobacterium bifidum, Bifidobacterum longum, Commamonas sp., Coryneobacterium glutamicum, Rhodococcus sp., Aspergilus nidulans, Aspergillus niger, Aspergillus terreus, Saccaromyces cerevisiae, Pichia pastoris, Kluyveromyces lactis, Candida albicans, Candida antarctica, Serratia marescens, Rhizobium trifolii, Rhisobium radiobacter, Sporangium cellulosum, Chondromyces crocatus, Gluconobacter oxydans, Neurospora crassa, Acremonium chrysogenum and Mucor mucor;
   and most preferably, the host cell is selected from the group consisting of S. clavuligerus, S. lividans, S. avermitilis, and E. coli.
(20) The method according to any of the previous items, wherein the origin of replication is ori S.
(21) The method according to any of the previous items, wherein the origin of transfer is ori T.
(22) The method according to any of the previous items, wherein the site-specific recombination system is a ΦC31 attP-int recombination system.
(23) The method according to any of the previous items, wherein the method is a plasmid rescue method.
(24) A vector comprising
   (1) an origin of replication;
   (2) an origin of transfer;
   (3) a site-specific recombination system containing a functional integrase and an attP site;
   (4) a DNA sequence that allows for homologous recombination with a corresponding substantially homologous DNA sequence being present in the genomic DNA of a donor organism; and
   (5) a selection marker.
(25) The vector according to item (24), wherein the integrase is a φC31 integrase, and/or wherein the donor organism, which preferably does not contain a functional attB site, is a donor organism as defined in item (17) or (18).
(26) A vector comprising
   (1) an origin of replication;
   (2) an origin of transfer;
   (3) a site-specific recombination system containing an attP site however no functional integrase; and
   (4) a selection marker.
   In a preferred embodiment, the vector of item (26) comprises SEQ ID NO: 6; more preferred, the vector of item (26) has a sequence according to SEQ ID NO: 6.
(27) The vector according to any of items (24) to (26), wherein said vector comprises one origin of replication.
(28) The vector according to item (26) or (27), wherein said vector further comprises a DNA sequence that allows for homologous recombination with a corresponding substantially homologous DNA sequence being present in the genomic DNA of a donor organism.
(29) The vector according to any of items (24) to (28), wherein said vector is a BAC-vector suitable for cloning and/or transfer of a DNA fragment.
(30) The vector according to any of items (24) to (29), wherein the BAC-vector is a shuttle BAC-vector, preferably an E. coli - Actinomycetes conjugative vector.
(31) The vector according to any of items (24) to (30), wherein said vector further comprises a DNA fragment that is intended to be cloned or transferred, preferably said DNA fragment is as defined in items (13) to (16).
(32) The vector according to any of items (24) to (31), wherein the origin of replication is ori S, and/or the origin of transfer is ori T.
(33) Use of a vector of any of items (24) to (32) for cloning and/or transferring of a DNA fragment.
(34) Use according to item (33), wherein the DNA fragment is as defined in items (13) to (16).
(35) A donor organism, comprising a BAC-vector as defined in items (1) (a1) - (a5), or as defined in any one of items (24) or (25), or (27) to (32).
(36) The donor organism according to item (35), wherein said donor organism does not contain a functional attB site.
(37) The donor organism according to item (35) or (36), wherein the BAC-vector comprises a ΦC31 integrase.
(38) A donor organism, comprising a BAC-vector as defined in item (3) (a1) to (a5), or as defined in any one of items (26) to (32).
(39) The donor organism according to any of items (35) to (38), wherein the BAC-vector is a shuttle BAC-vector, preferably an E. coli ― Actinomycetes conjugative vector.
(40) The donor organism according to any of items (35) to (39), wherein the origin of replication of the BAC-vector is ori S, and/or wherein the origin of transfer is ori T.
(41) The donor organism according to any of items (35) to (40), wherein the site-specific recombination system is a ΦC31 attP-int recombination system.
(42) The donor organism according to any of items (35) to (41), wherein the donor organism is as defined in items (17) or (18).
(43) An expression system, comprising
   (1) a host cell, and
   (2) a BAC-vector, wherein said BAC-vector comprises
      i) an origin of replication;
      ii) an origin of transfer;
      iii) a site-specific recombination system containing an attP site and a functional integrase;
      iv) a DNA fragment comprising a whole gene cluster or a part of a gene cluster encoding one or more gene product(s) that is (are) part of a biosynthetic pathway for secondary metabolites; and
      v) a selection marker.
(44) An expression system, comprising
   (1) a host cell, wherein said host cell is provided with a functional integrase, and
   (2) a BAC-vector, wherein said BAC-vector comprises
      i) an origin of replication;
      ii) an origin of transfer;
      iii) a site-specific recombination system containing an attP site however no functional integrase;
      iv) a DNA fragment comprising a whole gene cluster or a part of a gene cluster encoding one or more gene product(s) that is (are) part of a biosynthetic pathway for secondary metabolites; and
      v) a selection marker.
(45) The expression system according to item (43) or (44), wherein the BAC-vector comprises one origin of replication.
(46) The expression system according to any of items (43) to (45), wherein the host cell is as defined in item (19), the origin of replication is ori S, the origin of transfer is ori T, the site-specific recombination system is ΦC31 attP-int recombination system, and/or the BAC-vector is as defined in items (29) or (30).
(47) Use of the expression system according to any of items (43) to (46) for cloning and/or transfer of the DNA fragment, and/or for expression of said DNA fragment.
(48) A vector comprising the sequence according to SEQ ID NO: 3.
(49) A vector comprising the sequence according to SEQ ID NO: 53.
(50) The vector according to item (48) or (49), additionally comprising a DNA sequence that allows for homologous recombination with a corresponding substantially homologous DNA sequence being present in the genomic DNA of a donor organism.
(51) The vector according to item (50), additionally comprising a DNA fragment as defined in any of items (13) to (16).
(52) Use of a vector according to any of items (48) to (51) for cloning and/or transfer of a DNA fragment, and/or for expression of said DNA fragment.

### Definitions of terms as used within the meaning of the present invention

As used herein, the term "donor organism" refers to the organism that comprises the DNA fragment(s) in its genome that is intended for being cloned and/or transferred e.g. into a host cell. Within the meaning of the present invention, such a donor organism is any microorganism which accepts foreign DNA via the conjugal transfer from E. coli, and preferably integrates said DNA via homologous integration, and does not support replication of the BAC replicon.

Herein, the term "host cell" denotes the organism that finally receives the cloned DNA fragment of the donor organism. In one embodiment according to the present invention, the cloned DNA fragment is multiplicated (e.g. duplicated), or amplified, respectively, in the host cell. It is also possible that the cloned DNA fragment is expressed in a host organism that, naturally, does not express said DNA fragment.

Within the meaning of the present invention, the term "DNA fragment" refers to a piece of DNA that has a size of desired length, suitably of at least about 5 kb, preferably at least about 10 kb, more preferably at least about 20 or 30 kb, even more preferably at least about 40 or 50 kb. The upper size limit of said piece of DNA can be chosen as desired, suitably is about 300 kb, preferably about 350 or 400 kb. In some embodiments, the upper size limit is even higher, such as 450 or 500 kb. Further within the meaning of the present invention, the DNA fragment originates from the genome of a donor organism and preferably comprises a whole gene cluster or a portion of a gene cluster of said donor organism.

The term "about" defines a deviation of 20%, preferably of 10%, more preferably of 7% and even more preferably of 3% of a given value.

A "vector" is a replicon, such as plasmid, phage, bacterial artificial chromosome (BAC) or cosmid, to which another DNA segment (e.g. a foreign gene) may be incorporated so as to bring about the replication of the attached segment, resulting in expression of the introduced sequence. Vectors may comprise a promoter and one or more control elements (e.g., enhancer elements) that are heterologous to the introduced DNA but are recognized and used by the host cell. Alternatively, the sequence that is introduced into the vector retains its natural promoter that may be recognized and expressed by the host cell (Bormann et al., J. Bacteriol1996;178:1216-1218). A "replicon" is any genetic element (e.g., plasmid, chromosome, virus) that functions as an autonomous unit of DNA replication within a cell, i.e., capable of replication under its own control.

A BAC-vector within the meaning of the present invention denotes a cloning vector derived from a bacterial chromosome into which a large genomic DNA fragment can be inserted, typically up to 400 kb. BAC-vectors are F-plasmid (F factor) derived. Further, BAC-vectors can comprise a prokaryotic F factor partitioning system, which ensures that the daughter cells inherit a copy of the parental plasmid.

A vector can also be a Bacterial Artificial Chromosome (BAC) shuttle vector. This is a vector that permits conjugation between e.g., Streptomyces and E. coli. As known to a skilled person, a common way to insert one segment of DNA into another segment of DNA involves the use of specific enzymes called restriction enzymes that cleave DNA at specific sites (specific groups of nucleotides) called restriction sites. A "cassette" refers to a DNA coding sequence or segment of DNA that codes for an expression product that can be inserted into a vector at defined restriction sites. The cassette restriction sites are designed to ensure insertion of the cassette in the proper reading frame. Generally, foreign DNA is inserted at one or more restriction sites of the vector DNA, and then is carried by the vector into a host cell along with the transmissible vector DNA.

A common type of vector is a "plasmid", which generally is a self-contained molecule of double-stranded DNA, usually of bacterial origin, that can readily accept additional (foreign) DNA and which can be readily introduced into a suitable host cell. A plasmid vector often contains coding DNA and promoter DNA and has one or more restriction sites suitable for inserting foreign DNA. Promoter DNA and coding DNA may be from the same gene or from different genes, and may be from the same or different organisms. Recombinant cloning vectors will often include one or more replication systems for cloning or expression, one or more markers for selection in the host, e.g. antibiotic resistance, and one or more expression cassettes. Vector constructs may be produced using conventional molecular biology and recombinant DNA techniques within the skill of the art. Such techniques are explained fully in the literature. See, e.g., Sambrook, Fritsch & Maniatis, Molecular Cloning: A Laboratory Manual, Second Edition (1989) Cold Spring Harbor Laboratory Press, Cold Spring Harbor, New York (herein "Sambrook et al., 1989"); DNA Cloning: A Practical Approach, Volumes I and II (D.N. Glover ed. 1985); F.M. Ausubel et al. (eds.), Current Protocols in Molecular Biology, John Wiley & Sons, Inc. (1994).

Within the meaning of the present invention, the term "BAC-vector derived from..." (e.g. derived from a previous (or predecessor) BAC-vector) defines a BAC-vector that comprises most of the regulatory elements of the previous BAC-vector from which it is derived, however does not completely correspond to said previous BAC-vector. For instance, a BAC-vector that is derived from a previous BAC-vector still comprises regulatory elements such as origin of replication, origin of transfer, a site-specific recombination system (e.g. containing an integrase and an attP site, or containing an attP site however no functional integrase), and/or a selection marker. Further within the meaning of the present invention, the BAC-vector that is derived from a previous BAC-vector comprises a nucleic acid (e.g. DNA) fragment that comprises a whole gene cluster or a portion of a gene cluster, preferably a whole gene cluster, which gene cluster, in turn, comprises a nucleic acid (e.g. DNA) sequence that is substantially homologous or completely homologous to a nucleic acid (e.g. DNA) sequence that is present in the previous BAC-vector. Said nucleic acid sequence that is present in the previous BAC-vector in general is of a length that allows for homologous recombination, and, in general, has a suitable size such as about 1kb-2kb. The above gene cluster represents the gene cluster that is intended to be cloned and/or transferred.

Within the meaning of the present invention, the term "plasmid rescue method" defines a method that can be used for isolating (chromosomal) DNA adjacent to an inserted piece of DNA in various organisms, such the donor organisms as defined herein elsewhere. In other words, the "plasmid rescue method" provides for a means of isolating (i.e. removing of a material of interest such as a DNA fragment or a genetic cluster form its natural environment) or recovering a gene cluster that lies adjacent to the integration site, such that it is free of any other contaminating genetic or cellular material.

The term "gene cluster" denotes any group of two or more closely linked genes that encode for the same or similar products.

Secondary metabolites often play an important role in plant defense against herbivory and other interspecies defenses. In bacteria, secondary metabolites have a primary role in the struggle for nutrients and habitat in a complex microbial environment. Consequently, most secondary metabolites have biological activity against competing bacteria, fungi or other orgamisms. Some secondary metabolites are acting as inhibitors of competitor's nutrient uptake enzymes, others directly displaying antibacterial or antifungal activity, others have activity which counters competitor's defence mechanisms and yet others counter competitor's offence mechamism. It is well known that secondary metabolites show increadible wealth of diversity in terms of chemical characteristics. Therefore, humans use some secondary metabolites as medicines, flavorings, and recreational drugs. Secondary metabolites can be divided in the following categories: Small "small molecules", such as beta-lactams, alkaloids, terpenoids, glycosides, natural phenols, phenazines, biphenyls and dibenzufurans; big "small molecules", produced by large, modular, "molecular factories", such as polyketides, complex glycosides, nonribosomal peptides, and hybrids of the above three; and non-"small molecules" - DNA, RNA, ribosome, or polysaccharide "classical" biopolymers, such as ribosomal peptides. Preferably, the secondary metabolites according to the present invention are small "small molecules", such as beta-lactam.

In order to propagate a vector in a host cell, it may contain one or more "origins of replication" sites (often also termed "ori"), which is a specific nucleic acid sequence at which replication is initiated. Accordingly, the term "origin of replication" or "ori" refers to a nucleic acid sequence that initiates nucleic acid replication.

"Ori 2" or "ori S" refers to an "origin of replication" from an E. coli plasmid that allows for single copy replication (Shizuya, H., Birren, B., Kim, U.-J., Mancino, V., Slepak, TI, Tachiiri, Y., and Simon, M. 1992. Proc. Natl. Aca. Sci. 890:8794-8797).

"Ori T" refers to an "origin of transfer" that permits the transfer of the vector from one bacterial cell to another.

The "origin of transfer" (e. g, ori T) represents the site on the vector where the transfer process is initiated. It is also defined genetically as the region required in cis to the DNA that is to be transferred. Conjugation-specific DNA replication is initiated within the ori T region which also encodes plasmid transfer factors.

An enzyme called "integrase" recognizes two att sites, joins the two DNA molecules and catalyzes a DNA double-strand breakage. A rejoining event results in the integration of one of the DNA molecules into the other DNA of the recipient cell. (N. D. Grindley, K. L. Whiteson, P. A. Rice, 2006. Annu. Rev. Biochem. 75, 567-605.)

The integrases ΦBT1 or ΦC31, in combination with attP or attB, allow for site-specific recombination, which permits site-specific integration of the vector into the respective attB sites of the recipient cell's chromosome. (Gregory, M.A., Till, R., and Smith, M.C.M. 2003. J Bacteriol. 185:5320-5323; GenBank Accession Number AJ550940). The site-specific recombination is facilitated by an "att" site, which is a site that has nucleic acid identity or similarity which facilitates said site-specific recombination. The "attB" site refers to the attachment site on the bacterial cell chromosome, and the "attP" site refers to the attachment site on the bacteriophage. The sequence of att sites is different and specific for disctinct integrases such as ΦBT1 or ΦC31, therefore integration into the recipient genome is at different loci.

The term "functional" defines that the respective matter exhibits its natural function. For instance, a functional attB or attP site is an attB or attP site that mediates, in combination with a functional integrase, a site-specific recombination. An attB/P site or integrase that is not functional (any more) can for instance have lost its respective function due to genetic modification, such as gene deletion, or gene disruption.

A "site-specific recombination" refers to a recombination process between two DNA molecules that occurs at unique sites ― the integration site ― of each molecule which are generally 20 to 30 bases long, and that are called attachment (att) sites. A specialised enzyme called integrase recognizes the two att sites, finally resulting in the integration of one of the DNA molecules into the other DNA of the recipient cell (N. D. Grindley, K. L. Whiteson, P. A. Rice, 2006. Annu. Rev. Biochem. 75, 567-605.).

Within the meaning of the present invention, two DNA sequences are "substantially homologous" if they are able to mediate a homologous recombination. This may for instance the case when at least about 80%, preferably at least about 90% or 95%, more preferably at least about 97% or 98% of the nucleotides match over a defined length of the DNA sequences, as determined by sequence comparison algorithms known to a person skilled in the art, such as BLASTA, FASTA, DNA Strider, and so on. It is also possible that two DNA sequences exhibit 100% match of the nucleotides over a defined length. In this case, the two DNA sequences are homologous to each other. Within the meaning of the present invention, a DNA sequence in the BAC-vector is substantially homologous, or homologous, to a desired target integration site in the donor organism.

The term "homologous recombination" refers to a type of genetic recombination, a process of physical rearrangement occurring between two different strands of DNA molecules. Homologous recombination involves the alignment of identical or similar sequences, a crossover between the aligned homologous DNA strands of the two molecules, and breaking and repair of the DNA to produce an exchange of material between the strands. Homologous recombination is distinguished from other types of recombination. For example, "site specific recombination", as exemplified by invertible elements, resolvases, and some phage integration events are examples of non-homologous recombination. Though in many cases identical or similar sequences are required at the two recombining sites, the sequences are short, distinguishing them from the longer stretches (hundreds of base pairs) used in homologous recombination. (J Rubnitz and S Subramani. 1984, Mol Cell Biol. 4: 2253-2258).

The term "selecting" refers to the identification and isolation of a recipient cell such as the donor organism or host organism (or donor cell/donor strain or host cell/strain, respectively) or a further prokaryotic organism (such as E. coli) that contains the vector of interest (such as a BAC-vector as defined herein, e.g. a BAC-vector containing the cloned gene cluster). Transformed microorganisms, that is, those containing recombinant molecules such as a vector or plasmid, may be selected with a variety of positive and/or negative selection methods or markers. For instance, a positive selection marker can be a gene that allows growth in the absence of an essential nutrient, such as an amino acid. A variety of suitable positive/negative selection pairs are available in the art. For example, various amino acid analogs known in the art could be used as a negative selection, while growth on minimal media (relative to the amino acid analog) could be used as a positive selection. Visually detectable markers are also suitable for being uses in the present invention, and may be positively and negatively selected and/or screened using technologies such as fluorescence activated cell sorting (FACS) or microfluidics. Examples of detectable markers include various enzymes, prosthetic groups, fluorescent markers, luminescent markers, bioluminescent markers, and the like. Examples of suitable fluorescent proteins include, but are not limited to, yellow fluorescent protein (YFP), green fluorescence protein (GFP), cyan fluorescence protein (CFP), umbelliferone, fluorescein, fluorescein isothiocyanate, rhodamine, dichiorotriazinylamine fluorescein, dansyl chloride, phycoerythrin and the like. Examples of suitable bioluminescent markers include, but are not limited to, luciferase (e.g., bacterial, firefly, click beetle and the like), luciferin, aequorin and the like. Examples of suitable enzyme systems having visually detectable signals include, but are not limited to, galactosidases, glucorinidases, phosphatases, peroxidases, cholinesterases and the like. In other aspects, the positive selection marker is a gene that confers resistance to a compound, which would be lethal to the cell in the absence of the gene. For example, a cell expressing an antibiotic resistance gene would survive in the presence of an antibiotic, while a cell lacking the gene would not. For instance, the presence of a tetracycline resistance gene could be positively selected for in the presence of tetracycline, and negatively selected against in the presence of fusaric acid. Suitable antibiotic resistance genes include, but are not limited to, genes such as ampicillin-resistance gene, neomycin-resistance gene, blasticidin-resistance gene, hygromycin-resistance gene, puromycin-resistance gene, chloramphenicol-resistance gene, apramycin-resistance gene and the like. In certain aspects, the negative selection marker is a gene that is lethal to the target cell in the presence of a particular substrate. For example, the thyA gene is lethal in the presence of trimethoprim. Accordingly, cells that grow in the presence trimethoprim do not express the thyA gene. Negative selection markers include, but are not limited to, genes such as thyA, sacB, gnd, gapC, zwJ, talA, taiB, coda, ppc, gdhA, pgi, Jbp, pykA, cit, acs, edd, icdA, groEL, secA and the like.

Within the meaning of the present invention, the term "isolating" refers to the removal of a material of interest from its environment (e.g. from an environment into which it has been placed). For instance, the material of interest can be genomic DNA, which is isolated from an organism, e.g. from a donor organism as defined herein, or from a host organism as defined herein, such as E. coli.

Further within the meaning of the present invention the term "conjugation" refers to the direct transfer of nucleic acid from one prokaryotic cell to another via direct contact of cells. The cell that carries the transferred DNA after conjugation is termed "exoconjugant" or "exconjugant". Thus, a "conjugative vector" (for example, pSBAC) is a vector that contains a nucleic acid of interest, whereby such nucleic acid is directly transferred (i.e. the passing of a nucleic acid sequence from one cell to another without isolation of the sequence) from one cell to another via direct contact between the cell containing the vector and a recipient cell to which the nucleic acid is transferred following direct contact of the two cells. As used herein, the term "conjugative transfer" refers to the temporary union of two bacterial cells during which one cell transfers part or all of its genetic material to the other.

"Secondary metabolites" are organic compounds that are not directly involved in the normal growth, development, or reproduction of organisms. Unlike primary metabolites, absence of secondary metabolites only results in mild impairment for the organisms such as: lowered survivability/fecundity, aesthetic differences, or else no change in phenotype at all.

Within the meaning of the present invention, the term "expression system" denotes a system that is specifically designed for the production of a gene product of choice. For instance, in the present invention the expression system is able to produce the gene product of the DNA fragment (or gene cluster, or part thereof) that previously has been intended for being cloned and/or transferred.

Within the meaning of the present invention, the term "attB deletion strain" denotes a (donor) strain that does not comprise a functional attB site. It is understood that some organisms may lack the attB site in their genome in their native state. Therefore, within the meaning of the present invention either situation is allowed to carry out the cloning of the gene clusters, i.e. using a donor organism that naturally lacks the attB site, and using a donor organism that had to be treated in a specific way (e.g. by using molecular cloning methods) in order to delete the attB site. In general, the integrase is not present in the donor strain.

A "prokaryotic F factor partitioning system" refers to an active positioning process that ensures proper segregation and faithful distribution of daughter F factors at cell division. This partitioning system contains three functionally distinct regions: two of them (sopA and sop B) encode gene products that act in trans, whereas the third region (sopC) functions in cis.

An "F factor" or "prokaryotic F factor" refers to a fertility factor found in prokaryotes. It is a small piece of episomal DNA that enables bacteria to mediate conjugation with other bacteria. The F factor "partitioning system" refers to the system that ensures that both daughter cells inherit a copy of the parental plasmid.

### Detailed description of the invention

The present invention is now described in more detail by preferred embodiments and examples, which are however presented for illustrative purpose only and shall not be understood as limiting the scope of the present invention in any way.

The present invention provides an improved method for targeted cloning and/or transferring of DNA fragments from a donor organism into a host cell.

This is achieved by (I) a method comprising the step of transferring a BAC-vector into the donor organism wherein said vector contains an integrase and an attP site and is introduced into a donor organism that does not contain a functional attB site.

Further, this is also achieved by (II) applying a method that comprises the step of transferring a BAC-vector into a donor organism, wherein said vector comprises a site-specific recombination system that contains an attP site however, no functional integrase and said donor organism may or may not have a functional attB site.

In prior art methods it has often been necessary in targeted cloning methods to modify the BAC-DNA plasmid that comprises the cloned DNA fragment and that is to be transferred into a host cell. Such modification can for instance be the introduction of attP-integrase cassettes: In case said cassette is introduced into the BAC-vector prior to the plasmid rescue attempt, the vector would preferably integrate into the attB site of the genome and not to the desired, targeted locus. Hence, in prior art methods, modifications such as the introduction of attP-integrase cassettes often have to be carried out on the BAC-DNA plasmid that comprises the cloned DNA fragment and that is to be transferred into a host cell. Such modifications are difficult as standard molecular methods do not apply, and, instead, are laborious, specialized and lengthy. Further, any additional propagation of the BAC-DNA plasmid that comprises the cloned DNA fragment, especially when the second, inducible multicopy replicon (e.g. ori V) is present in the BAC-vector, poses a risk of unwanted rearrangements in the BAC-DNA plasmid which may be caused by recombination events, faulty segregation etc.

It has been surprisingly found that by applying the method according to the present invention, an improvement of the presently known prior art methods for cloning and/or transfer of DNA fragments as defined herein and/or for expression of said DNA fragments, can be achieved. In particular, an improvement in terms of time required for cloning and/or transferring and/or expressing DNA fragments can be achieved, as the desired targeted gene cluster (which is comprised in a DNA fragment) can be cloned in a targeted approach and directly transferred to the host cell or host organism, respectively. "Directly transferred" in the context of the present invention denotes that there is no need to modify the rescued BAC-vector: Thus, a modification of the BAC-DNA plasmid that comprises the cloned DNA fragment and that is to be transferred into a host cell (e.g. a heteologous or homologous actinomyces host) can be avoided. This is reached by two approaches: One approach (I) is to prepare an attB deletion strain of the organism which is the donor of the DNA fragment to be cloned (donor organism). The BAC-vector, which comprises a functional attP site, a functional integrase and a DNA sequence that allows for homologous recombination with a desired target site of the genome of the donor organism, is transferred into such an attB deletion strain. The BAC-vector integrates into the desired target site on the genomic DNA of the donor organism via homologous recombination. Then, the genomic DNA comprising the desired DNA fragment from the donor which is to be cloned and/or expressed, is isolated from the donor organism, followed by a transfer of said genomic DNA, which is present in a BAC-vector, into a prokaryotic organism. This BAC-vector is then introduced into a host cell, whereupon said BAC-vector integrates into the host cell genome. The integration into the host cell's genome is mediated via attP/attB site and integrase. By applying approach (I), large clusters can be cloned in a targeted approach and directly transferred to the host cell or host organism, respectively. The same approach applies in case the donor organism does not contain an attB site in its genome already by nature. In this case, a donor organism that does not contain an attB site in its genome by nature, i.e. naturally, is provided.

The second approach (II) is to use a BAC-vector without the integrase gene however still harbouring the integrase plasmid binding site, the so-called attP site. In this case, the BAC-vector comprising the desired DNA fragment of the donor organism is integrated into the host cell's genome by action of an integrase the host cell is provided with. The integrase that is needed for attP/attB site directed integration of the BAC-vector into the host cell's genome can for instance be provided *in trans* by a helper plasmid. This latter approach additionally has the advantage that it is even more improved compared to approach (I), as it is for instance more universal as no deletion donor strain has to be prepared.

Further, the present invention can advantageously be used for genome sequence based, targeted cloning of large DNA fragments that originate for instance from actinomyces genomes, with the purpose of amplification or heterologous expression of complete genetic clusters in a broad range of host cells, such as bacteria. Hence, the invention is useful in the fields of (1) drug discovery, e.g. expression of silent secondary metabolite genetic clusters in heterologous host (genome mining); (2) synthetic biology, e.g. combinatorial assembly of biosynthetic pathways from distinct organisms to yield heterogenous or artificial molecules; (3) process improvement, e.g. heterologous expression of gene clusters where the parent host is inappropriate for industrial production processes or amplification of whole genetic clusters where higher productivities are desired.

Thus, the present invention relates to a method (I), preferably a plasmid rescue method (I), for targeted cloning and/or transferring of DNA fragments from a donor organism genome into a host cell, the method comprising the following steps:
(a) Transferring a Bacterial Artificial Chromosome (BAC)-vector into the donor organism,
   wherein said vector comprises
   (a1) an origin of replication, preferably ori S (ori 2);
   (a2) an origin of transfer, preferably ori T;
   (a3) a site-specific recombination system containing a functional integrase and an attP site;
   (a4) a DNA sequence that is substantially homologous to a desired target integration site in the donor organism and that allows for homologous recombination with a corresponding homologous DNA sequence being present in the genomic DNA of the donor organism; and
   (a5) a selection marker:
   wherein said donor organism does not contain a functional attB site,
(b) Isolating the genomic DNA from the donor organism;
(c) Transferring the genomic DNA from step (b), which is present in a BAC-vector derived from the BAC-vector of step (a), into a prokaryotic organism;
(d) Selecting a prokaryotic organism that contains the genomic DNA which is present in the BAC-vector of step (c);
(e) Optionally, isolating the BAC-vector from the prokaryotic organism of step (d);
(f) Introducing the BAC-vector from step (d), or, optionally, the isolated BAC-vector from step (e), via conjugation into the host cell, whereupon said BAC-vector integrates into the host cell genome.

Step (e), i.e. the isolation of the BAC-DNA from the prokaryotic organism of step (d), is optional, as it is also possible to directly use the prokaryotic organism (such as E. coli) with the BAC-vector for conjugal transfer to a host organism e.g. by applying a suitable "three parental conjugal transfer protocol" which is known to a person skilled in the art, or by additionally transforming the BAC carrying prokaryotic organism by a pUZ type conjugation promoting plasmid. Moreover, the present invention further relates to a method (II), preferably a plasmid rescue method (II), for targeted cloning and/or transferring of DNA fragments from a donor organism genome into a host cell, the method comprising the following steps:
(a) Transferring a BAC-vector into the donor organism,
   wherein said vector comprises
   (a1) an origin of replication, preferably ori S (ori 2);
   (a2) an origin of transfer, preferably ori T;
   (a3) a site-specific recombination system containing an attP site however no functional integrase;
   (a4) a DNA sequence that is substantially homologous to a desired target integration site in the donor organism and that allows for homologous recombination with a corresponding homologous DNA sequence being present in the genomic DNA of the donor organism; and
   (a5) a selection marker;
(b) Isolating the genomic DNA from the donor organism;
(c) Transferring the genomic DNA from step (b), which is present in a BAC-vector derived from the BAC-vector of step (a), into a prokaryotic organism;
(d) Selecting a prokaryotic organism that contains the genomic DNA which is present in the BAC-vector of step (c);
(e) Optionally, isolating said BAC-vector from the prokaryotic organism of step (d);
(f) Introducing the BAC-vector from step (d), or, optionally, the isolated BAC-vector from step (e), via conjugation into the host cell, whereupon said BAC-vector integrates into the host cell genome by action of an integrase the host cell is provided with.

Moreover, by applying the method according to the present invention, DNA fragments having a very large size can be stably maintained in a prokaryotic organism such as E. coli, and, if desired, further integrated in a host cell genome, and e.g. expressed.

As already indicated above, by using the inventive methods, a modification of the BAC-vector of step (c) with the cloned DNA fragment can be avoided. This is a great advantage since molecular modification of DNA larger than about 30 kb is difficult as standard molecular methods do not apply, and, instead, laborious, specialized and lengthy procedures have to be applied. Further, by applying the inventive methods, there is more freedom e.g. with regard to the planned cloning strategy.

In a preferred embodiment, the vectors according to the present invention comprise only one origin of replication. In prior art methods, in general the use of a second (multicopy) ori has been applied in order to improve isolation yields of BAC DNA or in order to icrease the efficiency of conjugal transfer. However, at the same time, upon induction of such multicopy mode of BAC-vectors, there exists a danger of losing the most important property of BAC-vectors, i.e. the stable maintenance of large DNA fragments. Within the meaning of the present invention, it has been unexpectedly found that even by using only one origin of replication, surprisingly a sufficient and satisfactory e.g. conjugal transfer of the BAC-vectors from E. coli to a host cell can be obtained, while at the same time the stable maintenance of DNA fragments of larger size, i.e. of a size as indicated elsewhere herein, can be obtained.

In a further preferred embodiment of the present invention, the integrase ΦC31 is used. This means that in method (I), the site-specific recombination system in step (a3) contains a ΦC31 integrase, and in method (II), in step (f) the integrase the host cell is provided with is a ΦC31 integrase. By using a ΦC31 integrase, an improved transfer/integration frequency can be obtained compared to the transfer/integration frequency obtained when using other integrases, such as ΦBT1 integrase.

In a first step, a BAC-vector comprising one origin of replication, an origin of transfer, a site-specific recombination system containing an integrase and an attP site (method (I)) or containing an attP site however no functional integrase (method (II)), and a DNA sequence that allows for homologous recombination with a corresponding DNA sequence being present in the genomic DNA of the donor organism, is transferred into said donor organism. In order to allow for homologous recombination with a defined target sequence (that is present in the donor organism), the DNA sequence is substantially homologous or homologous to the desired, defined target sequence, e.g. the desired target integration site in the genome of the donor organism. Preferably, the desired target integration site in the genome of the donor organism is located adjacent to the DNA fragments (preferably gene cluster(s)) that are intended for being cloned. The presence of DNA sequences in the BAC-vector that are substantially homologous or homologous to said integration site in the genome of the donor organism ensures that upon homologous recombination the BAC-vector integrates in said desired target integration site.

In a preferred embodiment, the BAC-vector additionally comprises a prokaryotic F factor partitioning system. This system ensures that the daughter cells inherit a copy of the parental plasmid.

In general, a donor organism can be any suitable organism that is known to a skilled person and that comprises in its genome the DNA fragment(s) that are desired to be cloned. Within the meaning of the present invention, the donor organism is any microorganism which accepts foreign DNA via the conjugal transfer from E. coli, and preferably integrates said DNA via homologous integration, and does not support replication of the BAC replicon. In a preferred embodiment of the present invention, such a donor organism can for instance be selected from the group consisting of bacteria (gram positive and gram negative bacteria) archaea, and eukaryota (e.g. yeasts, plants, and the like). In one embodiment of the present invention, the donor organism is a bacterium, and preferably is selected from the group consisting of acidobacteria, actinobacteria, bacetroidets, firmicutes, proteobacteria or thermodesulfobacteria; more preferably, the donor organism is selected from the group consisting of the family *Acidobacteriaceae, Actinomycetaceae, Actinopolysporaceae, Corynebacteriaceae, Gordoniaceae, Mycobacteriaceae, Nocardiaceae, Brevibacteriaceae, Cellulomonadaceae, Demequinaceae, Microbacteriaceae, Micrococcaceae, Promicromonosporaceae, Aarobacteraceae, Micromonosporaceae, Nocardioidaceae, Propionibacteriaceae, Actinosynnemataceae, Pseudonocardiaceae, Streptomycetaceae, Nocardiopsaceae, Streptosporangiaceae, Thermomonosporaceae, Bifidobacteriaceae, Bacteroidaceae, Marinilabiliaceae, Morphyromonadaceae, Cyclobacteriaceae, Cytophagaceae, Flammeovirgaceae, Rhodothermaceae, Blattabacteriaceae, Cryomorphaceae, Flavobacteriaceae, Schleiferiaceae, Chitinophagaceae, Saprospiraceae, Sphingobacteriaceae, Chlamydiaceae, Parachlamydiaceae, Simkaniaceae, Waddliaceae, Bacillaceae, Listeriaceae, Paenibacillaceae, Planococcaceae, Staphylococcaceae, Aerococcaceae, Carnobacteriaceae, Enterococcaceae, Lactobacillaceae, Leuconostocaceae, Streptococcaceae, Clostridiaceae, Eubacteriaceae, Heliobacteriaceae, Peptococcaceae, Ruminococcaceae, Caulobacteraceae, Hyphomonadaceae, Bradyrhizobiaceae, Brucellaceae, Hyphomicrobiaceae, Methylobacteriaceae, Rhizobiaceae, Xanthobacteraceae, Rhodobacteraceae, Acetobacteraceae, Rhodospirillaceae, Alcaligenaceae, Burkholderiaceae, Comamonadaceae, Oxalobacteraceae, Neisseriaceae, Spirillaceae, Rhodocyclaceae, Desulfobacteraceae, Cystobacteraceae, Myxococcaceae, Nannocystaceae, Phaselicystidaceae, Polyangiaceae, Sandaracinaceae, Syntrophaceae, Syntrophobacteraceae, Syntrophorhabdaceae, Campylobacteraceae, Helicobacteraceae, Acidithiobacillaceae, Aeromonadaceae, Succinivibrionaceae, Alteromonadaceae, Celerinatantimonadaceae, Colwelliaceae, Ferrimonadaceae, Idiomarinaceae, Moritellaceae, Pseudoalteromonadaceae, Psychromonadaceae, Shewanellaceae, Chromatiaceae, Ectothiorhodospiraceae, Granulosicoccaceae, Enterobacteriaceae, Coxiellaceae, Legionellaceae, Alcanivoracaceae, Hahellaceae, Halomonadaceae, Litoricolaceae, Pasteurellaceae, Moraxellaceae, Pseudomonadaceae, Vibrionaceae, Nevskiaceae, Sinobacteraceae, thomonadaceae Brachyspiraceae, Brevinemataceae, Leptospiraceae, Spirochaetaceae, Haloplasmataceae and Mycoplasmataceae;* even more preferably, the donor organism is a bacterium selected from the group consisting of the families *Acidobacteriaceae, Actinomycetaceae, Actinopolysporaceae, Corynebacteriaceae, Mycobacteriaceae, Nocardiaceae, Brevibacteriaceae, Microbacteriaceae, Micrococcaceae, Promicromonosporaceae, Aarobacteraceae, Micromonosporaceae, Nocardioidaceae, Propionibacteriaceae, Actinosynnemataceae, Pseudonocardiaceae, Streptomycetaceae, Nocardiopsaceae, Streptosporangiaceae, Bifidobacteriaceae, Bacteroidaceae, , Flavobacteriaceae, Chlamydiaceae, Bacillaceae, Listeriaceae, Paenibacillaceae, Planococcaceae, Staphylococcaceae, Aerococcaceae, Enterococcaceae, Lactobacillaceae, Leuconostocaceae, Streptococcaceae, Clostridiaceae, Eubacteriaceae, Heliobacteriaceae, Caulobacteraceae, Methylobacteriaceae, Rhizobiaceae, Xanthobacteraceae, Rhodobacteraceae, Acetobacteraceae, Rhodospirillaceae, Alcaligenaceae, Burkholderiaceae, Comamonadaceae, Neisseriaceae, Myxococcaceae, Nannocystaceae, Phaselicystidaceae, Polyangiaceae, Sandaracinaceae, Syntrophaceae, Syntrophobacteraceae, Syntrophorhabdaceae, Campylobacteraceae, Aeromonadaceae, Alteromonadaceae, Pseudoalteromonadaceae, Psychromonadaceae, Shewanellaceae, Enterobacteriaceae, Alcanivoracaceae, Hahellaceae, Pseudomonadaceae, Vibrionaceae and Mycoplasmataceae;* most preferably, the donor organism is selected from the group consisting of *Acidobacteriaceae, Actinomycetaceae, Actinopolysporaceae, Corynebacteriaceae, Nocardiaceae, Brevibacteriaceae, Micromonosporaceae, Nocardioidaceae, Actinosynnemataceae, Pseudonocardiaceae, Streptomycetaceae, Nocardiopsaceae, Streptosporangiaceae, Bacillaceae, Listeriaceae, Lactobacillaceae, Leuconostocaceae, Eubacteriaceae, Xanthobacteraceae, Rhodobacteraceae, Acetobacteraceae, Rhodospirillaceae, Alcaligenaceae, Burkholderiaceae, Comamonadaceae, Myxococcaceae, Nannocystaceae, Phaselicystidaceae, Polyangiaceae, Syntrophobacteraceae, Syntrophorhabdaceae, Campylobacteraceae, Aeromonadaceae, Enterobacteriaceae, Alcanivoracaceae and Pseudomonadaceae;* and
even most preferred, the donor organism is selected from the group consisting of *Actinomycetaceae, Actinopolysporaceae, Brevibacteriaceae, Nocardioidaceae, Micromonosporaceae, Actinosynnemataceae, Streptomycetaceae, cardiopsaceae, Pseudonocardiaceae, Bacillaceae, Listeriaceae, Lactobacillaceae, Leuconostocaceae, Xanthobacteraceae,* and *Rhodobacteraceae.*

In a further preferred embodiment, the donor organism is selected from the group consisting of *Streptomyces ambofaciens, Streptomyces avermitilis, Streptomyces capreolus, Streptomyces carcinostaticus, Streptomyces cervinus, Streptomyces clavuligerus, Streptomyces davawensis, Streptomyces fradiae, Streptomyces griseus, Streptomyces hygroscopicus, Streptomyces lavendulae, Streptomyces lividans, Streptomyces natalensis, Streptomyces noursei, Streptomyces kanamyceticus, Streptomyces nodosum, Streptomyces tsukubaensis, Streptomyces sp., Streptomyces coelicolor, Streptomyces cinnamonensis, Streptomyces platensis, Streptomyces rimosus, Streptomyces spectabilis, Streptomyces verticillus, Streptomyces venezuelae, Streptomyces violaceoniger, Streptomyces violaceoruber, Streptomyces mobaraensis, Streptomyces peuceticus, Streptomyces coeruleorubidus, Actinomyces bovis, Actinomyces bowdenii, Actinomyces canis, Actinomyces catuli, Actinomyces coleocanis, Actinomyces europaeus, Actinomyces funkei, Actinomyces georgiae, Actinomyces hongkongensis, Actinomyces israelii, Actinomyces marimammalium, Actinomyces meyeri, Actinomyces oricola, Actinomyces slackii, Actinomyces streptomycini, Actinomyces suis, Actinoplanes teichomyceticus, Actinosynnema pretiosum, Pseudonocardia autotrophica, Amycolatopsis mediteranei, Amycolatopsis orientalis, Saccharopolyspora eritharaea, Saccharopoylspora hirsuta, Pseudomonas aeruginosa, Pseudomonas putida, Pseudomonas syringae, Xantomonas orysae, Burkholderia cenocepacia, Bacillus subtilis, Bacilllus licheniformis, Bacillus megaterium, Mixococcus xantus, Lactobacillus acidophilus, Lactobacillus casei, Lactobacillus brevis, Lactococcus lactis, Brevibacterium casei, Brevibacterium oxydans, Bifidobacterium bifidum, Bifidobacterum longum, Commamonas sp., Corynebacterium glutamicum, Rhodococcus sp., Serratia marescens, Rhizobium trifolii, Rhizobium radiobacter, Sporangium cellulosum, Chondromyces crocatus, Gluconobacter oxydans, Micromonospora grisea, Micrococcus sp., Micromonospora rhodorangea, Micromonospora zionensis, Micromonospora purpurea, Micromonospora inyoensis, Micromonospora sagamiensis, Palnomonospora parontospora, Dactylosporangium matsuzakiense. Nocardia lactamdurans, Bacillus colistinus, Planobispora rosea, Streptomyces arenae, Streptomyces antibioticus, Streptomyces kasugaensis, Streptomyces mitakaensis, Streptomyces bikiniensis, Streptomyces alboniger, Streptomyces tenebrarius, Bacillus circulans, Streptomyces ribosidificus, Bacillus colistinus, Streptomyces tenjimariensis, and Streptomyces pactum;* preferably, the donor organism is *Streptomyces clavuligerus.*

DNA fragments in the genome of the donor organism preferably contain (a) gene cluster(s). In a preferred embodiment of the present invention, said DNA fragments or gene clusters comprise a whole, or a portion of a gene cluster that encodes one or more gene product(s) that are part of a specific biosynthetic pathway for secondary metabolites. In a preferred embodiment of the present invention, the gene clusters encode e.g. multienzyme systems responsible for the synthesis of secondary metabolites. In one embodiment of the present invention, the gene clusters that are desired to be cloned encode the secondary metabolites such as beta-lactams, alkaloids, terpenoids, glycosides, natural phenols, phenazines, biphenyls and dibenzufuranslarge, polyketides, complex glycosides, nonribosomal peptides, and hybrids of the above or hybrids of the afore-mentioned. In a preferred embodiment, the gene clusters intended for being cloned and/or transferred within the meaning of the present invention encode any known or putative cluster of genes for which at least one gene is predicted to encode an activity associated with biosynthesis of secondary metabolites as known in the art.

In this context, beta lactams can be selected from the group consisting of an antibiotic or a lactamase inhibitor. Well known examples of beta lactams are penicillins, cephalosporins, cephamycins, carbapenems, clavams etc.. Alkaloids can for instance be selected from the goup consisting of an analgetic, an alkaloid acting on adrenergic system, an antimalaric, etc.. Well known examples of alkaloids are ergot alkaloids, opioids, quinines, reserpine, caffeine, and the like. Glycosides can for instance be selected from the group consisting of an antibiotic and a cardiotonic. Well known examples of glycosides are streptomycine, kanamycine, gentamycine, tobramycine, digitoxine, digoxine. Terpenoids can for instance be selected from the goup consisting of anticancer agents, an antimalarial agent and an anticoagulant. Well known examples of terpenoides are for instance taxol, artemisine, coumarines etc. Hybrids of the afore-mentioned chemical groups can for instance be selected from the group of an antibiotic, an antifungal, an anticancer agent, an antigout agent, or an inhibitor of lipases. Further examples can be derived from suitable textbook literature or further publications, such as David J. Newman and Gordon M. Cragg, 2007, Natural Products as Sources of New Drugs over the Last 25 Years, J. Nat. Prod. 2007, 70, 461-477.

The polyketide can for instance be selected from the group consisting of an antibiotic, an immunosuppressant, an anti-cancer agent, an antifungal agent and a cholesterol lowering agent.

The term "non-ribosomal polypeptides" refers to polypeptides that are synthesized using a modular enzyme complex, which functions much like a conveyor belt. Nonribosomal peptides are confined primarily to unicellular organisms, plants and fungi. All of these complexes are laid out in a similar fashion, and they can contain many different modules to perform a diverse set of chemical manipulations on the developing product. In general, these peptides are cyclic (often with highly-complex cyclic structures), although linear nonribosomal peptides are common. Since the system is modular and closely related to the machinery for building fatty acids and polyketides, hybrid compounds are often found. Oxazoles, thiazoles and their reduced counterparts often indicate that the compound was synthesized in this fashion. Other examples of non-ribosomal polypeptides include vancomycin, thiostrepton, ramoplanin, teicoplanin, gramicidin, and bacitracin.

The DNA fragment to be cloned (and finally being present in the BAC-vector) has a size as defined herein, e.g of from suitably at least about 5 kb, suitably to about 500 kb, preferably of from about 10 kb to about 300 kb. By applying the method(s) according to the present invention, such large DNA fragments can be cloned in their entirety, without the need for a previous preparation and screening of BAC- or Cosmid-libraries.

The BAC-vector comprising the elements (a1) to (a5) can be transferred to the donor organism by applying any suitable method that is known to a person skilled in the art, e.g. via conjugation. By doing so, in a first step the vector to be transferred is for instance introduced via electroporation into an electrocompetent cell such as E. coli (e.g. E. coli strain ET12567) that additionally contains a conjugation helper plasmid. Afterwards, conjugation procedure is carried out under appropriate conditions that are known to a person skilled in the art. A suitable protocol is for instance described in Kieser et al., 2000 (Practical Streptomyces genetics, A laboratory Manual. ISBN0-7084-0623-8). As a donor organism, for instance S. clavuligerus is used.

After having transferred the BAC-vector into the donor organism, a selection step for the donor organism that contains the BAC-vector incorporated into its genome (this means the exoconjugant) can be carried out. Such a selection step can be carried out by applying any suitable method that is known to a person skilled in the art. Some of said methods are described elsewhere herein. In a preferred embodiment, the selection is carried out based on the presence of an antibiotic resistance marker in the transferred DNA (e.g. apramycin resistance).

In a next step, the genomic DNA from the donor organism is isolated. This isolation step can be carried out by any suitable method that is known to a person skilled in the art. One possible protocol is for instance described in Keiser T., Bibb M.J., Chater K.F., Hopwood D., 2000. Practical Streptomyces genetics. Norwich, John Innes Foundation.

The isolated genomic DNA can then be cleaved, purified e.g. by using the phenol/chloroform extraction and ethanol precipitation protocol from Sambrook, and Russell, 2000. Molecular Cloning: A Laboratory Manual, ISBN-978-087969577-4, and self-ligated in a cyclisation reaction. After ligation, a further DNA purification step can be carried out. In general, the genomic region of the gene cluster to be cloned is known, including restriction enzyme sites. Therefore, the restriction enzymes that are used for carrying out the cleavage reaction are chosen such that the resulting cleaved DNA fragments preferably comprise the whole gene cluster to be cloned. In a preferred embodiment, one restriction site is located at the border of the targeted gene clusted, opposite to the BAC integration site (homologous cluster) and the second site is located in the BAC-vector, adjacent to the target homologous fragment. More precisely the BAC contained restriction site is located at the border of the homologous fragment on the side pointing towards the targeted gene cluster. Therefore, an improved BAC vector can be provided wherein majority of the BAC backbone restriction sites are removed and a large multiple cloning site is integrated. The cyclized rescued DNA fragments represent, or are part of, respectively, a BAC-vector, further comprising the rescued DNA fragment (e.g. the gene cluster or parts thereof) that was located adjacent to the integration site in the donor organism's genome.

In a next step, the obtained genomic DNA which is now present in a BAC-vector derived from the BAC-vector used in step (a) is transferred into a prokaryotic organism. Said prokaryotic organism the genomic DNA from step (b) or the BAC-vector derived from the BAC-vector used in step (a), respectively, is transferred to can be any suitable prokaryotic organism that is known to a person skilled in the art. In a preferred embodiment, said prokaryotic organism is an Escherichia (E.) coli strain, preferably an electrocompetent E. coli strain such as DH5α, and the transfer of DNA is carried out by electroporation.

After the transfer of the genomic DNA that is comprised in the BAC-vector, a selection step is carried out for a prokaryotic organism that contains said transferred BAC-vector. This selection is for instance based on an antibiotic resistance, e.g. apramycin resistance.

Optionally, the BAC-vector can then be isolated form the prokaryotic organism by using a suitable protocol, e.g. by using the PhasePrep BAC DNA Kit (Sigma, USA) according to the manufacturer's recommendations. As already described elsewhere herein this step is optional. For instance, a helper plasmid allowing conjugal transfer can be directly provided to the DH5 strain harbouring the BAC-vector with the cloned fragment. In this case this strain is directly used for transfer to host organism. Alternatively, the BAC-vector can be rescued already into a ET12567 pUZ8002 strain. Another alternative is a so called three-patental conjugal transfer.

Finally, the optionally isolated BAC-vector is introduced into the host cell, e.g. via conjugation as already described elsewhere herein, followed by an attP/B site/integrase mediated integration of the BAC-vector in the genome of the host cell.

If the plasmid rescue method (II) is carried out, in step (f) the optionally isolated BAC-vector has to be introduced into a host cell that is provided with a functional integrase, e.g. a functional ΦC31 integrase. If the plasmid rescue method (I) is carried out, it is not necessary to provide the host cell with an integrase.

However, independent of which method ((I) or (II)) is used, by applying the method(s) according to the present invention the BAC-vector derived from the BAC-vector of step (a) that comprises the genomic DNA of the donor organism, which genomic DNA, in turn, comprises the DNA fragment to be cloned, does not have to be modified prior to its introduction into the host cell and, preferably, into the host cell's genome. Such a BAC-vector that does not have to be modified is for instance the BAC-vector of step (c), (d), (e), and/or (f). In a preferred embodiment, the BAC-vector from step (c) is not modified during steps (c) to (f). In a further preferred embodiment, the BAC-vector from step (c) is not modified during steps (c) to (f) prior to its introduction into the host cell and, preferably, into the host cell's genome. This is an advantage over prior art protocols, as in the methods described in prior art modification step(s) was (were) necessary, e.g. for ensuring proper integration in the hosts cell's genome. Within the meaning of the present application, the term "modification step" or "modification" denotes any (genetic) manipulation e.g. carried out by a human that is carrried out on the BAC-vector that results in an amended BAC-vector, amended e.g. in terms of the nucleic acid sequence of the vector. A modification step can for instance be a cloning step. Such a cloning step e.g. results in the deletion or addition of funtional elements such as attP site(s), origin of replication(s), integrase encoding sequence(s), attP-site integrase cassette(s), or in the addition or deletion of restriction enzyme sites. In a preferred embodiment, no attP-site, attP-site integrase cassette, and/or integrase cassette is introduced into said BAC-vector. By avoiding said manipulation step(s) (or by avoiding further cloning step(s), respectively), time-consuming and laborious working steps can be avoided. This avoidance is all the more advantageous as manipulation of large DNA fragments (e.g. fragments having a length of more than 30 kb) is difficult and complicated as standard protocol methods do not apply.

The host cell can be any suitable cell that is known to a person skilled in the art. The host cell can be any microorganism which has the ability to accept foreign DNA via conjugal transfer. A preferred host cell additionally has a ΦC31 specific attB site in the genome, and/or supports replication of the BAC replicon. As exemplified below, both *E. coli* and *Streptomyces,* taxonomically very distant organisms, can be used as hosts. Therefore, the the host organism may be selected from any microorgamism which is convenient for cultivation and preferably has metabolic properties supporting the synthesis of the cloned cluster encoded secondary metabolites. Such organisms may be selected from, but are not limited to, the group consisting of the genera Streptomyces, Saccharopolyspora, Nocardia, Pseudonocardia, Amycolatopsis, Actinobacterium, Pseudomonas, Gluconobacter, Xantomonas, Burkholderia, E. coli, Bacillus, Mixoccoccus, Lactobacillus, Lactococcus, Bifidobacterium. Comamonas, Corynebacterium, Brevibacterium, Rhodococcus, Azotobacter, Citrobacter, Enterobacter, Clostridium, Klebsiella, Salmonella, Saccharomyces, Zygosaccharomyces, Pichia, Kluyveromyces, Candida, Hansenula, Dunahella, Debaryomyces, Mucor, Torulopsis, Serratia, Rhizobium, Zymomonas, Propionibacterium, Acetobacter, Arthrobacter, Ralstonia, Sporangium, Chondromyces, Gluconobacter, Propionibacterium, Aspergillus, Neurospora, Chrysosporium, Thielavia, Neurospora, Aureobasidium, Fihbasidium, Piromyces, Cryplococcus, Acremonium, Tolypocladium, Scytalidium, Schizophyllum, Sporotrichum, Penicillium, Mucor, Fusarium and Trichoderma; preferably the host cell is selected from the group consisting of Streptomyces, Saccharopolyspora, Nocardia, Pseudonocardia, Amycolatopsis, Pseudomonas, Xantomonas, Burkholderia, Bacillus, Mixoccoccus, Lactobacillus, Lactococcus, Brevibacterium, Bifidobacterium, Comamonas, Corynebacterium , Rhodococcus, Saccharomyces, Pichia, Kluyveromyces, Candida, Serratia, Rhizobium, Sporangium, Chondromyces, Gluconobacter, Aspergillus, Neurospora, Acremonium, Penicillium, Mucor and Escherichia coli;
preferably, the host cell belongs to the genus Streptomyces and, more preferably, is selected from the group consisting of Streptomyces clavuligerus, Streptomyces avermitilis, Streptomyces ambofaciens, Streptomyces fradiae, Streptomyces griseus, Streptomyces hygroscopicus, Streptomyces lavendulae, Streptomyces lividans, Streptomyces natalensis, Streptomyces platensis, Streptomyces rimosus, Streptomyces venezuelae, Pseudonocardia autotrophica, Amycolatopsis mediteranei, Amycolatopsis orientalis, Saccharopolyspora eritharaea, Saccharopoylspora hirsute, Pseudomonas aeruginosa, Pseudomonas putida, Pseudomonas syringae, Penicillium citrinum, Penicillium chrisogenium, Xantomonas orysae, Burkholderia cenocepacia, Bacillus subtilis, Bacilllus licheniformis, Bacillus megaterium, Mixococcus xantus, Lactobacillus acidophilus, Lactobacillus casei, Lactobacillus brevis, Lactococcus lactis, Brevibacterium casei, Brevibacterium oxydans, Bifidobacterium bifidum, Bifidobacterum longum, Commamonas sp., Coryneobacterium glutamicum, Rhodococcus sp., Aspergilus nidulans, Aspergillus niger, Aspergillus terreus, Saccaromyces cerevisiae, Pichia pastoris, Kluyveromyces lactis, Candida albicans, Candida antarctica, Serratia marescens, Rhizobium trifolii, Rhisobium radiobacter, Sporangium cellulosum, Chondromyces crocatus, Gluconobacter oxydans, Neurospora crassa, Acremonium chrysogenum and Mucor mucor;
and most preferably, the host cell is selected from the group consisting of S. clavuligerus, S. lividans, S. avermitilis, and E. coli.

The present invention also refers to a BAC-vector comprising (1) an, preferably one, origin of replication, (2) an origin of transfer, (3) a site-specific recombination system containing an attP site however no functional integrase, and (4) a selection marker; as well as to a vector comprising (1) an, preferably one, origin of replication, (2) an origin of transfer, (3) a site-specific recombination system containing a functional integrase and an attP site, (4) a DNA sequence that allows for homologous recombination with a corresponding substantially homologous DNA sequence being present in the genomic DNA of a donor organism, and (5) a selection marker.

In a preferred embodiment of the present invention, the above vectors are BAC-vectors suitable for cloning and/or transfer of a DNA fragment, preferably as described herein. As described above, for this purpose, the respective vector further comprises a DNA sequence that allows for homologous recombination with a corresponding homologous DNA sequence being present in the genomic DNA of a donor organism. In a further preferred embodiment, the vectors are used in methods (I) and (II) as described elsewhere herein, with the vector having no functional integrase preferably being used in method (II) and the vector containing an integrase and an attP site preferably being used in method (I). With regard to the terms "DNA fragment"; "homologous recombination", "BAC-vector", "donor organism", "origin of replication", "site-specific recombination system containing an attP site however no functional integrase", "selection marker" and "DNA sequence that allows for homologous recombination", reference is made to the disclosure elsewhere herein, as well as with the use of said vector.

Further as described above, after having carried out homologous recombination with the genome of a donor organism, the vector further comprises a genomic DNA of the donor organism. This genomic DNA comprises a DNA fragment that is intended to be cloned or transferred, preferably said DNA fragment is as defined elsewhere herein.

Further, the present invention refers to the use of the above vectors for cloning and/or transferring of a DNA fragment as described elsewhere herein.

Further, the present invention refers to a donor organism, preferably to a donor organism as described elsewhere herein, comprising a BAC-vector as defined in item (1) (a1) to (a5) or as defined in any one of items (24) or (25), or (27) to (32) and throughout the specification, and wherein said donor organism does not contain a functional attB site. In a preferred embodiment, said BAC-vector comprises a ΦC31 integrase. The donor organism comprising the above BAC-vector (i.e. the BAC-vector comprising a functional integrase and an attP site) is preferrably used in method (I) as described herein.

Additionally, the present invention refers to a donor organism, preferably to a donor organism as described elsewhere herein, comprising a BAC-vector as defined in item (3) (a1) to (a5) or as defined in any one of items (26) to (32), and throughout the specification. The donor organism comprising the above BAC-vector, i.e. the BAC-vector comprising an attP site however no functional integrase, is preferably used in method (II) as described herein.

In a preferred embodiment, the BAC-vectors of the respective donor-organism are shuttle-BAC-vectors, preferably E. coli - Actinomycetes conjugative vectors.

In a further preferred embodiment, the origin of replication of the respective BAC-vector is ori S, and/or the origin of transfer is ori T.

In a further preferred embodiment, the site-specific recombination system of the respective BAC-vector is a ΦC31 attP-int recombination system.

In a further preferred embodiment, the respective BAC-vectors comprise a prokaryotic F factor partitioning system.

The present invention also refers to an expression system, comprising
(1) a host cell, and
(2) a BAC-vector, wherein said BAC-vector comprises
   i) an, preferably one, origin of replication;
   ii) an origin of transfer;
   iii) a site-specific recombination system containing an attP site and a functional integrase;
   iv) a DNA fragment comprising a whole gene cluster or a part of a gene cluster encoding one or more gene product(s) that is (are) part of a biosynthetic pathway for secondary metabolites; and
   v) a selection marker.

With regard to the terms "expression system", "host cell", "functional integrase", "BAC-vector", "origin of replication", "origin of transfer", "site-specific recombination system", "attP site"; "selection marker", "DNA fragment", "gene cluster", and "biosynthetic pathway for secondary metabolites", reference is made to the description elsewhere herein. Further, in one embodiment, the features i), ii), iii) and v) of the BAC-vector correspond to features (a1), (a2), (a3) and (a5) of the BAC-vector of item (1), and/or to features (1), (2), (3) and (4) of the vector of item (24). In a preferred embodiment, the expression system is used within method (I) as described elsewhere herein.

The present invention further refers to an expression system, comprising
(1) a host cell, wherein said host cell is provided with a functional integrase, and
(2) a BAC-vector, wherein said BAC-vector comprises
   i) an, preferably one, origin of replication;
   ii) an origin of transfer;
   iii) a site-specific recombination system containing an attP site however no functional integrase;
   iv) a DNA fragment comprising a whole gene cluster or a part of a gene cluster encoding one or more gene product(s) that is (are) part of a biosynthetic pathway for secondary metabolites; and
   v) a selection marker.

With regard to the terms "expression system", "host cell", "functional integrase", "BAC-vector", "origin of replication", "origin of transfer", "site-specific recombination system", "attP site"; "selection marker", "DNA fragment", "gene cluster", and "biosynthetic pathway for secondary metabolites", reference is made to the description elsewhere herein. Further, in one embodiment, the features i), ii), iii) and v) of the BAC-vector correspond to features (a1), (a2), (a3) and (a5) of the BAC-vector of item (3), and/or to features (1), (2), (3) and (4) of the vector of item (26). In a preferred embodiment, the functional integrase the host cell is provided with is a ΦC31 integrase. In a further preferred embodiment, the expression system is used within method (II) as described elsewhere herein.

Further, the present invention refers to the use of the expression system for cloning and/or transfer of a DNA fragment as described herein, and/or for expression of said DNA fragment. Preferably, the DNA fragment is a DNA fragment as described elsewhere herein.

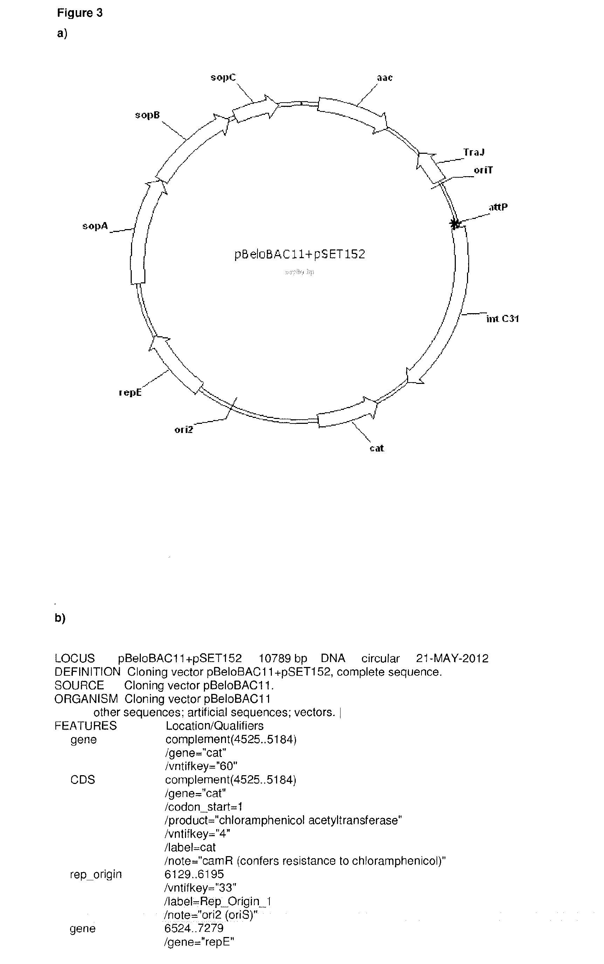

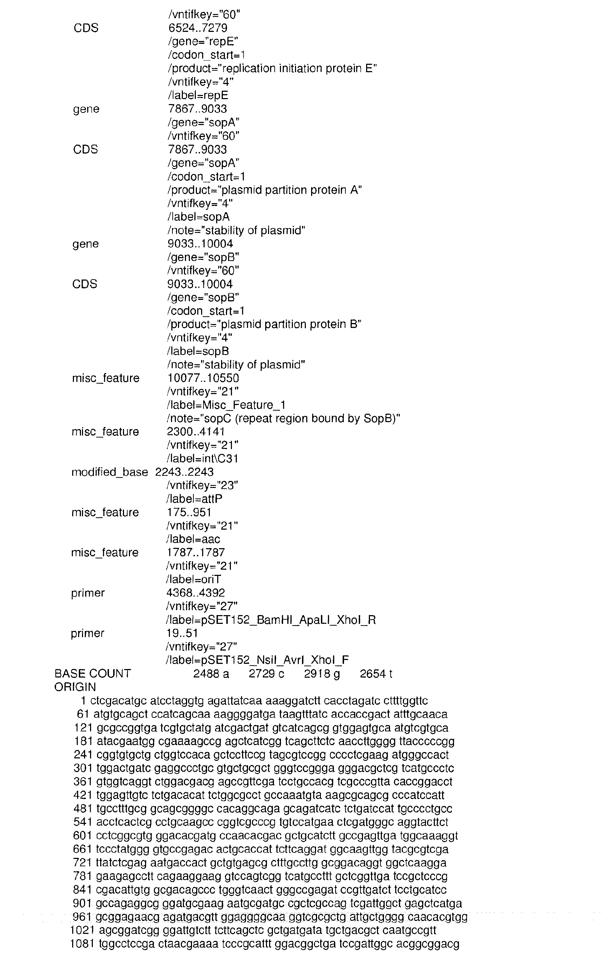

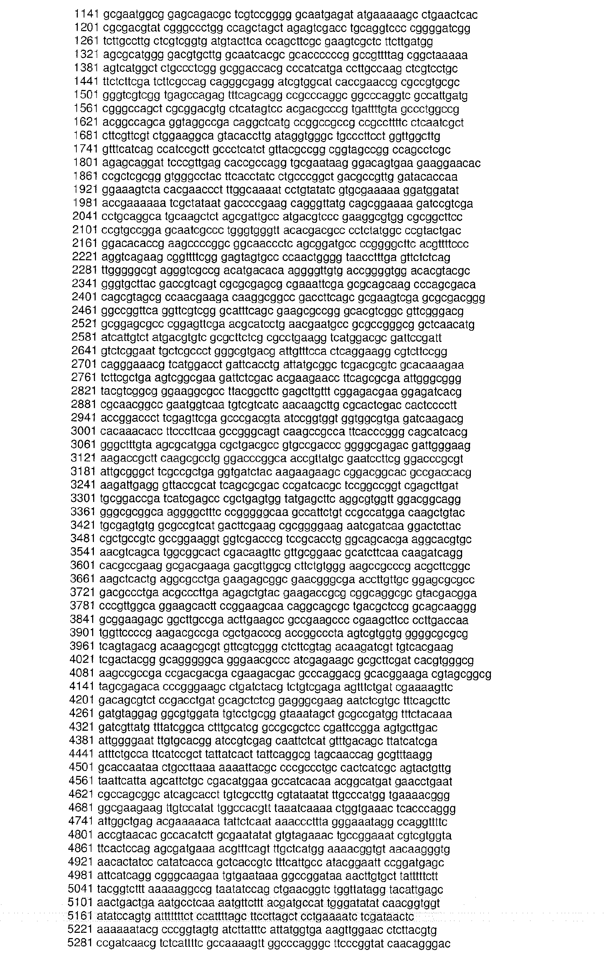

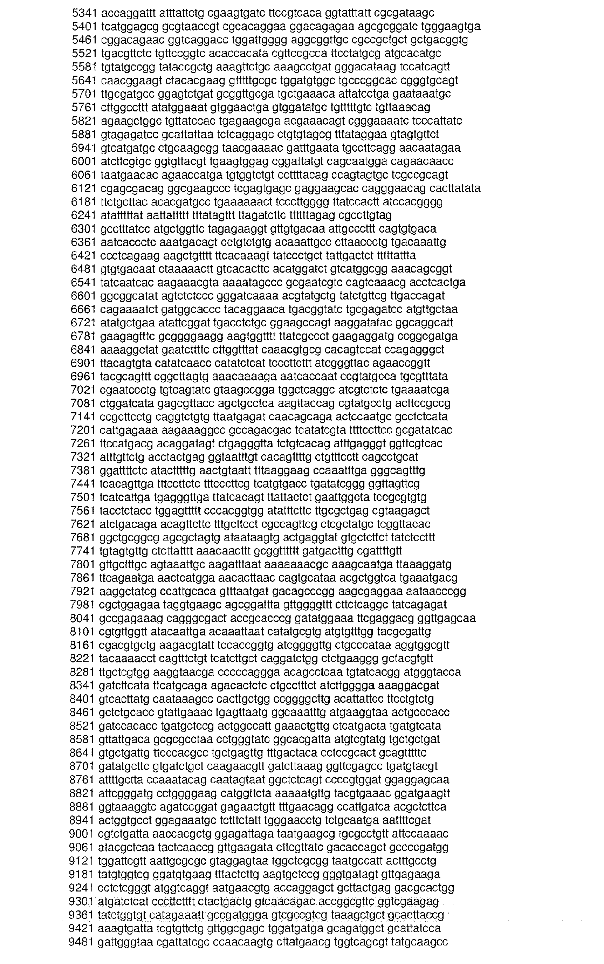

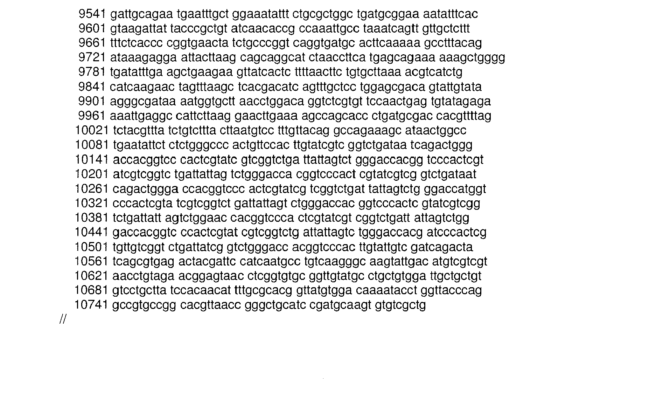

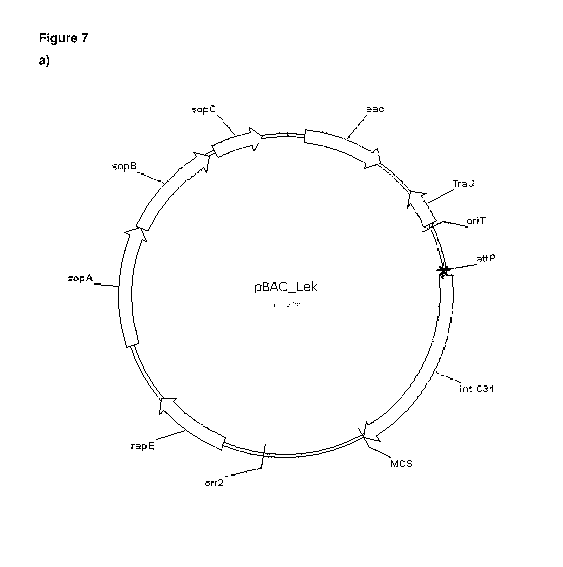

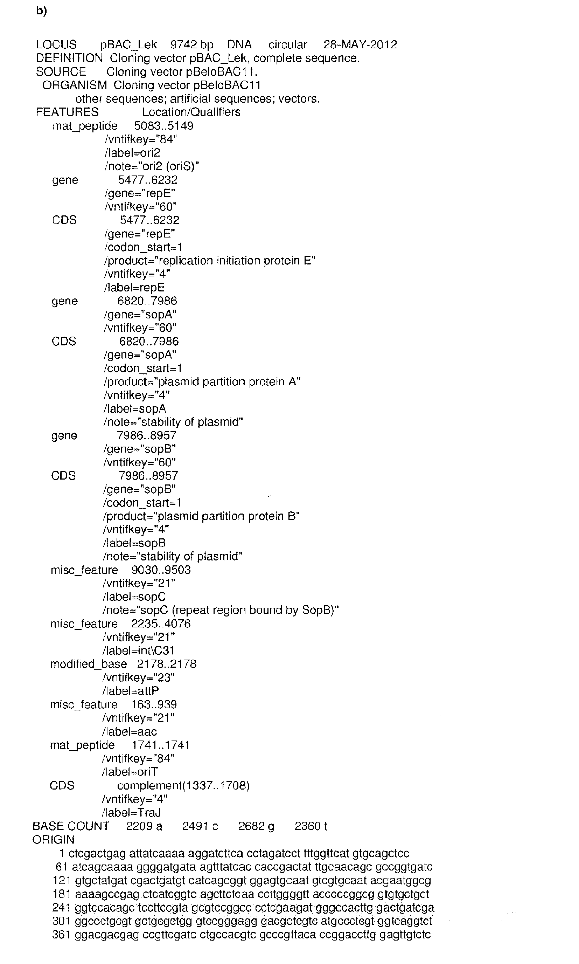

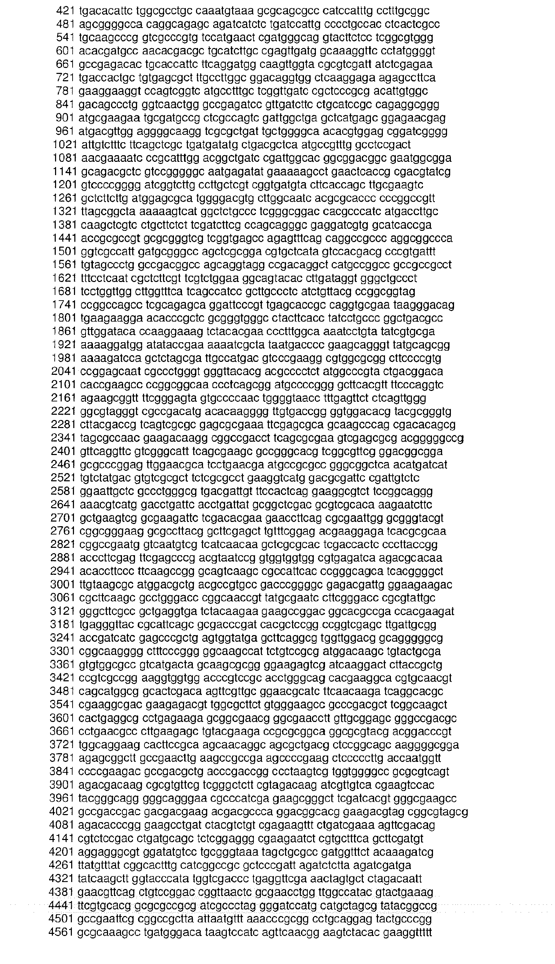

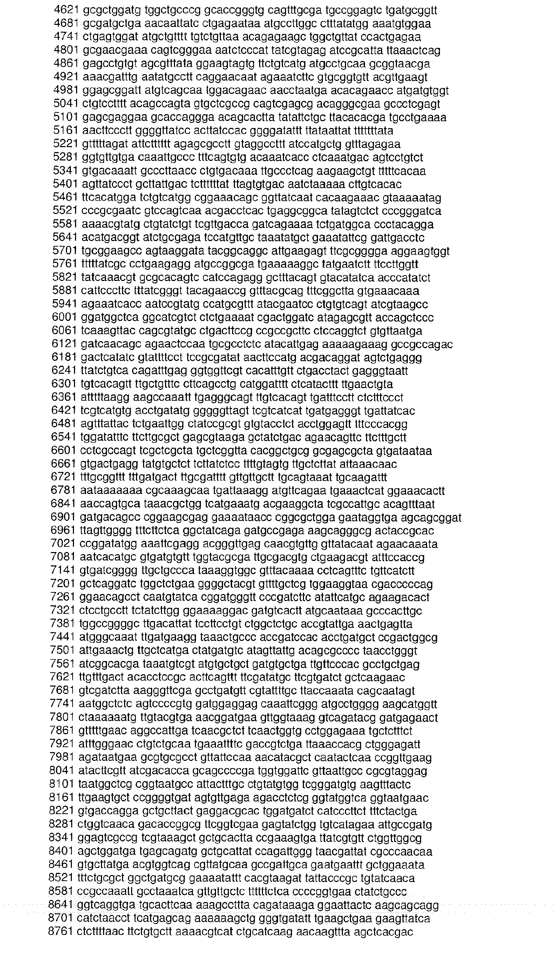

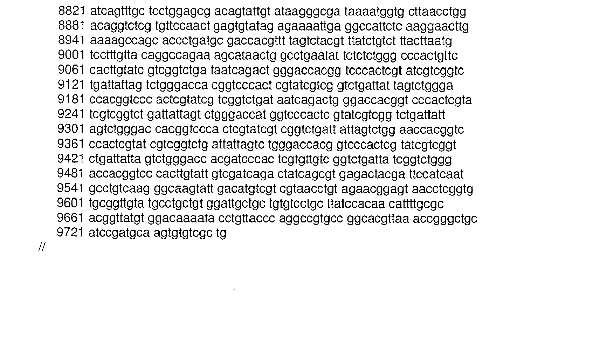

Further, the present invention refers to a vector, preferably a BAC-vector, comprising a sequence according to SEQ ID NO: 3 (SEQ ID NO: 3 is depicted in Fig. 3 b)), and a BAC-vector comprising SEQ ID NO: 3 is for instance the vector pBeloBAC11+pSET152, depicted in Fig. 3 a)), or to SEQ ID NO: 53 (depicted in Fig. 7 b)). SEQ ID NO: 53 represents an improved version of the BAC-vector comprising SEQ ID NO: 3 in that the BAC-vector comprising SEQ ID NO: 53 has deleted restriction sites in the backbone. A BAC-vector that comprises SEQ ID NO: 53 is for instance the vector pBAC_LEK, depicted in Fig. 7 a).

In a preferred embodiment, said vectors additionally comprise a DNA sequence that allows for homologous recombination with a corresponding substantially (or even completely) homologous DNA sequence being present in the genomic DNA of a donor organism. Further preferred, the vectors comprise a DNA fragment as described elsewhere herein. In a further preferred embodiment, the vectors are used for cloning and/or transfer of a DNA fragment, and/or for expression of said DNA fragment.

Therefore, the present invention also refers to the use of the above vectors for cloning and/or transfer of a DNA fragment, and/or for expression of said DNA fragment. In a preferred embodiment, the vectors are used in method (I) as described herein.

### Description of the figures

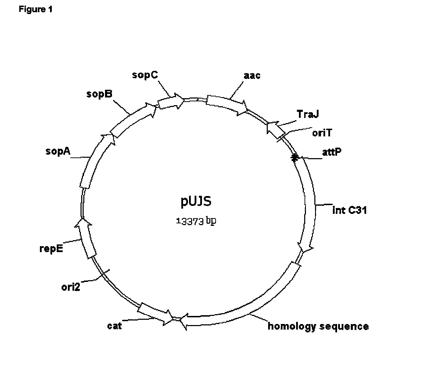

**Fig. 1****:** BAC-vector pUJS.
Figure 1 shows the BAC-vector pUJS.
**Fig. 2****:** Schematic view of gene cluster cloning.

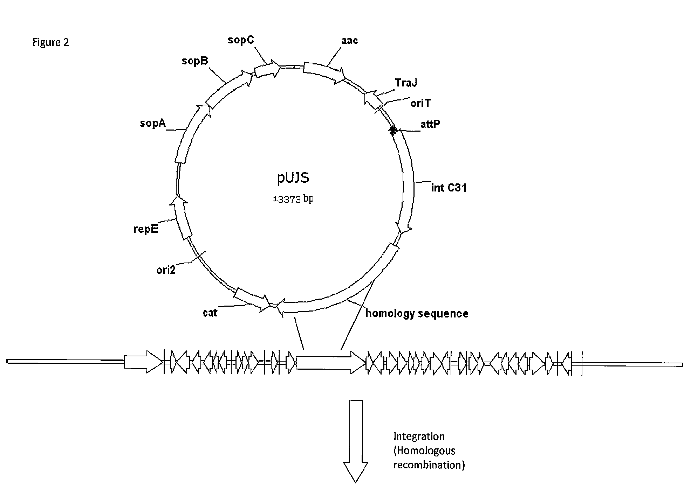

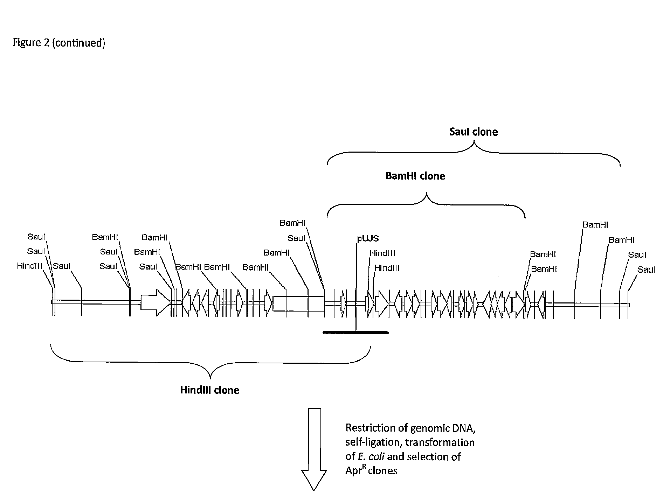

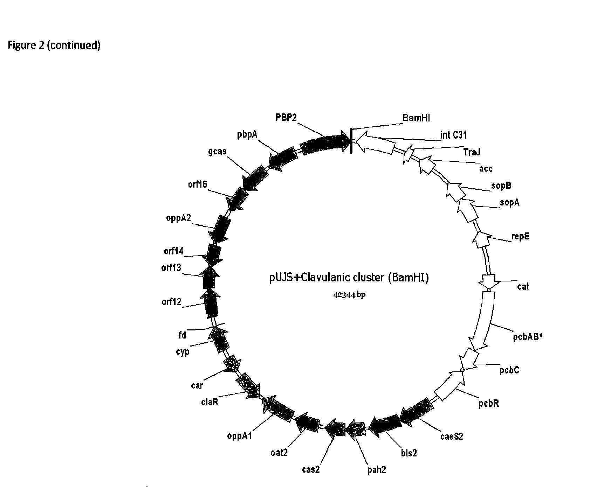

Figure 2 shows a schematic view of gene cluster cloning. The part of the genome sequence represented is GeneBank PRJNA42475: 142996-211017.
**Fig. 3****:** pBeloBAC11+pSET152
Figure 3 a) shows the vector pBeloBAC11+pSET152.
Figure 3 b) shows the various features and the nucleotide sequence (SEQ ID NO: 3) of the BAC-vector pBeloBAC11+pSET152.

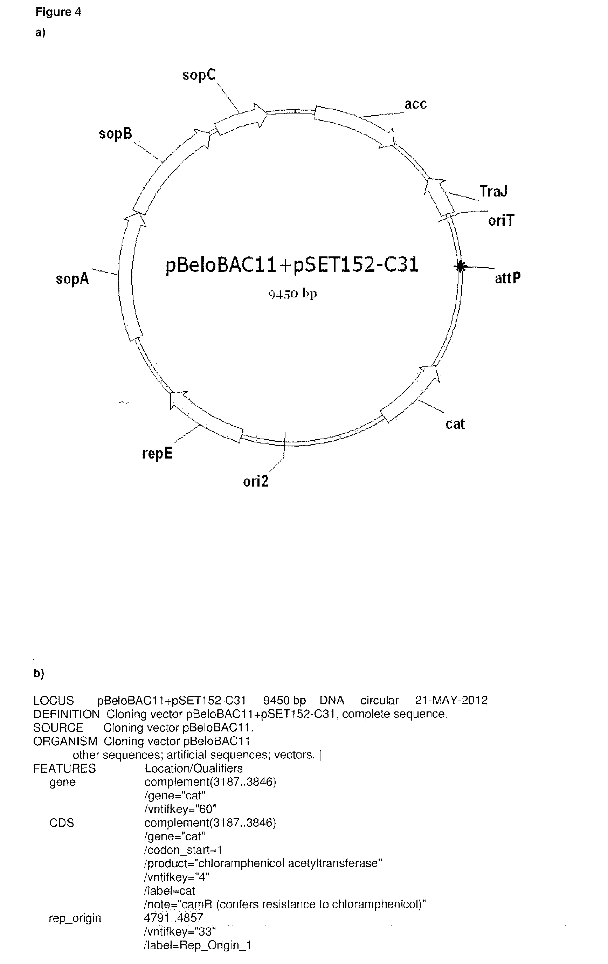

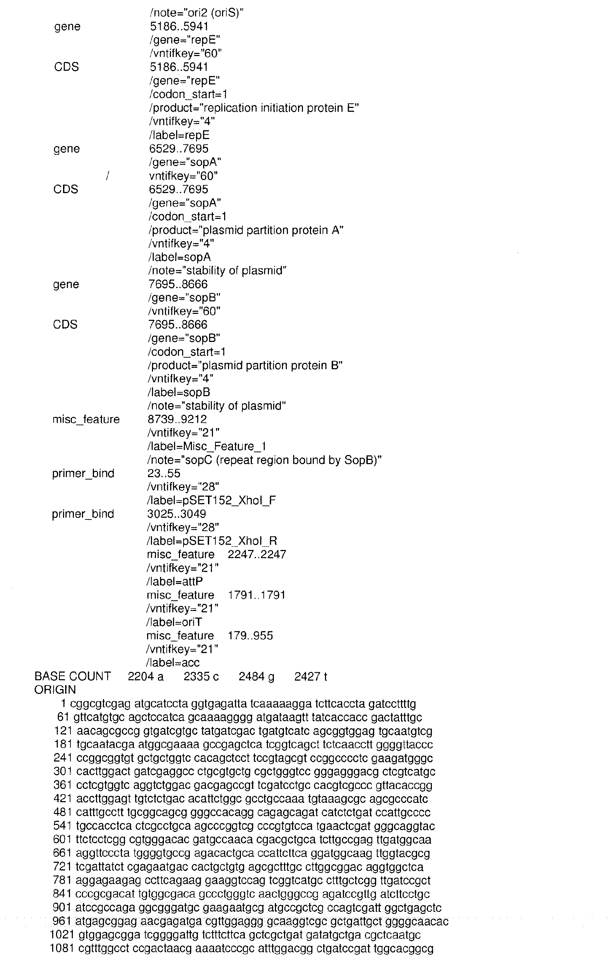

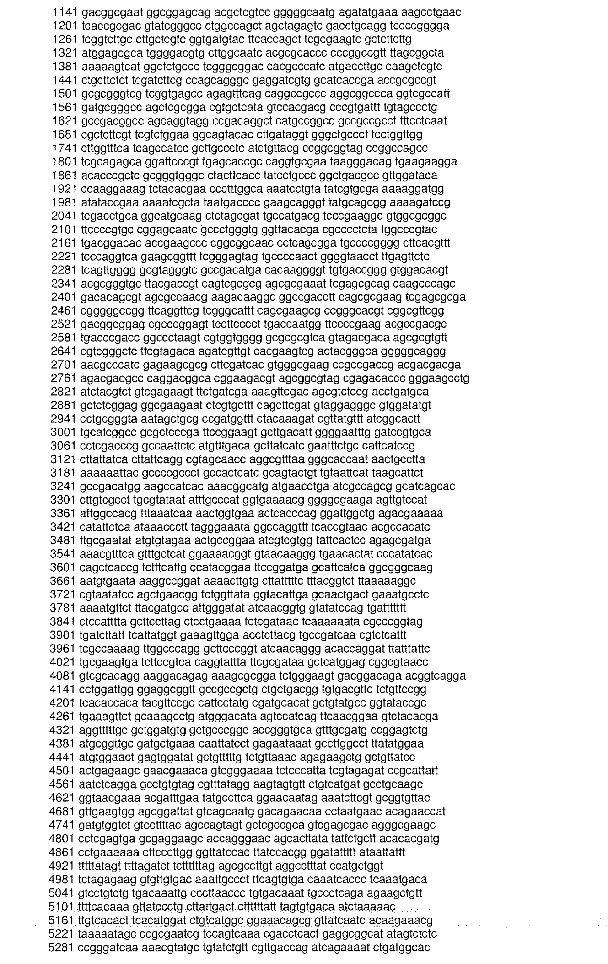

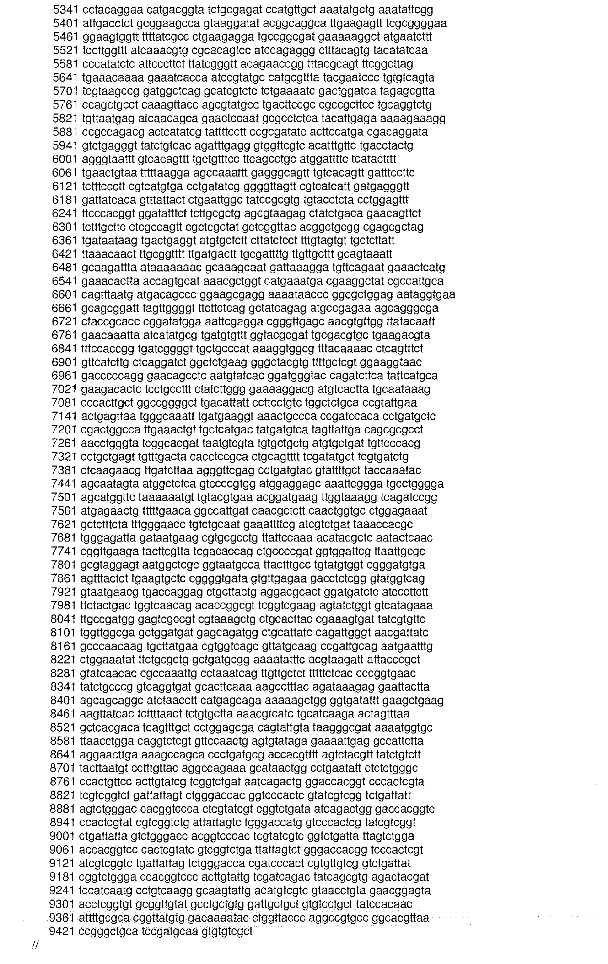

**Fig. 4****:** pBeloBAc11+pSET152-C31
Figure 4 a) shows the vector pBeloBAC11+pSET152-C31.
Figure 4 b) shows the various features and the nucleotide sequence of the BAC-vector pBeloBAC11+pSET152-C31 (SEQ ID NO: 6).

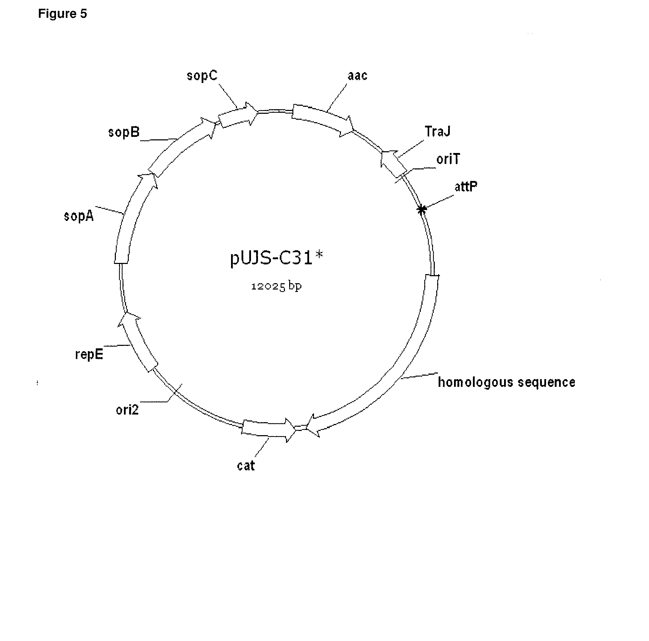

**Fig. 5****:** pUJS-C31
Figure 5 shows the BAC-vector pUJS-C31.

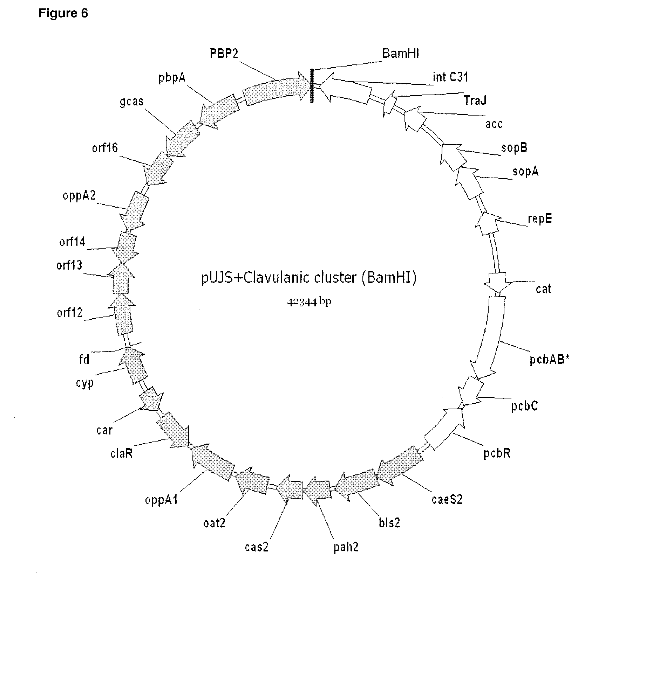

**Fig. 6****:** pUJS+Clavulanic cluster (BamHI)
Figure 6 shows the BAC vector pUJS+Clavulanic cluster (BamHI).
**Fig. 7****:** pBAC_Lek
Figure 7 a) shows the BAC-vector pBAC_Lek
Figure 7 b) shows the various features and the nucleotide sequence of the BAC-vector pBAC_LEK (SEQ ID NO: 53). pBAC_LEK is a vector having deleted restriction sites in the backbone. It has a multiple cloning site containing more than 50 unique restriction sites. All duplicate deletion sites have been deleted from the rest of the BAC-vector backbone. The following unique restriction sites are present in the multiple cloning site: BgIII, Bst98I, AfIII, ClaI, EcoRV, HindIII, KpnI, Acc65I, NdeI, SaII, Bsu36I, SauI, SfuI, BstBI, SpeI, XbaI, MunI, MfeI, AcII, PvuII, BspEI, HpaI, NruI, SexAI, MscI, SnaBI, ApaLI, AscI, BssHII, PvuI, AsiSI, AvrII, BamHI, BfrBI, NsiI, Ppu10I, SphI, NheI, BmtI, BstZ17I, FseI, EcoRI, NotI, Pacl, AseI, PmeI, DraI, SacII, SbfI, PstI, Scal and SrfI. Further, pBAC_LEK comprises one origin of replication, an origin of transfer, a site-specific recombination system containing an integrase and an attP site, and a selection marker.

### Examples

### General Molecular biology methods

The PCR reactions were carried out with PCR Extender System (5PRIME) and the buffer provided by the manufacturer (10x tuning buffer with magnesium) in the presence of 200 µM dNTP, 4% DMSO, 2 µM of each primer, approximately 50 ng of template DNA (or 1 µl when colony PCR templates were used) and 1 unit of enzyme in a final volume of 25 µl for 30 cycles. The thermal profile started with a denaturation step at 94°C for 10 min. The thermal cycle for the following 30 cycles was 94°C for 45 sec (denaturation step), 45 sec of annealing step with temperatures from 54°C to 64°C (depending on the primer pair used), and 72°C for 150 sec (extension step). The final elongation step was carried out at 72°C for 7min.

Cleavage with restriction nucleases, T4 DNA ligase reactions, DNA isolation and purification methods were performed according to the recommendations of the manufacturer. General molecular methods are well known in the art but can be reviewed in Sambrook, and Russell, 2000. Molecular Cloning: A Laboratory Manual, ISBN-978-087969577-4.

### Example 1: construction of pUJS BAC vector

The pUJS BAC vector was assembled by PCR amplification of a fragment containing ΦC31 integrase and *attP* site, *aac(3)IV* gene conferring Apramycin resistance, *traJ* gene and ori T, facilitating conjugal transfer of plasmids, was amplified from pSET152 (Kieser et al., 2000, Practical Streptomyces genetics, A laboratory Manual. ISBN0-7084-0623-8) in a PCR reaction using oligonucleotide primers
CGGCCTCGAGATGCATCCTAGGTGAGATTATCAAAAAGGATCTTCACCTAGATCC
(SEQ ID NO: 1) and
GGCCCTCGAGGGATCCGTGCACAAATTCCCCAATGTCAAGCACTTCC
(SEQ ID NO: 2),
incorporating Nsil, Avrl, Xhol and BamHI, ApaLl, Xhol restriction sites (underlined) respectively. The obtained fragment was cleaved with XhoI restriction endonuclease and ligated to the large fragment obtained with the restriction of pBeloBAC11 with SaII. The resulting vector
pBeloBAC11_pSET152 (Figure 3 a) and b); SEQ ID NO: 3)
was then cleaved with ApaLI and BamHI in order to insert a fragment obtained by cleaving pGEM/homolog with the same fore mentioned restriction endonucleases. The above fragment is homologous to the target integration site (genome sequence GenBank PRJNA42475: 174376-171802) for the plasmid rescue cloning and was obtained by amplification by PCR from the genome of S. *clavuligerus* (oligonucleotide primers:
CGGCGTGCACAGTGTGTGACGGACGGCTATCTCAA
(SEQ ID NO: 4) and
GGCCGGATCCCCTCAGGAGTGGTAGTTGATCGGATCGAGGATGT (SEQ ID NO: 5))
and cloning into the pGEM-T easy PCR cloning vector (Promega) yielding plasmids pGEM/homolog. The sequence analysis of the cloned PCR fragment confirmed its respective sequence as expected from the primer design procedure. The final vector was designated pUJS and its features are shown in Figure 1.

A derivative of the pUJS vector, pUJS-C31 (Fig. 5), was prepared by removing the ΦC31 integrase gene via restriction with SfuI and HindIII, blunt-ending of the cleaved large fragment and cyclisation of the fragment using T4 ligation reaction. Similarly the ΦC31 integrase gene was removed also from pBeloBAC11_pSET152 yielding pBeloBAC11_pSET152-C31 (Figure 4; SEQ ID NO: 6).

The produced vector does not code functional ΦC31 integrase, however the attP site, recognized by the integrase is conserved.

### Example 2: Isolation of S. clavuligerus ATCC27064 ΦC31 attB deletion mutant

### i) Gene deletion construct

The plasmid pKONC was designed as a suicide plasmid for *Streptomyces.* Only E. coli replicative element is included in the plasmid (originating from pSET151, Kieser et al., 2000, Practical Streptomyces genetics, A laboratory Manual. ISBN0-7084-0623-8), therefore upon transformation and exposure to selection pressure only those cells of S. *clavuligerus* form colonies that have the plasmid integrated into the chromosome. Two selection markers are included into the plasmid: aac(3)IV gene conferring apramycin resistance, originating from pSET152, a well known plasmid for use in Streptomyces (Kieser et al., 2000, Practical Streptomyces genetics, A laboratory Manual. ISBN0-7084-0623-8), and codA counterselection marker gene originating from pMG302M, producing a toxic 5-fluoro-uracyl in presence of 5-fluoro-cytosine (Dubeau, et al., 2009).
codA gene was amplified using PCR amplification of pMG302M plasmid DNA using oligonucleotide primers
GCGCTCTAGAGCGCTTGTAGTCGATGGCCT (SEQ ID NO: 7) and
GCGGCATATGGTTGACATCTTTTGCCGATTCTGG (SEQ ID NO: 8).

The PCR-amplified product was fragment was cloned into the pGEM-T easy PCR cloning vector (Promega) yielding plasmid pGEM/codA. The sequence analysis of the cloned PCR fragment confirmed its respective sequence as expected from the primer design procedure.
aac(3)IV gene was amplified using PCR amplification of pSET152 plasmid DNA (Kieser et al., 2000, Practical Streptomyces genetics, A laboratory Manual. ISBN0-7084-0623-8) using oligonucleotide primers
CGCGTCTAGAGGATCTTCACCTAGATCCTTTTGGTTC (SEQ ID NO: 9) and
CCGGGAATTCCCCGATCCGCTCCACGTG (SEQ ID NO: 10).

The PCR-amplified product was cloned into the pGEM-T easy PCR cloning vector (Promega) yielding plasmids pGEM/Apr2. The sequence analysis of the cloned PCR fragment confirmed its respective sequence as expected from the primer design procedure. The plasmid pGEM/CodA (as described above) was cleaved using restriction endonucleases NdeI and XbaI (both Promega), the mixture was resolved on the agarose gel electrophoresis gel. The resulting 1368 bp fragment was purified from the agarose gel. In parallel the plasmid pGEM/Apr2 was cleaved with the restriction endonucleases NdeI and XbaI (Both Promega) so that 3987 bp was isolated from the band cut out of an agarose electrophoresis band. The fragments were purified of the remaining agarose and assembled in a DNA ligation reaction using T4 DNA Ligase (Promega) to yield pGEM/codA_Apr2 with overall length of 5357 bp.

Plasmid pSET151 (Kieser et al., 2000 (Practical Streptomyces genetics, A laboratory Manual. ISBN0-7084-0623-8) was then cleaved using restriction endonuclease EcoRI and NdeI (both Promega) under conditions of partial restriction. 5897 bp fragment containing the larger part of the plasmid was purified of the remaining agarose. In parallel, the plasmid pGEM/coda_Apr2 was cleaved with the restriction endonucleases NdeI and EcoRI (Both Promega) so that 2373 bp fragment containing aac(3)IV and codA genes was isolated from the band cut out of an agarose electrophoresis band. The fragments were assembled in a DNA ligation reaction using T4 DNA Ligase (Promega), yielding pSET151/codA_Apr2 with overall length of 8270 bp.

Finally, the plasmid pSET151/codA_Apr2 was cleaved using restriction endonuclease DraI (Promega) and 5428 bp fragment containing the larger part of the plasmid was purified and selfligated in a DNA ligation reaction using T4 DNA Ligase (Promega) yielding pKONC with overall length of 5428 bp which differs from pSET151/codA_Apr2 in loss of fragment containing genes xylE, tsr and bla. The resulting clones were verified using restriction analysis with several distinct restriction endonucleases.

### ii) Gene deletion construct with ΦC31 attB flanking fragments:

The DNA fragments, flanking the attB site from either side were amplified by PCR from S. clavuligerus genomic DNA using oligonucleotide primers
GCGCGAATTCCCAGGATGGACTTCGCGGTGT (SEQ ID NO: 11) and
GGCCGGATCCCGGGCATGGGAAGACCTCCTT (SEQ ID NO: 12)
for the amplification of the 5' flanking region of attB, and oligonucleotide primers
CGGCGGATCCACGGCCTTCAGCTCTGGGTCA (SEQ ID NO: 13), and
GCCGAAGCTTACCGTTGTCGATGAGCGCCTT (SEQ ID NO: 14)
for the amplification of the 3' flanking region of the attB.

The fragments were cloned into the pGEM-T easy PCR cloning vector (Promega) yielding plasmids pGEM/attB_L1 containing 5' flanking fragment and pGEM/attB_D1 containing 3' flanking fragment. The sequence analysis of the cloned PCR fragments confirmed its respective sequence as expected from the primer design and genomic sequence. The plasmid pKONC (as described above) was cleaved using restriction endonucleases EcoRI and HindIII (both Promega) and the 5377 bp fragment was purified from the agarose gel. In parallel the plasmid pGEM/attB_L1 was cleaved with the restriction endonucleases EcoRI and BamHI (Both Promega) so that 976 bp 5' flanking fragment was isolated. The 1079 bp 3' fragment was excised from pGEM/attB_D1 using BamHI and HindIII and purified. The fragments were assembled in a single step DNA ligation reaction using T4 DNA Ligase (Promega) yielding the final construct designated pKONC/attB with overall length of 7454 bp.

### iii) Selection of the ΦC31 attB deletion strain.

Plasmid construct pKONC/attB was introduced by transformation into electrocompetent E. coli strain ET12567 containing the conjugation helper plasmid pUZ8002 (Paget et al., 1999 J. Bacteriol. 181: 204-211). Conjugation procedure was done as described in Kieser et al., 2000 (Practical Streptomyces genetics, A laboratory Manual. ISBN0-7084-0623-8) using S. clavuligerus ATCC 27064 spores. KKA-2L agar plates were used for selection of exoconjugants and apramycin (Sigma) was used for selection at 60 µg/ml. Transformation with vector pKONC/attB yielded 12 exconjugants resistant to apramycin. As the plasmid pKONC/attB is unable to replicate itself in S. clavuligerus, the exoconjugants must have had the said plasmid integrated into the genome. Only tops of exconjugant colonies were transferred in a dense spread to KKA-2L medium supplemented with 60 µg/ml apramycin (KKA-2L Apr) and grown at 25°C for 10 days. Exoconjugants were tested by a PCR method, described later in detail, and were shown that they all contain a single crossover genotype as expected.

Selection for stable secondary crossover recombinant strains of S. clavuligerus with disrupted attB site was achieved by counterseletion to codA (Cytosine-deaminase) marker gene with fluoro-cytosine (Sigma) and therefore loss of all genetic markers together with the pKONC/attB plasmid backbone. The approach is known by skilled persons as scarless gene deletion (Dubeau et al., 2009).

**Table 1.: The composition of the KKA-2L medium and the mineral solution used within.**

| **Material** | **Amount** |
|---|---|
| Corn dextrine (ROQUETTE) | 10 g |
| K₂HPO₄ | 1 g |
| MgSO₄ × 7H₂O | 1 g |
| NaCl | 1 g |
| (NH₄)₂SO₄ × 7H₂O | 2 g |
| CaCO₃ | 4 g |
| Mineral solution | 1 ml |
| Bacto agar (DIFCO BD) | 20 g |
| Tap water | To a final volume of 1 liter |
| | |

| **Material** | **Amount** |
|---|---|
| 5M HCl | 10 ml |
| CaCl₂ × 2H₂O | 25 g |
| NaCl | 12 g |
| MgCl₂ × 6H₂O | 66 g |
| KCI | 100 g |
| FeCl₃ × 6H₂O | 5 g |
| ZnCl₂ | 0.7 g |
| CuCl₂ × 2H₂O | 0.3 g |
| MnSO₄ × H₂O | 0.4 g |
| Ultra pure water | To a final volume of 1 liter |

| | |
|---|---|
| The pH is naturally ~ 7.0, there is no pH adjustment prior to sterilization. Sterilization is performed at 121±2°C, 220±10 kPa for 20 minutes. | |

Spores of S. *streptomyces* exconjugants with vector pKONC/attB, grown at 25°C on KKA-2L medium supplemented with 60 µg/ml apramycin were inoculated into liquid SNK-t medium with apramycin (60 µg/ml) and grown in deep-well microliter plates at 28°C and 260 rpm to produce dense mycelium culture. After 24h, this culture was subcultivated in parallel wells of fresh deep-well microtiter plates, one containing SNK-t medium supplemented with 60 µg/l apramycin and the other containing SNK-t medium supplemented with 100 µg/l 5-fluoro-cytosine (FC) medium and grown at 25°C and 260 rpm until growth appeared in the wells containing 5-fluoro-cytosine. The cultures were subcultivated repeatedly in parallel wells, as soon as growth appeared in the SNK-t FC containing wells in a manner described above (the 0.1% inoculum used, was always taken from the preceding generation in SNK-t FC wells).

**Table 2.: The composition of the SNK-t medium.**

| **Material** | **Amount** |
|---|---|
| Bacto Soytone (DIFCO BD) | 20 g |
| NaCl | 5 g |
| K₂HPO₄ | 2.5 g |
| Glycerol (Sigma) | 24 g |
| soya oil (GEA) | 5 g |
| Ultra-pure water | To a final volume of 500 ml |

The procedure was repeated for as little as three to as many as ten cycles of selection, until growth was no longer appearing in the wells with SNK-t supplemented with apramycin. The cultures were then plated onto ISP-2 medium containing 100 µg/ml 5-fluoro-cytosine in appropriate dilutions yielding approximately 100 colonies per plate. After 5-10 days, the colonies were spread in parallel onto KKA-2L and KKA-2L Apr agar plates. A large majority of the transferred strains showed growth only on KKA-2L but not on KKA-2L Apr agar plates indicating loss of genetic markers from the genome and thereby successful secondary recombination. Approximately 30 independently obtained secondary recombinants were isolated.

### iv) Conformation of deletion of the ΦC31 attB by genetic characterization

PCR was used for conformation of correct integration and homologous recombination events during the selection pressure cycles. A set of conformation oligonucleotide primers were designed which distinguish between wild type, single crossover situation and the final double recombination situation in the locus of the attB site in the S. *clavuligerus* genome.

DNA templates ware prepared in with a "colony PCR" approach. Specifically, the spores of the tested strains were cultivated in SNK-t medium at 25 °C and 260 rpm for 48 h, followed by separation of the mycelium by filtration, and partial permeabilization of the mycelium using resuspension in TE buffer (Sambrook, and Russell, 2000. Molecular Cloning: A Laboratory Manual, ISBN-978-087969577-4), supplemented with 2.5 µg/ml RNase (Roche, 500 µg/ml). The mycelium as filtered off and the filtrated was used as a DNA template for the PCR reactions with the oligonucleotide primer pair
ACGATGATGCTACCCACCAGTAATAAGTTG (SEQ ID NO: 15) and
GACATAGGCGAGTCCGTTGAAGTCCT (SEQ ID NO: 16).
Expected amplicon lengths are given in table 3.

**Table 3.: The expected lengths of the conformation PCR products**

| | Wild type | Single cross-ower | Double cross-ower |
|---|---|---|---|
| expected lenght | 1399 bp | 994 bp, 1399 bp | 994 bp |

5 strains originating from *S*. *clavuligerus* ATCC 27064 had the expected PCR product profile. These strains were additionally tested for absence of genetic marker cytosine deaminase and marker for apramycin resistance using PCR and were all found negative. Other strains have reverted to the wild type genotype and were therefore discarded. From the 5 isolated strains, one *(S. clavuligerus* ATCC 27064 ΔattB) was selected for further studies.

### Example 3: Characterization of integration frequencies in S. clavuligerus ΦC31 attB deletion mutant

Several vectors were prepared in order to study the frequency of integration via homologous recombination v.s. phage integrase driven integration into the genome of *S*. *clavuligerus* ATCC 27064 w.t. and *S. clavuligerus* ATCC 2706 ΔattB. In addition role of putative pseudo-attB sites in *S. clavuligerus* ATCC 27064 was investigated.

pSET152 (Kieser et al., 2000, Practical Streptomyces genetics, A laboratory Manual. ISBN0-7084-0623-8) was used as the basis of further modified vectors.

pSET152_attP was prepared by modification of the ΦC31 attP site located on the pSET152 plasmid. Targeted PCR mutagenesis was used using QuickChange site-directed mutagenesis kit (Stratagene, USA) and oligonucleotide pair
CCAACTGGGGTAACCTCGAGTTCTCTCAGTTGGGGGCGT (SEQ ID NO: 17) and
ACGCCCCCAACTGAGAGAACTCGAGGTTACCCCAGTTGG (SEQ ID NO: 18).

The underlined part shows the XhoI restriction site which was introduced with the site directed mutagenesis procedure, thereby replacing the original sequence CTTTGAG containing the thymidine pair critical for ΦC31 integrase function on the site with the CTC-GAG. In this way a vector was designed which is practically homologous to the pSET152 however its integration into the chromosome through ΦC31 integrase action was disabled.

Into both pSET152 and pSET152_attP, a fragment homologous to the target site in the genome of *S. clavuligerus* ATCC 27064 was inserted by first cleaving the above vectors and plasmid pGEM/homolog (described in example 1) with EcoRI restriction endonuclease and the assembling the fragments in a ligation reaction to obtain pSET152/homolog and pSET152_attP/homolog.

In addition, to check the integration frequency of alternative phage integrase, specifically ΦBT1 integrase, a pSET152 type vector having the ΦC31 integrase replaced by ΦBT1 integrase was constructed. ΦBT1 integrase gene was chemically synthesized based on the sequence deposited by Gregory et al., 2003 with the GeneBank accession number AJ550940 (sequence position 38669-40697). In addition SphI and BamHI recognition sites were added at 5' and 3' end respectively. The pSET152 was cleaved with BamHI and SphI restriction endonucleases and the large fragment was ligated to BamHI, SphI cleaved ΦBT1 integrase, ΦBT1 attP site fragment. The resulting plasmid was designated pSET152/BT1

Plasmids pSET152, pSET152_attP, pSET152/homolog, pSET152_attP/homolog, pAET/BT1 were introduced by transformation into electrocompetent *E*. *coli* strain ET12567 containing the conjugation helper plasmid pUZ8002 (Paget et al., 1999 J. Bacteriol. 181: 204-211 Conjugation procedure was done as described in Kieser et al., 2000 (Practical Streptomyces genetics, A laboratory Manual. ISBN0-7084-0623-8) using *S*. *clavuligerus* ATCC 27064 w.t. and *S*. *clavuligerus* ATCC 27064 *ΔattB* spores. KKA-2L agar plates were used for selection of exoconjugants and apramycin (Sigma) was used for selection at 60 µg/ml. The experiments were conducted with utmost care that the conjugation conditions were equal for all tested plasmids and were repeated four times independently.

**Table 4.: Frequency of integration, based on the number of exoconjugant collonies after four independent experiments of conjugal transfer.**

| | Plasmid | Number of exoconjugants | | | |
|---|---|---|---|---|---|
| *S. clavuligerus* w.t. | pSET152 | >100000 | >100000 | >100000 | >100000 |
| *S. clavuligerus* w.t. | pSET152/BT1 | 3 | 4 | 10 | 6 |
| *S. clavuligerus* w.t. | pSET152_attP | 0 | 0 | 0 | 0 |
| *S. clavuligerus* w.t. | pSET152_attP/homolog | 115 | 122 | 106 | 131 |
| *S. clavuligerus ΔattB* | pSET152 | 1 | 4 | 0 | 1 |
| *S. clavuligerus ΔattB* | pSET152_attP | 0 | 0 | 0 | 0 |
| *S. clavuligerus ΔattB* | pSET152/homolog | 45 | 52 | 55 | 48 |
| *S. lividans* TK24 | pSET152 | >100000 | >100000 | >100000 | >100000 |
| *S. lividans* TK24 | pSET152/BT1 | 35 | 42 | 56 | 47 |

In another set of experiments, frequencies of integration into the genome of *S*. *clavuligerus* ATCC 27064 w.t. and S. *clavuligerus* ATCC 27064 Δ*attB* for BAC plasmids pBeloBAC11_pSET152, pBeloBAC11_pSET152-C31, pUJS and pUJS-C31 were studied based on the number of exoconjugants obtained after the conjugation procedure as described above.

**Table 5.: Frequency of integration, based on the number of exoconjugant collonies after four independent experiments of conjugal transfer.**

| | Plasmid | Number of exoconjugants | | | |
|---|---|---|---|---|---|
| *S. clavuligerus* w.t. | pBeloBAC11_pSET152 | 350 | 370 | 400 | 350 |
| *S. clavuligerus* w.t. | pBeloBAC11_pSET152-C31 | 0 | 0 | 0 | 0 |
| *S. clavuligerus* w.t. | pUJS-C31 | 1 | 1 | 0 | 0 |
| *S. clavuligerus ΔattB* | pBeloBAC11_pSET152 | 0 | 0 | 0 | 0 |
| *S. clavuligerus ΔattB* | pUJS | 1 | 3 | 5 | 1 |

The results in table 4. show that:
i) The frequency of integration into the genome of *S*. *clavuligerus* w.t., based on the transformation efficiency with pSET152/BT1 (ΦBT1 integrase) are drastically lower than efficiency with pSET152 (ΦC31 integrase). This result shows that the ΦBT1 integrase is not very suitable for the BAC based targeted cloning when considering that frequencies of transformation with conjugal transfer are lower for larger, low copy- number vectors (as shown with the ΦC31 integrase BAC based vectors). A similar result was obtained when *S. lividans* TK24 was used as a host. This approach was abandoned as it would be difficult to introduce the BAC vectors with large inserts into host organisms especially into *S. clavuligerus.*
ii) The frequency of integration of pSET152_attP into *S*. *clavuligerus* w.t. is 0. This confirmed that the vectors used for the study do not have nonspecific means of integration into the *S. clavuligerus* genome, which would be theoretically possible by means of unknown integration mechanism, some sort of replicative mechanism or homologous recombination with unexpected homology in the genome.
iii) The frequency of integration pSET152_attP/homolog is reasonable although a 1000 fold lower than with pSET152. Taking into account the previously described result the only way this integration is possible is via homologous recombination. The lower efficiency of integration with homologous recombination than via the ΦC31 integrase is expected. It is surprising that the frequency of integration with homologous recombination is higher than the frequency of integration via ΦBT1 integrase - result described under i). This is in contrast with the prior knowledge (Gregory et al. 2003).
iv) The frequency of integration into the genome of *S. clavuligerus* Δ*attB* using pSET152 is drastically lower than then *S. clavuligerus* w.t. is the host strain. The few detected transformants are likely obtained by integration of pSET152 into the "pseudo-attB" sites. This result also suggests that the frequency of integration into the "pseudo-attB" sites via the ΦC31 integrase action is negligible compared to integration into the prime attB site and even compared to integration via homologous recombination - result described in iii).
v) The frequency of integration of pSET152/homolog into the genome of *S*. *clavuligerus ΔattB* is approximately half of that obtained by transformation of *S. clavuligerus* w.t. which is likely due to small differences in spore concentration in conjugation procedure. Still the frequency is sufficiently high for further work in targeted insertion of BAC based vector into the genome of *S. clavuligerus.*

The results in Table 5. show that:
i) The frequency of integration into the genome of *S*. *clavuligerus* w.t., based on the transformation efficiency with pBeloBAC11_pSET152 (ΦC31 integrase) are significantly lower than efficiency with pSET152. This result shows that other property of the BAC skeleton has an effect on transformation efficiency, most probably due efficiency of conjugal transfer. The copy number of BAC vectors in the donor *E*. *coli* strain is one while pSET152 type vectors are multi-copy plasmids.
ii) The frequency of integration of pBeloBAC11_pSET152-C31 into *S*. *clavuligerus* w.t. is 0. This shows that the vectors without C31 integrase function or substantial holologous regions to the host organism genome do not have nonspecific means of integration. The frequency of integration of pUJS-C31 which contains such homology is low, however two transformats were obtained with target specific integration of the BAC vector at the site of plasmid rescue cloning. Molecular analysis of the strains showed that integration of the vector occurred by homologous recombination at the correct targeted site in the genome. One of the strains was designated K816 and was used for further work. This results shows possibility to use the BAC complete BAC vector in a w.t. gene cluster donor strain. Upon reintegration or heterologous expression of the cloned fragment, ΦC31 integrase function can be provided *in trans* (such as on temperature sensitive replicative vector) therefore avoiding necessity of preparation of the *ΔattB* gene cluster donor strain. Alternatively, as shown in WO2009/017692 ΦC31 integrase can be provided by subsequent molecular modification of the large BAC vector, which is often difficult due to special methods necessary for molecular modification of large fragments of DNA.
iii) The frequency of integration of pUJS into *S*. *clavuligerus ΔattB* is low compared to pSET152/homolog, likely due to copy number reason. However 10 transformants have been isolated and all have been confirmed at the molecular level to have correct target specific integration in the genome which occurred due to homologous recombination event. One of the strains was designated K771 and was used for further experiments. A schematic figure of the rescued BAC vector comprising the clavulanic cluster is depicted in Fig. 6 ("BAC vector pUJS+Clavulanic cluster (BamHI)").

### Example 4: Plasmid rescue procedure

Strains K816 and K771 were used in the plasmid rescue procedure. Construction of the BAC vectors used to obtain the aforementioned strains, specifically the homology fragment to the target site in the genome, was such that integration of the vectors occurred in the gene *pcbAB* in the cephamycin C gene cluster in *S*. *clavuligerus.* The targeted gene cluster for cloning was the neighbouring clavulanic acid cluster. Due to integration of the BAC vectors into the *pcbAB* gene, a peptide synthase essential for cephamycin C synthesis, the target specific integration could be confirmed both by genotype analysis (PCR) and phenotype analysis (LC-MS; abolishment of cephamycin C accumulation). Figure 2. depicts the complete integration and plasmid rescue procedure for one of the cloned clusters. Experimental procedure is described in detail below.
i) Isolation of genomic DNA: Strains K816 and K771 were grow in the SNK-t medium at 25 °C and 260 rpm for 48h to obtain mycelium. The genomic DNA was isolated according to the procedure described in Keiser T., Bibb M.J., Chater K.F., Hopwood D., 2000. Practical Streptomyces genetics. Norwich, John Innes Foundation.
ii) ~ 1µg of Genomic DNA was cleaved under standard conditions using several restriction endonucleases. Restriction with each of the 3 selected endonucleases would yield distinct gene cluster cloned into the BAC vector. BamHI cleavage would afford clavulanic acid biosynthesis gene cluster, SauI an elongated clavulanic acid biosynthesis gene cluster and HindIII a partial cephamycin C gene cluster. (Figure 2.)
iii) The cleaved DNA was purified according to the phenol/chloroform extraction and ethanol precipitation protocol from Sambrook, and Russell, 2000. Molecular Cloning: A Laboratory Manual, ISBN-978-087969577-4. The DNA was then self-ligated in a T4 ligase (Promega) cyclisation reaction. The ligation conditions were: 14°C 18h, 1 Weiss unit of T4 ligase per 100ul of ligation mixture. After ligation, the DNA was purified by phenol/chloroform extraction (as above), ethanol precipitated and dissolved in 5ul of pure water.
iv) The 5µl of purified ligation mixture DNA was mixed with 30ul *E*. *coli* DH5α Electromax (Invitrogen, USA) electro competent cells and electroporation transformation procedure was carried out as recommended by the manufacturer. The transformants were selected on LB agar plates supplemented with 25ug/ml Apramycin. The plates were incubated overnight at 37°C. The numbers of transformants obtained for each strain - restriction enzyme combination are given in table 6.

**Table 6.: Number of E. coli colonies obtained after plasmid rescue procedure starting from either K771 or K816 genomic DNA cleaved with BamHI, HindIII or SauI restriction endonuclease.**

| | BamHI | Hindlll | Saul |
|---|---|---|---|
| *S. clavuligerus* K771. | *85* | *8* | *6* |
| *S. clavuligerus* K816 | *23* | *34* | *15* |

The BAC DNA was isolated from 5 colonies of each group of E. coli DH5α clones using PhasePrep BAC DNA Kit (Sigma, USA) according to manufacturer's recommendations. The BAC clones were designated pUJS+Clavulanic cluster (BamHI) (for the BamHI clones originating from K771), pUJS/(HindIII) (for the Hindlll clones originating from K771), pUJS+Clavulanic cluster (Saul) (for the Saul clones originating from K771). The BAC clones without ΦC31 integrase function were designated pUJS-C31/BamHI (for the BamHI clones originating from K816), pUJS-C31/Hindlll (for the HindIII clones originating from K816), pUJS-C31/SauI (for the Saul clones originating from K816). Figure 2: Schematic view of gene cluster cloning. The part of the genome sequence represented is GeneBank PRJNA42475: 142996-211017.

### Example 5: Characterization of cloned fragments

The isolated BAC clones were first analyzed by restriction analysis and after conformation of identical restriction pattern for each group of clones, one of each was selected for sequencing. The shot-gun sequencing was performed using Roche FLX Titanium sequencing method. The coverage of the sequencing after assembly with Celera assembler to the reference was higher than 48 for all positions. The ratio of reads assembled to the GeneBank PRJNA42475 reference and reads assembled to the pUJS vector backbone sequence corresponds precisely to the ratio of the length of insert to the length of the pUJS vector backbone. The sequencing result show that the cloned fragment corresponds to the reference GeneBank PRJNA42475 with 100% identity. The cloned fragment length for pUJS+Clavulanic cluster (BamHI) and pUJS-C31/BamHI was 29892 bp and matched position between 174379 and 204271 on the GeneBank PRJNA42475 reference. The cloned fragment length for pUJS/HindIII and pUJS-C31/HindIII was 57433bp and matched position between 116727 and 174160 on the GeneBank PRJNA42475 reference. The cloned fragment length for pUJS+Clavulanic cluster (SauI) and pUJS-C31/SauI was 50427bp and matched position between 174379 and 224806 on the GeneBank PRJNA42475 reference.

### Example 6: Transfer of cloned clusters to S. clavuligerus and S. lividans

Vectors pUJS+Clavulanic cluster (BamHI) and pUJS+Clavulanic cluster (SauI), were introduced by transformation into electrocompetent *E*. *coli* strain ET12567 containing the conjugation helper plasmid pUZ8002 (Paget et al., 1999 J. Bacteriol. 181: 204-211). Conjugation procedure was done as described by Kieser et al., 2000 (Practical Streptomyces genetics, A laboratory Manual. ISBN0-7084-0623-8) using *S*. *clavuligerus* ATCC 27064 w.t. and *S. lividans* TK24 spores. KKA-2L agar plates were used for selection of exoconjugants and apramycin (Sigma) was used for selection at 60 µg/ml. The experiments were conducted with utmost care that the conjugation conditions were equal for all tested plasmids. The number of colonies obtained in three independent experiments is shown in table 7.

**Table 7.: The number of exoconjugant colonies obtained after conjugal transfere of secondary metabolite clusters to S. clavuligerus and S. lividans.**

| | pUJS+Clavulanic cluster (BamHI), | pUJS+Clavulanic cluster (Saul) |
|---|---|---|
| *S. clavuligerus* ATCC 27064 | 31,27,43 | 18, 16,27 |
| *S. lividans TK24* | 233,177,262 | 132, 105, 165 |

**Table 7.**: The number of exoconjugant colonies obtained after conjugal transfere of secondary metabolite clusters to *S. clavuligerus* ATCC 27064 and *S. lividans.*

The presence of heterologous genes in *S. lividans* exoconjugants was confirmed by colony PCR amplification of a number of smaller fragments from the cloned secondary metabolite gene cluster which are distributed along the cluster length. The oligonucleotide primers and the expected length of amplified fragment and the target gene are given in table 8. Al the tested exoconjigants (5 for *S. lividans* TK24 pUJS+Clavulanic cluster (BamHI) and 5 for S. *lividans* TK24 pUJS+Clavulanic cluster (Saul) showed positive PCR with fragments of expected length, whereas in the negative control *S*. *lividans* TK24 no such fragment was observed.

**Table 8.: Oligonucleotide primers used for conformation of transfer of clavulanic acid cluster to S. lividans. *primers were used to confirm complete integration of pUJS+Clavulanic cluster (SauI).**

| | Oligonucleotide primer pair | product length | gene amplified |
|---|---|---|---|
| | AATGATTAAAGGATGTTCAGAATGAAACTCATG (SEQ ID NO: 19) | 958 bp | *sopA* : vector |
| | GGGACTGAGAGCCATTACTATTGCTGTATTT (SEQ ID NO: 20) | | backbone |
| | AGTTGTGAAGGAGACATCGTGTCATGG (SEQ ID NO: 21) | 1261 bp | *cas2* |
| | TCGCACTCTGCATTATTTCCCATAGAG (SEQ ID NO: 22) | | |
| | GCCGCATATGGTGGAGCGCATCGACTCGCA (SEQ ID NO: 23) | 953 bp | *pah2* |
| | CGCGTCTAGACGATGTCTCCTTCACAACTGGGTTCTG (SEQ ID NO: 24) | | |
| | GCCGTCTAGAACCGACCAGGTCTGACACCCGT (SEQ ID NO: 25) | 1849 bp | *claR* |
| | GCCGGAATTCGGTCATCGGTTCCGTATCCTGTACC (SEQ ID NO: 26) | | |
| | CGGCCATATGATGCCATCCGCACTCCAGG (SEQ ID NO: 27) | 751 bp | *car* |
| | GCCGTCTAGAGGTGTCAGACCTGGTCGGTGGG (SEQ ID NO: 28) | | |
| | GCGCCATATGATGATGAACGAGGCAGCGCCT (SEQ ID NO: 29) | 1439 bp | *cyp,fd* |
| | GCGCTCTAGACTAGCCCTCGGTGACCGTGATG (SEQ ID NO: 30) | | |
| * | CCGGCATATGGTGATTGCCCTGCGCGAGAGAAC (SEQ ID NO: 31) | 617 bp | ORF21 |
| | GCGCTCTAGAACGGGGACGGCCTAGGCGGT (SEQ ID NO: 32) | | |
| * | CGCGCATATGAGGAGACAGGACACGATGGTTTGATG (SEQ ID NO: 33) | 2689 bp | ORF22,23 |
| | CGCGAGATCTGATCACCGAGGTGTACCTCTACCGG (SEQ ID NO: 34) | | |
| * | GCCGCATATGGCGGCACTGTGGTCCCG (SEQ ID NO: 35) | 567 bp | ORF24 |
| | GGCCTCTAGATCATGCCACCGCTCCCTTCACC (SEQ ID NO: 36) | | |

Duplication of the cloned clusters in *S. clavuligerus* ATCC 27064 was confirmed by qPCR coppy number determination. Gene copy numbers of *pah2, claR* and ORF13 genes were determined and the copy number of *ceaS1* gene (located elsewhere in the genome and present in one copy in both cases) was used for normalization. Primers and TaqMan MGB probes were designed by Applied Biosystems (Assay By Design service).

Primers and probes sequences were:

| | | |
|---|---|---|
| SEQ ID NO: 37: | Scla_claR_F | ACCCGGCTCCACATCAC |
| SEQ ID NO: 38: | Scla_claR_R | GAGCGGCGCACCAG |
| SEQ ID NO: 39: | Scla_claR_S | CTCGCCCACCCACCC |
| | | |
| SEQ ID NO: 40: | Scla_pah2_F | GCGGCGATGGTCCAGATC |
| SEQ ID NO: 41: | Scla_pah2_R | TCGTCCGCCGTGACC |
| SEQ ID NO: 42: | Scla_pah2_S | CTCGCTCGACTACGCC |
| | | |
| SEQ ID NO: 43: | Scla_orf13_F | GCCGCTGGTGCTGAC |
| SEQ ID NO: 44: | Scla_orf13_R | CCGGCTCGCGATATTGC |
| SEQ ID NO: 45: | Scla_orf13_S | CCGAACGCCGCCACCA |
| | | |
| SEQ ID NO: 46: | Scla_ceaS1_F | CGGCTACATGGACGGCATT |
| SEQ ID NO: 47: | Scla_ceaS1_R | GATCGCCAGGAGGAGGTC |
| SEQ ID NO: 48: | Scla_ceaS1_S | CACCCGGCCCTCGAC |

Separate 5 µl qPCR reactions for all tested genes were performed for each sample. The reaction mix contained 2.5 µl of TaqMan® Universal PCR Master Mix (Applied Biosystems), 0.25 µl of Gene Expression Assay Mix (Applied Biosystems) and 2 µl of diluted sample DNA. Each sample was analyzed in duplicate of two serial ten-fold dilutions and template-free controls (NTC) were set. The reactions were performed in an ABI PRISM 7900 Sequence Detection System (Applied Biosystems) using a 384-well thermo block and standard cycling settings. The raw data were normalized to a passive reference dye by the SDS 2.4 (Applied Biosystems) software. Baseline was set automatically and threshold was set manually and Ct values were calculated by the software. Relative quantification was performed using comparative C_{T} method (ΔΔC_{T}). Data showed clearly that the genes *pah2, claR* and ORF13 were present in two copies in the *S. clavuligerus* ATCC 27064 pUJS+Clavulanic cluster (BamHI) and *S. clavuligerus* ATCC 27064 pUJS+Clavulanic cluster (Saul) compared to *S*. *clavuligerus* ATCC 27064 where one copy of the said genes was measured when data were normalized to *ceaS1* gene Ct values, confirming the integration of the BAC vector with clavulanic acid gene cluster to *S*. *clavuligerus.*

### Example 7: Transfer of cloned clusters to S. clavuligerus and S. lividans with provision of ΦC31 integrase function in trans

First, a helper vector based on the heat sensitive replicative vector pKC1139 (Kieser et al., 2000, Practical Streptomyces genetics, A laboratory Manual. ISBN0-7084-0623-8) was prepared. pKC1139 was cleaved with SacI and Purified. The Ampicilin, Thiostreptone resistance cassette was amplified from pSET151 (Kieser et al., 2000, Practical Streptomyces genetics, A laboratory Manual. ISBN0-7084-0623-8) in a PCR reaction using oligonucleotide primers
GCGCGAGCTCTCTGACAGTTACCAATGCTTAATCAGTGAGG (SEQ ID NO: 49) and
CCGGGAGCTCTCACTGACGAATCGAGGTCGAGGA (SEQ ID NO: 50).
After cloning into a pGEM-T easy PCR cloning vector (Promega) and cleavage with SacI restriction endonuclease (Promega), 2134 bp fragment was purified and coupled to the SacI cleaved pKC1139 using T4 DNA ligase. The resulting vector pKC1139/Amp_Tsr was then cleaved with XbaI restriction nuclease (Promega). The ΦC31 integrase gene was then amplified from pSET152 in a PCR reaction using oligonucleotide primers
GCGCGAGCTCTCTGACAGTTACCAATGCTTAATCAGTGAGG (SEQ ID NO: 51) and
CCGGGAGCTCTCACTGACGAATCGAGGTCGAGGA (SEQ ID NO: 52).

After cloning into a pGEM-T easy PCR cloning vector (Promega) and cleavage with XbaI restriction endonuclease (Promega), 1753 bp fragment was purified and coupled to the XbaI cleaved purified and coupled to the SacI cleaved pKC1139 using T4 DNA ligase.

The resulting vector pKC1139/C31, was introduced by transformation into electrocompetent *E*. *coli* strain ET12567 containing the conjugation helper plasmid pUZ8002 (Paget et al., 1999 J. Bacteriol. 181: 204-211). Conjugation procedure was done as described by Kieser et al., 2000 (Practical Streptomyces genetics, A laboratory Manual. ISBN0-7084-0623-8) using *S. clavuligerus* ATCC 27064 and *S*. *lividans* TK24 spores. KKA-2L agar plates were used for selection of exoconjugants and Thiostreptone (Sigma) was used for selection at 4 ug/mL. Strains *S*. *clavuligerus* ATCC 27064 pKC1139/ΦC31 and *S. lividans* TK24 pKC1139/ΦC31 were thus obtained.

Next, pUJS-C31/BamHI, was introduced by transformation into electrocompetent *E. coli* strain ET12567 containing the conjugation helper plasmid pUZ8002 (Paget et al., 1999 J. Bacteriol. 181: 204-211). Conjugation procedure was done as described by Kieser et al., 2000 (Practical Streptomyces genetics, A laboratory Manual. ISBN0-7084-0623-8) using *S*. *clavuligerus* ATCC 27064 pKC1139/ΦC31 and *S*. *lividans* TK24 pKC1139/ΦC31 spores. KKA-2L agar plates were used for selection of exoconjugants and apramycin (Sigma) was used for selection at 60 µg/ml. The experiments were conducted with utmost care that the conjugation conditions were equal for all tested plasmids.

4 colonies were obtained with *S*. *clavuligerus* ATCC 27064 pKC1139/ΦC31 and 7 colonies with *S*. *lividans* TK24 pKC1139/ΦC31. PCR and qPCR analysis (methods as in example 5) confirmed the integration of the cloned gene cluster into the genome of both host strains. Strains were then grown in SNK-t medium supplemented with 60µg/ml of Apramycin (inoculated with 1% v/v of spore suspension) and subcultivated for several cycles at 34°C until growth on 4 µg/ml Thiostrepton supplemented KKA-2L agar plates was no longer observed. The strains were cured of the helper plasmid which was additionally confirmed with colony PCR using primers described above (used for amplification of ΦC31 integrase).

### Relevant literature :

Baltz R.H. 1998. Genetic manipulation of antibiotic-producing Streptomyces. Trends Microbio., 6: 67-83 Bendich A.J., Drlica K. 2000. Prokaryotic and eukaryotic chromosomes: what's the difference? Bioessays, 22: 481-486
Benton W.D., Davis R.W. 1977. Screening λgt recombinant clones by hybridization to single plaques in situ. Science, 196:180-182
Bentley S.D., Chater K.F., Cerdeno-Tarraga A.M., Challis G.L., Thomson N.R., James K.D., Harris D.E., Quail M.A., Harper D. 2002. Complete genome sequence of model actinomycete Streptomyes coelicolorA3 (2). Nature, 417: 141-147
Campbell T.N., Choy Y.M. 2002. Approaches to library screening. J. Mol. Microbiol. Biotechnol, 4 (6): 551-554 Combes P., Till R., Bee S., Smith M.C.M. 2002. The Streptomyces genome contains multiple pseudo-attB Sites for the ΦC31-encoded site-specific recombination system. Journal of Bacteriology, 184: 5746-5752
Challis G.L., Hopwood D.A 2003. Synergy and contingency as driving forces for the evolution of multiple secondary metabolite production by Streptomyces species. Proceedings of the National Academy of Sciences of the USA, 100 2: 14555-14561
Dyson P. 2011. Streptomyces: Molecular biology and biotechnology. Norfolk, Caister Academic Press: 257 str. Flärdh K., Buttner M.J. 2009. Streptomyces morphogenetics: Dissecting differentiation in a filamentous bacterium. Nature Reviews Microbiology, 7: 36-49
Flett F., Mersinias V., Smith C.P. 1997. Federation of European Microbiological Societies Microbiological Letters, 155: 223-229
Griffiths J.F., Wessler S.R., Lewontin R.C., Carroll A.B. 2008. Introduction to genetic analysis. New York, W.H. Freeman: 838 str.
Groth A.C., Olivares E.C., Thyagarajn B., Calos M.P. 2000. A phage integrase directs efficient site-specific integration in human cells. Proc. Natl. Acad. Sci:, 97: 5995-6000
Grunstein M., Hogness D.S. 1975. Colony hybridization: a method for the isolation of cloned DNAs that contain a specific gene. PROC. Natl. Acas. Sci., 72:3961-3965
Gottelt M., Kol., Gomez-Escribano J.P., Bibb M., Takano E. 2010. Deletion of a regulatory gene within the cpk gene cluster reveals novel antibacterial activity in Streptomyces coelicolor A3 (2). Microbiology, 256: 2343-2353
Fermentas. 2011. GeneRuler™ DNA Ladder Mix, 100-10,000 bp. Burlington, Formentas http://www.fermentas.com/en/products/all/dna-electrophoresis/generuler-dna-ladders/sm033-generuler-mix (avgust, 2011)
Higgens C.E., Kastner R.E. 1971. Streptomyces clavuligerus sp. Nov., a beta-lactam antibiotic producer. Internal Journal of Systematic Bacteriology, 21 (4): 326-331
Hopwood D.A. 1999. Forty years of genetics with Streptomyces: from in vivo through in vitro to in silico. Microbiology, 145: 2183-2202
Hopwood D.A. 2006. Soil to genomics: the Streptomyces chromosome. Annu Rev Genet, 40: 1-23
Hütter R. 1986. Overproduction of microbal metabolites. V: Biotechnology: a comprehensive treatis in 8 volumes. Vol. 4: Microbial products II. Pape H., Rehm H.J. (eds.). Weinheim, VCH: 3-17
Ikeda H., Ishikawa J., Hanamoto A., Shinose M., Kikuchi H., Shiba T., Sakaki Y., Hattori M., Omura S. 2003. Complete genome sequence and comparative analysis of the industrial microorganism Streptomyces avermitilis. Nat. Biotechnol, 21: 526-531
Janawali N. H., Yoo C. J., Sohng K. J. 2011. Improvment of clavulanic acid production in Streptomyces clavuligerus by genetic manipulation of structual biosynthesis genes. 33: 1221-1226
Keiser T., Bibb M.J., Chater K.F., Hopwood D.. 2000. Practical Streptomyces genetics. Norwich, John Innes Foundation: 610 str.
Kim U.J., Birren B.W., Slepak T., Mancino V., Boysen C., Kang H.L., Simon M.I., Shizuya H. 1996. Construction and characterization of a human bacterial artificial chromosome library. Genomics, 34: 213-218
Liras P., Gomez-Escribano P. J., Santamarta I. 2008. Regulatory mechanisms controlling antibiotic production in Streptomyces clavuligerus. 35: 667-676
Liu H., He M. 2009. Vectros and methods for cloning gene clusers or portions thereof. US patent application publication US 2009/0075283 A1.
Madigan M.T., Martinko J.M., Parker J. 2003. Brock biology of microorganisms. 10th edition. New Jersy, Pretence-Hall: 1019 str.
Martin J.F., Casqueiro J., Liras P. 2005. Secretion system for secondary metabolites: how producer cells send out messages of intrecellular communication. Current Opinion in Microbiology, 8: 282-293
Mazodier P., Petter R., Thompson C. 1989. Intergeneric conjugation between Echerichia coli and Streptomyces species. Journal of Bacteriology, 171: 3593-3585
Medema M.H., Trefzer A., Kovalchuk A., van den Berg M., Müller U., Heijne W., Wu L., Alam M.T., Ronning C.M., Nierman W.C., Bovenberg A.L., Breitling R., Takano E. 2010. The sequence of a 1.8-Mb bacterial linear plasmid reveals a rich evolutionary reservoir of secondary metabolic pathways. Genome Biol. Evol., 2: 212-224 Medema M.H., Breitling R., Takano E. 2011. Synthetic biology in Streptomyces bacteria. Methods in Enzymology, 497: 485-502
Narayanan K., Chen Q. 2010. Bacterial artificial chromosome mutagenesis using recombineering. Journal of Biomeicine and Biotechnology, 2011: 1-10
Paradkar A.S., Mosher R.H., Anders C., Giffin S., Griffin J., Hughes C., Greaves P., Barton B., Jensen S.E. 2001. Application of Gene Replacement Technology to Streptomyces clavuligerus strain development for clavulanic acid production. Appl. Environ. Microbiol., 67 (5): 2292-2297
Perez-Redondo R., Rodriguez-Garzia A., Martin J.F., Liras P. 1999. J.Bacteriol., 181 (22): 6922-6928
She K. 2003. So you want to work with giants: The BAC vector. BioTeach Journal, 1: 69-74
Shizuya H., Birren B., Kim U.J., Mancino V., Slepak T., Tachiiri Y., Simon M. 1992. Cloning and stable maintenance of 300-kilobase-pair fragments of human DNA in Escherichia coli using an F-factor-based vector. Proc Natl Acad Sci USA,89 18: 8794-8797
Shizuya H., Kouros-Mehr H. 2001. The development and application od the bacterial artificial chromosome cloning system. Keio J Med, 50 1: 26-30
Smith M.C.M., Thorpe H.M. 2002. Diversity in serine recombinases. Mol. Microbio., 44: 299-307
Song J. Y., Jensen E. S., Lee K. J. 2010. Clavulanic acid biosynthesis and genetics manipulation for its overproduction. 88: 659-669
Tala A., Wang G., Zemanova M., Okamoto A., Ochi K., Alifano P. 2009. Activation of dormant bacterial genes by Nonmuraea sp. strain ATCC 39727 mutant-type RNA polymerase. J. Bacteriol, 191: 805-814
Townsend C.A. 2002. New reactions in clavulanic acid biosynthesis. Current Opinion in Chemical Biology, 6: 583-589
Vining L.C. 1986. Secondary metabolism. V: Biotechnology: a comprehensive treatis in 8 volumes. Vol. 4: Microbial products II. Pape H., Rehm H.J. (eds.). Weinheim, VCH: 19-38
Watve M.G., Tickoo R., Jog M.M., Bhole B.D. 2001. How many antibiotics are produced by the genus Streptomyces. Archives of Microbiology, 176: 386-390
You F.M., Luo M.C., Xu K., Deal K.R., Anderson O.D., Dvorak J. 2010. A new implementation of high-throughput five-dimensional clone pooling strategy for BAC library screening. Genomics, 11:692-701

## Claims

1. A method for targeted cloning and/or transferring of DNA fragments from a donor organism genome into a host cell, the method comprising the following steps:
(a) Transferring a Bacterial Artificial Chromosome (BAC)-vector into the donor organism,
wherein said vector comprises
(a1) an origin of replication;
(a2) an origin of transfer;
(a3) a site-specific recombination system containing a functional integrase and an attP site; (a4) a DNA sequence that is substantially homologous to a desired target integration site in the donor organism and that allows for homologous recombination with a corresponding homologous DNA sequence being present in the genomic DNA of the donor organism; and
(a5) a selection marker,
wherein said donor organism does not contain a functional attB site
(b) Isolating the genomic DNA from the donor organism;
(c) Transferring the genomic DNA from step (b), which is present in a BAC-vector derived from the BAC-vector of step (a), into a prokaryotic organism;
(d) Selecting a prokaryotic organism that contains the genomic DNA which is present in the BAC-vector of step (c);
(e) Optionally, isolating said BAC-vector from the prokaryotic organism of step (d);
(f) Introducing the BAC-vector from step (d), or, optionally, the isolated BAC-vector from step (e), via conjugation into the host cell, whereupon said BAC-vector integrates into the host cell genome.

2. A method for targeted cloning and/or transferring of DNA fragments from a donor organism genome into a host cell, the method comprising the following steps:
(a) Transferring a BAC-vector into the donor organism,
wherein said vector comprises
(a1) an origin of replication;
(a2) an origin of transfer;
(a3) a site-specific recombination system containing an attP site however no functional integrase;
(a4) a DNA sequence that is substantially homologous to a desired target integration site in the donor organism and that allows for homologous recombination with a corresponding homologous DNA sequence being present in the genomic DNA of the donor organism; and
(a5) a selection marker,
(b) Isolating the genomic DNA from the donor organism;
(c) Transferring the genomic DNA from step (b), which is present in a BAC-vector derived from the BAC-vector of step (a), into a prokaryotic organism;
(d) Selecting a prokaryotic organism that contains the genomic DNA which is present in the BAC-vector of step (c);
(e) Optionally, isolating said BAC-vector from the prokaryotic organism of step (d);
(f) Introducing the BAC-vector from step (d), or, optionally, the isolated BAC-vector from step (e), via conjugation into the host cell, whereupon said BAC-vector integrates into the host cell genome by action of an integrase the host cell is provided with.

3. The method according to any of the previous claims, wherein the BAC-vector comprises one origin of replication.

4. The method according to any of the previous claims, wherein between any of steps (c) to (f), preferably between steps (c) and (f) the BAC-vector from step (c) is not modified, preferably no attP-site, attP-site integrase cassette, and/or integrase cassette is introduced into said BAC-vector.

5. The method according to any of the previous claims, wherein the genomic DNA that is present in the BAC-vector from steps (c) to (f) comprises a DNA fragment from the donor organism.

6. The method according to claim 5, wherein the DNA fragment has a size of from about 5 kb to about 500 kb.

7. A vector comprising
(1) an origin of replication;
(2) an origin of transfer;
(3) a site-specific recombination system containing a functional integrase and an attP site;
(4) a DNA sequence that allows for homologous recombination with a corresponding substantially homologous DNA sequence being present in the genomic DNA of a donor organism; and
(5) a selection marker.

8. A vector comprising
(1) an origin of replication;
(2) an origin of transfer;
(3) a site-specific recombination system containing an attP site however no functional integrase; and
(4) a selection marker.

9. The vector according to claim 7 or 8, wherein said vector comprises one origin of replication.

10. The vector according to claim 8 or 9, wherein said vector further comprises a DNA sequence that allows for homologous recombination with a corresponding substantially homologous DNA sequence being present in the genomic DNA of a donor organism.

11. A donor organism, comprising a BAC-vector as defined in claims 1 (a1) - (a5), or as defined in any one of claims 7, or 9 or 10.

12. The donor organism according to claim 11, wherein said donor organism does not contain a functional attB site.

13. A donor organism, comprising a BAC-vector as defined in claim 2 (a1) to (a5), or as defined in any one of claims 8 to 10.

14. An expression system, comprising
(1) a host cell, and
(2) a BAC-vector, wherein said BAC-vector comprises
i) an origin of replication;
ii) an origin of transfer;
iii) a site-specific recombination system containing an attP site and a functional integrase;
iv) a DNA fragment comprising a whole gene cluster or a part of a gene cluster encoding one or more gene product(s) that is (are) part of a biosynthetic pathway for secondary metabolites; and
v) a selection marker.

15. An expression system, comprising
(1) a host cell, wherein said host cell is provided with a functional integrase, and
(2) a BAC-vector, wherein said BAC-vector comprises
i) an origin of replication;
ii) an origin of transfer;
iii) a site-specific recombination system containing an attP site however no functional integrase;
iv) a DNA fragment comprising a whole gene cluster or a part of a gene cluster encoding one or more gene product(s) that is (are) part of a biosynthetic pathway for secondary metabolites; and
v) a selection marker.

16. The expression system according to claim 14 or 15, wherein the BAC-vector comprises one origin of replication.

17. A vector comprising the sequence according to SEQ ID NO: 3.

18. A vector comprising the sequence according to SEQ ID NO: 53.

19. The vector according to claim 17 or 18, additionally comprising a DNA sequence that allows for homologous recombination with a corresponding substantially homologous DNA sequence being present in the genomic DNA of a donor organism.

20. Use of a vector according to any of claims 17 to 19 for cloning and/or transfer of a DNA fragment, and/or for expression of said DNA fragment.
